(19) Europäisches Patentamt
European Patent Office
Office européen des brevets

(11) EP 4 745 238 A1

(12) EUROPEAN PATENT APPLICATION
published in accordance with Art. 153(4) EPC

(43) Date of publication:
**20.05.2026 Bulletin 2026/21**

(21) Application number: **24842335.2**

(22) Date of filing: **13.07.2024**

(51) International Patent Classification (IPC):
***C12N 15/113*** (2010.01) ***A61K 31/713*** (2006.01)
***A61P 9/12*** (2006.01) ***A61P 9/10*** (2006.01)
***A61P 9/00*** (2006.01) ***A61P 13/12*** (2006.01)
***A61P 9/04*** (2006.01) ***A61P 3/10*** (2006.01)

(52) Cooperative Patent Classification (CPC):
**A61K 31/713; A61P 3/10; A61P 9/00; A61P 9/04; A61P 9/10; A61P 9/12; A61P 13/12; C12N 15/113**

(86) International application number:
**PCT/CN2024/105377**

(87) International publication number:
**WO 2025/016342 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 CN 202310869337**
**22.09.2023 CN 202311237018**
**23.04.2024 CN 202410490275**
**05.07.2024 CN 202410904439**

(71) Applicant: **Anlong Biopharmaceutical Co., Ltd.**
**Beijing 101318 (CN)**

(72) Inventors:
- **GAO, Jie**
  **Beijing 101318 (CN)**
- **ZHENG, Wenzhi**
  **Beijing 101318 (CN)**
- **WANG, Haojun**
  **Beijing 101318 (CN)**

(74) Representative: **Casalonga**
**Casalonga & Partners**
**Bayerstraße 71/73**
**80335 München (DE)**

Remarks:
The complete document including Reference Table(s) and the Sequence Listing(s) can be downloaded from the EPO website

# (54) OLIGONUCLEOTIDE TARGETING ANGIOTENSINOGEN AND USE THEREOF

(57) The present invention relates to an oligonucleotide targeting angiotensinogen and the use thereof. The oligonucleotide significantly inhibits the expression level of an AGT gene, and has long-lasting efficacy.

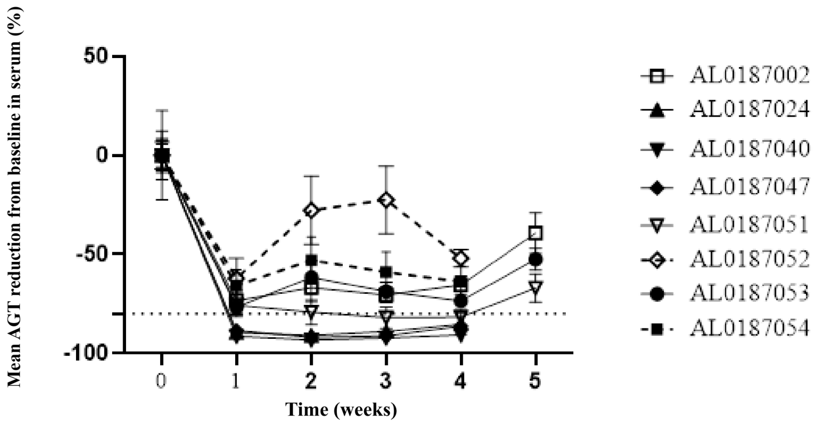


FIG. 8



EP 4 745 238 A1

## Description

### TECHNICAL FIELD

The present disclosure relates to an oligonucleotide, particularly a targeting oligonucleotide for treating hypertension or related diseases.

### BACKGROUND

In 1998, two American scientists, Andrew Fire and Craig Mello, discovered a biological mechanism: small interfering RNA (siRNA) molecules can mediate the degradation of specific mRNAs (Fire, Andrew, et al. *Nature* 391.6669 (1998): 806-811). This mechanism is induced and activated when RNA molecules appear in cells in double-stranded form, leading to the phenomenon known as RNA interference. This discovery heralded the beginning of a new research field, for which the two scientists were awarded the Nobel Prize in Physiology or Medicine in 2006. Upon binding to the protein complex Dicer, double-stranded RNA (dsRNA) is cleaved into fragments by Dicer. Subsequently, another protein complex, RISC, binds to these fragments. One strand in the double-stranded siRNA is removed, while the other strand remains bound to the RISC complex. Through the guidance of the single-stranded RNA, RISC recognizes and degrades the mRNA of the target gene, thereby inhibiting the expression of the specific protein and thus causing specific gene silencing.

RNA interference has opened up a new field for the application of gene technology. Double-stranded RNA (dsRNA) molecules have been artificially designed to silence specific genes in humans, animals, or plants. These artificially designed and synthesized double-stranded small interfering RNA (siRNA) molecules for gene silencing are introduced into cells and activate the RNA interference mechanism to degrade the corresponding mRNA. Currently, this method is an important research tool in biology and biomedicine. In addition, numerous siRNA drugs have been developed to treat viral infections, cardiovascular diseases, cancer, endocrine disorders, and various other diseases. Most of the siRNA therapies, whether in the research and development stage or already approved for marketing, have shown good therapeutic effects. Since the launch of the first siRNA drug in 2018, at least 6 siRNA therapies have been approved for marketing in the European Union or the United States. Therefore, utilizing RNA interference technology to inhibit the expression of specific target genes has become an effective approach for disease treatment.

The asialoglycoprotein receptor (ASGPR) in the liver is a highly efficient endocytic receptor specifically expressed on hepatocytes. Under physiological conditions *in vivo,* after sialic acid is removed from various glycoproteins by enzymatic or acidic hydrolysis, the exposed subterminal residue is a galactose residue. Therefore, the sugar specifically bound by ASGPR is galactosyl, hence ASGPR is also known as a galactose-specific receptor. Monosaccharide and polysaccharide molecules such as galactose, galactosamine, and N-acetylgalactosamine (GalNAc) all exhibit high affinity for ASGPR. The primary physiological function of ASGPR is to mediate the clearance of asialoglycoproteins, lipoproteins, and other substances in the blood. It is also closely associated with the occurrence and development of liver diseases such as viral hepatitis, cirrhosis, and liver cancer. The discovery of this characteristic of ASGPR has played an important role in the diagnosis and treatment of hepatic diseases (Ashwell G, Harford J, Carbohydrate specific Receptors of the Liver, Ann Rev Biochem 1982 51:531-554). Hepatic disease therapeutic drugs that contain galactose or galactosamine or derivatives thereof in their structure can specifically bind to ASGPR with affinity, thereby exhibiting active liver-targeting properties without requiring other carrier systems for delivery.

Blood pressure refers to the pressure exerted by blood on the walls of blood vessels in the circulatory system. Blood pressure is mainly due to the beating of the animal's heart. During each heartbeat, blood pressure varies between a maximum (systolic) blood pressure (SBP) and a minimum (diastolic) blood pressure (DBP). Mean arterial pressure (MAP) is the average arterial pressure during a cardiac cycle. Blood pressure can be measured with a sphygmomanometer (i.e., a blood pressure measuring device). Normal blood pressure at rest falls within the range of 100-140 mmHg systolic and 60-90 mmHg diastolic, and is typically expressed as systolic blood pressure (highest reading)/diastolic blood pressure (lowest reading) in mmHg.

The *Clinical Practice Guidelines for the Management of Hypertension in China* (2022 Edition) recommend lowering the diagnostic threshold for hypertension in Chinese adults from SBP ≥ 140 mmHg and/or DBP ≥ 90 mmHg to SBP ≥ 130 mmHg and/or DBP ≥ 80 mmHg (1B).

It is recommended that adult hypertension patients in China be classified according to blood pressure levels as follows:

Grade 1 (SBP 130-139 mmHg and/or DBP 80-89 mmHg);
individuals with SBP of 130-139 mmHg and/or DBP of 80-89 mmHg exhibit a significantly elevated absolute risk of cardiovascular disease only when accompanied by clinical comorbidities, target organ damage, or ≥ 3 cardiovascular risk factors; and

Grade 2 (SBP ≥ 140 mmHg and/or DBP ≥ 90 mmHg) (1B);
research evidence shows that individuals with SBP ≥ 140 mmHg and/or DBP ≥ 90 mmHg have already reached a 10-year cumulative incidence risk of cardiovascular disease of 15%, and ≥ 80% of them are accompanied by 2 or more cardiovascular risk factors, so that the vast majority of patients with SBP ≥ 140 mmHg and/or DBP ≥ 90 mmHg belong to the high-risk cardiovascular population.

Based on the cause of the disorder, hypertension can also be classified as primary hypertension and secondary hypertension. Primary hypertension refers to high blood pressure caused by multiple factors or by unknown reasons. It may stem from various causes such as genetics, geographical location, sodium and water retention, sympathetic excitation, and RAS activation. Therefore, primary hypertension can only be managed, not cured. Secondary hypertension refers to elevated blood pressure caused by an underlying disease, where hypertension is one of the clinical symptoms of the primary disease, accounting for 95% of cases. Generally, secondary hypertension is commonly seen in renal hypertension, renal artery stenosis, primary aldosteronism, pheochromocytoma, Takayasu's arteritis, etc.

The renin-angiotensin-aldosterone system (abbreviated as RAAS or RAS (renin-angiotensin system)) is a system in the human body that regulates cardiovascular function. It is governed by the sympathetic nervous system, and the angiotensin it secretes has a vasoconstrictive effect. Excessive stimulation or activity of the RAS pathway is one of the causes of hypertension formation. Angiotensinogen (AGT) is a member of the serpin family, also known as SERPINA8. It is encoded by the AGT gene and serves as the sole precursor for all angiotensin peptides in RAS. Human AGT has 485 amino acids, including a 33-amino acid signal peptide. It is primarily produced in the liver and released into the systemic circulation, where renin converts it into angiotensin I. Subsequently, angiotensin I is converted into angiotensin II by the angiotensin-converting enzyme (ACE). Angiotensin I can stimulate the adrenal medulla to secrete epinephrine, but its direct vasoconstrictive effect is not significant. Angiotensin II can cause systemic arteriolar constriction, thereby elevating blood pressure; additionally, it can also promote the secretion of aldosterone from the adrenal cortex. Aldosterone acts on the renal tubules, promoting sodium retention, water retention, and potassium excretion, thereby leading to increased blood volume and elevated blood pressure.

For initial antihypertensive medication in hypertension patients without clinical comorbidities, the Clinical Practice Guidelines for the Management of Hypertension in China (2022 Edition) recommend ACEIs, ARBs, CCBs, and diuretics as first-line initial antihypertensive drugs (1B). For hypertension patients with blood pressure ≥ 140/90 mmHg, initial combination antihypertensive drug therapy is recommended (1B). For hypertension patients who require combination antihypertensive drug therapy, single-pill combination (SPC, 2C) is recommended as the first choice. For the selection of SPC, it is recommended to preferentially choose a combination of renin-angiotensin system inhibitor (RASI) + CCB or RASI + diuretic (2C). On March 11, 2023, at the 2022 China Hypertension Meeting and the 24th International Symposium on Hypertension & Related Diseases, the expert group of the *Chinese Guidelines for Prevention and Treatment of Hypertension* committee previewed and discussed the key updates in the *2023 Chinese Guidelines for Prevention and Treatment of Hypertension* (then under revision). The focus was primarily on aspects such as the addition of new drug recommendations, hypertension epidemiological data, and the refinement of risk factors. As one of the key points in the preview, angiotensin receptor-neprilysin inhibitors (ARNIs) have been included in the guideline recommendations for the first time as a commonly used drug for hypertension. Its mechanism of action is illustrated in FIG. 1.

Currently, these six classes of drugs commonly used in clinical practice all require long-term, daily, uninterrupted administration. However, poor adherence among some patients is primarily attributed to the side effects associated with these drugs. Both ACEIs and ARBs may cause dry cough and edema, and in severe cases, may lead to renal insufficiency. Calcium channel blockers (CCB) and β-blockers may cause side effects such as tachycardia, facial flushing, headache, and pedal edema. β-blockers may also lead to fatigue and affect blood glucose and blood lipid metabolism. Diuretics may cause physical weakness and cramps, and in severe cases, may induce gout. Furthermore, due to the existence of alternative pathways, all current oral antihypertensive drug targets frequently exhibit drug escape in the RAAS system. Therefore, there is a need in this field for more effective treatments with novel mechanisms of action different from existing clinical drugs and with fewer side effects.

One of the off-target effects of siRNA is the miRNA-like effect, where the argonaute protein, a core effector in RNA interference, processes the artificially introduced siRNA designed to induce RNA interference as if it were microRNA (miRNA) (Lam et al. (2015) Molecular Therapy Nucleic Acids (2015) 4, e252). miRNAs primarily recognize target genes through base pairing between the seed region (positions 2-9 from the 5' end) and the target mRNA for gene suppression. Off-target effects caused by siRNA originate from the base complementarity of the seed region of the RISC-loaded antisense strand of iRNA with one or more mRNAs. In several studies, the miRNA-like off-target effects in siRNAs have been reported, which, depending on the sequence of the seed region, influence the expression of multiple genes and are severe enough to account for up to 30% of positive hits in siRNA-based phenotypic screens. Furthermore, in the case of miRNAs, when the interaction between the seed region and the target is weakened, they have also been reported to silence the target gene through compensatory pairing within their 3'-end region (3'-compensatory pairing), suggesting that miRNA-like off-target effects may be regulated by this mechanism. It is known that siRNA designs incorporating chemical

modifications at different positions on the siRNA antisense strand can eliminate or reduce the miRNA-like off-target effects of siRNA without impairing the gene silencing efficacy of siRNA gene therapy. For example, by replacing bases at different positions on the siRNA antisense strand with glycol nucleic acid (GNA)-modified bases (common glycol nucleic acids include Tgn, Cgn, Agn, and Ggn), or by linking the 2'-phosphate to the 5'-phosphate of the next nucleic acid, the miRNA-like off-target effects of siRNA can be eliminated or reduced (Schlegel et al. (2021), CN 110582283 A).

## SUMMARY

In order to solve the problems existing in the prior art, the present disclosure aims to provide an inhibitor targeting AGT with good therapeutic effect, high safety, and long-lasting efficacy.

In one aspect, the present disclosure provides an oligonucleotide or a pharmaceutically acceptable salt thereof for reducing AGT expression, wherein the oligonucleotide described above comprises a sense strand and an antisense strand; the sense strand described above has a sequence having at least 80% or more sequence identity, preferably 85%, 90%, 95%, 96%, 97%, 98%, 99% or more sequence identity, to any one of the sequences set forth in SEQ ID NOs: 2-219, 789-804, and 868-871 or a fragment thereof, or a modified sequence of the sequence or the fragment thereof described above, and the antisense strand described above has a sequence having at least 80% or more sequence identity, preferably 85%, 90%, 95%, 96%, 97%, 98%, 99% or more sequence identity, to any one of the sequences set forth in SEQ ID NOs: 221-424, 805-821, and 872-875 or a fragment thereof, or a modified sequence of the sequence or the fragment thereof described above.

In another aspect, the present disclosure provides a composition, comprising the oligonucleotide or the pharmaceutically acceptable salt thereof described above, and optionally a pharmaceutically acceptable carrier.

In another aspect, the present disclosure provides use of the oligonucleotide or the pharmaceutically acceptable salt thereof, or the composition described above in the preparation of a medicament for treating or/and preventing an AGT-related disease. Experiments have demonstrated that the candidate compounds of the present disclosure exhibit significant inhibitory effects on the AGT gene expression level in the human liver cancer cell line Hep3B; some of these candidate compounds achieved 90% or more inhibition of AGT gene expression level in the Hep3B cell strain, and some of the compounds achieved 70%, 80%, or even 90% or more inhibition of AGT gene expression in cynomolgus monkey hepatocytes, showing good inhibitory effects. Some of the compounds could maintain about 60% reduction in AGT protein levels in hAGT transgenic mice approximately 2 months after administration (Day 29); some of the compounds could sustain 80% or more reduction in AGT protein levels for four consecutive weeks (Day 28) in mice, demonstrating long-lasting efficacy. These compounds can be used to treat primary hypertension and secondary hypertension, as well as related diseases.

The inhibition of AGT gene expression can be indicated by a reduction in the amount of mRNA expressed by a cell line (such cells may be present in, for example, a sample derived from a subject), in which the AGT gene is transcribed in the cell or cell population and which is treated (e.g., by contacting one or more cells with the iRNA of the present disclosure, or by administering the iRNA of the present disclosure to a subject in whom the cells are present or were previously present), such that expression of the AGT gene is inhibited as compared to a substantially identical but untreated cell line (control cells not treated with iRNA or not treated with iRNA targeting the gene of interest). In a preferred embodiment, the inhibition is evaluated in species-matched cell lines using siRNA concentrations of 0.5 nM and 0.05 nM according to the method provided in Example 1; the inhibition is represented by the mRNA expression level in treated cells relative to that in control cells, calculated as 2^-△△CT using the formula below, where a housekeeping gene (e.g., hTBP or GAPDH) is used as an internal reference for normalization:

$$\triangle CT = CT_{AGT} - CT_{\text{housekeeping gene}}$$

$$\triangle\triangle CT = \triangle CT_{\text{treated cells}} - \triangle CT_{\text{control cells}}$$

$$\text{mRNA level} = 2\text{^-}\triangle\triangle CT$$

In other embodiments, the inhibition of AGT gene expression can be evaluated based on a decrease in parameters related to the function of AGT gene expression, such as the level of AGT protein in the blood or serum from a subject. AGT gene silencing can be determined in any cell expressing AGT, whether endogenously or heterologously from an expression construct, and by any assay well known in the art.

The inhibition of AGT protein expression can be reflected by a reduction in the level of AGT protein expressed in a cell or cell population or a subject sample (e.g., the level of protein in a blood sample from a subject). As described above, to evaluate mRNA inhibition, the inhibition of the protein expression level in a treated cell or cell population can similarly be expressed as a percentage of the protein level in a control cell or cell population, or as a change in the protein level in a

subject sample (e.g., blood or serum from the subject); the inhibition is evaluated by the method provided in Example 3, Example 4, Example 5, Example 6, or Example 7, and is expressed as a percentage of the AGT expression in the treated sample (e.g., blood or serum from the subject) relative to the AGT expression in the control or relative to the AGT expression before administration, as shown in the formula below.

Percentage inhibition of mRNA = (protein expression level$_{\text{treated cells}}$ - protein expression level$_{\text{control cells}}$)/protein expression levelcontrol cells $\times$ 100%

Control cells, cell populations, or subject samples that can be used to evaluate the inhibition of AGT gene expression include cells, cell populations, or subject samples that have not been contacted with the RNAi agent of the present disclosure. For example, the control cells, cell lines, or subject samples may be derived from an individual subject (e.g., a human or animal subject) prior to treatment of the subject or an appropriately matched population control with the RNAi agent.

In some embodiments of the methods of the present disclosure, the iRNA is administered to a subject such that the iRNA is delivered to a specific site within the subject. The inhibition of AGT expression can be evaluated by measuring the level of, or the change in, AGT mRNA or AGT protein in a sample of fluid or tissue from a specific site (e.g., liver or blood) in the subject.

## BRIEF DESCRIPTION OF THE DRAWINGS

FIG. 1 shows the sites of action of renin-angiotensin-aldosterone system inhibitors, where the solid lines indicate the primary pathway, the dotted lines indicate the alternative pathway, and the dotted line indicate the blockade pathway. ACE: angiotensin-converting enzyme; ARB: angiotensin receptor blocker; AT-R: angiotensin receptor; DRI: direct renin inhibitor; LVH: left ventricular hypertrophy.

FIG. 2 shows steps in solid-phase siRNA synthesis.

FIG. 3 shows the *in vivo* efficacy I of AGT siRNAs in hAGT transgenic mice.

FIG. 4 shows the *in vivo* efficacy II of AGT siRNAs in hAGT transgenic mice.

FIGs. 5 and 6 show the *in vivo* efficacy III of AGT siRNAs in hAGT transgenic mice.

FIGs. 7 and 8 show the *in vivo* efficacy IV of AGT siRNAs in hAGT transgenic mice.

FIGs. 9, 10, and 11 show the *in vivo* efficacy of AGT siRNAs in non-primate (NHP) animals.

## DETAILED DESCRIPTION

In the present disclosure, unless otherwise specified, scientific and technical terms used herein have the meanings generally understood by those skilled in the art. In addition, the terms and laboratory procedures related to protein and nucleic acid chemistry, molecular biology, cell and tissue culture, microbiology, and immunology used herein are the terms and conventional procedures widely used in the corresponding fields. Moreover, in order to better understand the present disclosure, the definitions and explanations of related terms are provided below.

As used herein, the term "about" or "approximately", as applied to one or more target values, refers to a value that is similar to the reference value. In certain embodiments, unless otherwise specified or clearly indicated by the context, the term "approximately" or "about" refers to a range of values that fall within 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value (unless such a number would exceed 100% of a possible value).

As used herein, the term "angiotensinogen" may be used interchangeably with the term "AGT", referring to the well-known gene and polypeptide, also known in the art as: Serpin peptidase inhibitor, clade A, member 8; $\alpha$-1 antiproteinase; antitrypsin; SERPINA8; angiotensin I; Serpin A8; angiotensin II; $\alpha$-1 antiproteinase angiotensinogen; antitrypsin; pre-angiotensinogen 2; ANHU; serine protease inhibitor; and cysteine protease inhibitor.

As used herein, the term "complementary" refers to a structural relationship between nucleotides (e.g., two nucleotides on opposite nucleic acids or on opposite regions of a single nucleic acid strand) that allows the nucleotides to

form base pairs with each other. For example, a purine nucleotide of one nucleic acid that is complementary to a pyrimidine nucleotide of an opposite nucleic acid can be base-paired together by forming hydrogen bonds with each other. In some embodiments, complementary nucleotides can be base-paired in a Watson-Crick manner, or in any other manner that allows for the formation of a stable duplex. In some embodiments, two nucleic acids may have nucleotide sequences that are complementary to one another in order to form complementary regions, as described herein.

As used herein, the term "strand" refers to a single, contiguous sequence of nucleotides linked together by internucleotide linkages (e.g., phosphodiester linkages and phosphorothioate linkages). In some embodiments, a strand has two free ends, for example, a 5'-end and a 3'-end.

As used herein, the term "deoxyribonucleotide" refers to a nucleotide that has a hydrogen atom at the 2' position of its pentose sugar as compared to a ribonucleotide. A modified deoxyribonucleotide is a deoxyribonucleotide that has one or more modifications or substitutions of atoms other than at the 2' position (including modifications or substitutions in or of the sugar, phosphate group, or base).

As used herein, the term "double-stranded RNA" or "dsRNA" refers to a complex of ribonucleic acid molecules, which has a duplex structure comprising two antiparallel and substantially complementary nucleic acid strands with "sense" and "antisense" orientations relative to the target RNA (i.e., the AGT gene). In some embodiments of the present disclosure, the double-stranded RNA (dsRNA) triggers degradation of a target RNA (e.g., mRNA) through a post-transcriptional gene silencing mechanism referred to herein as RNA interference or RNAi. In general, the majority of nucleotides in each strand of a dsRNA molecule are ribonucleotides, but as described in detail herein, each strand or both strands may also comprise one or more non-ribonucleotides, such as deoxyribonucleotides or modified nucleotides. In addition, as used herein, "iRNA" may comprise ribonucleotides with chemical modifications; the iRNA may comprise substantial modifications at multiple nucleotides.

As used herein, the term "modified nucleotide" refers to a nucleotide that independently has a modified sugar moiety, a modified internucleotide linkage, or a modified nucleobase, or any combination thereof. Thus, the term "modified nucleotide" encompasses a substitution, addition, or removal of, for example, a functional group or atom, on the internucleoside linkage, sugar moiety, or nucleobase. Modifications applicable to the agent of the present disclosure include all types of modifications disclosed herein or known in the art.

As used herein, the term "oligonucleotide" refers to a short nucleic acid, for example, a short nucleic acid that is less than 100 nucleotides in length. The oligonucleotide may comprise a ribonucleotide, a deoxyribonucleotide, and/or a modified nucleotide, including, for example, a modified ribonucleotide. The oligonucleotide may be single-stranded or double-stranded. The oligonucleotide may or may not have a duplex region. As a group of non-limiting examples, the oligonucleotide may be, but is not limited to, a small interfering RNA (siRNA), a microRNA (miRNA), a short hairpin RNA (shRNA), a Dicer-substrate interfering RNA (dsiRNA), an antisense oligonucleotide, a short siRNA, or a single-stranded siRNA. In some embodiments, the double-stranded oligonucleotide is an RNAi oligonucleotide. As used herein, "conjugation" means that two or more chemical moieties each having a specific function are covalently linked to each other; accordingly, "conjugate" refers to a compound formed by covalent linking of the various chemical moieties. Further, "siRNA conjugate" refers to a compound formed by covalently linking one or more chemical moieties with specific functions to an siRNA. Hereinafter, the siRNA conjugate of the present disclosure is also sometimes referred to simply as "conjugate". The term "siRNA conjugate" shall be understood, depending on the context, as a general term for siRNA conjugates, a first type of siRNA conjugate or a second type of siRNA conjugate, or an siRNA sense strand conjugate or an siRNA antisense strand conjugate.

As used herein, the term "double-stranded oligonucleotide" refers to an oligonucleotide that is substantially in a duplex form. In some embodiments, complementary base pairing of one or more duplex regions of the double-stranded oligonucleotide is formed between antiparallel sequences of nucleotides in covalently separated nucleic acid strands. In some embodiments, complementary base pairing of one or more duplex regions of the double-stranded oligonucleotide is formed between antiparallel sequences of nucleotides in covalently linked nucleic acid strands. In some embodiments, complementary base pairing of one or more duplex regions of the double-stranded oligonucleotide is formed from a single nucleic acid strand that is folded (e.g., via a hairpin) to provide complementary antiparallel sequences of nucleotides that are base-paired together. In some embodiments, the double-stranded oligonucleotide comprises two covalently separated nucleic acid strands that are fully duplexed with one another. However, in some embodiments, the double-stranded oligonucleotide comprises two covalently separated nucleic acid strands that are partially duplexed, for example, having overhangs at one or both ends. In some embodiments, the double-stranded oligonucleotide comprises antiparallel sequences of nucleotides that are partially complementary, and thus, may have one or more mismatches, which may include internal mismatches or end mismatches.

As used herein, the term "nucleotide overhang" refers to at least one unpaired nucleotide that protrudes from the duplex structure of a double-stranded iRNA. For example, a nucleotide overhang is present when the 3' end of one strand of the dsRNA extends beyond the 5' end of the other strand, or vice versa. The dsRNA may comprise an overhang of at least one nucleotide; alternatively, the overhang may comprise at least two nucleotides, at least three nucleotides, at least four nucleotides, at least five nucleotides, or more. The nucleotide overhang may comprise or consist of nucleotide/nucleoside analogs, including deoxynucleotides/nucleosides. The overhang may be on the sense strand, the antisense strand, or any combination thereof. In addition, the nucleotides of the overhang may be present on the 5' end, the 3' end, or both ends of the antisense strand or the sense strand of the dsRNA.

As used herein, the term "naked sequence" refers to an unmodified nucleotide sequence.

As used herein, the term "subject" refers to an animal that expresses a target gene endogenously or heterologously, such as a mammal, including primates (e.g., humans or non-human primates such as monkeys and chimpanzees), non-primates (e.g., cattle, pigs, horses, goats, rabbits, sheep, hamsters, guinea pigs, cats, dogs, rats, or mice), or birds. In one embodiment, the subject is a human.

As used herein, the term "treating" or "treatment" refers to a beneficial or desired outcome, such as reducing at least one sign or symptom of an AGT-related disorder in a subject. The treatment also includes: reducing one or more signs or symptoms associated with undesired AGT expression; attenuating the degree of undesired AGT activation or stabilization; or ameliorating or alleviating undesired AGT activation or stabilization. The treatment also includes reducing one or more signs or symptoms associated with undesired AGT expression. "Treating" or "treatment" can also mean prolonging survival as compared to the expected survival without treatment.

As used herein, the term "prevention" or "preventing", when used in reference to a disease or disorder, will benefit from a reduction in AGT gene expression or AGT protein production.

As used herein, the term "therapeutically effective amount" is intended to include an amount of the RNAi agent that, when administered to a subject suffering from an AGT-related disorder, is sufficient to affect the treatment of the disease (e.g., by reducing, ameliorating, or maintaining the existing disease or one or more symptoms of the disease). The "therapeutically effective amount" may vary depending on the RNAi agent, how the agent is administered, the disease and its severity, as well as the medical history, age, body weight, family history, genetic composition, type of prior or concomitant treatment (if any), and other individual characteristics of the subject to be treated.

As used herein, the term "prophylactically effective amount" is intended to include an amount of the RNAi agent that, when administered to a subject suffering from an AGT-related disorder, is sufficient to prevent or ameliorate the disorder or one or more symptoms of the disorder. Ameliorating a disease includes slowing the progression of the disease or reducing the severity of the disease that develops at a later stage. The "prophylactically effective amount" may vary depending on the RNAi agent, how the agent is administered, the degree of disease risk, as well as the medical history, age, body weight, family history, genetic makeup, type of prior or concomitant treatment (if any), and other individual characteristics of the patient to be treated. As used herein, the term "pharmaceutically acceptable carrier" refers to a pharmaceutically acceptable material, composition, or vehicle, such as a liquid or solid filler, diluent, excipient, manufacturing aid (e.g., lubricant, magnesium talc, calcium stearate, zinc stearate, or stearic acid), or solvent encapsulating material (involved in carrying or transporting a subject compound from one organ or part of the body to another organ or part of the body). Each carrier must be "acceptable" in the sense that it is compatible with the other ingredients of the formulation and harmless to the subject being treated. Such carriers are known in the art. Pharmaceutically acceptable carriers include those used for administration by injection.

In one aspect, the present disclosure provides an oligonucleotide or a pharmaceutically acceptable salt thereof for reducing AGT expression, wherein the oligonucleotide described above comprises a sense strand and an antisense strand; the sense strand described above has a sequence having at least 80% or more sequence identity, preferably 85%, 90%, 95%, 96%, 97%, 98%, 99% or more sequence identity, to any one of the sequences set forth in SEQ ID NOs: 2-219, 789-804, and 868-871 or a fragment thereof, or a modified sequence of the sequence or the fragment thereof described above, and the antisense strand described above has a sequence having at least 80% or more sequence identity, preferably 85%, 90%, 95%, 96%, 97%, 98%, 99% or more sequence identity, to any one of the sequences set forth in SEQ ID NOs: 221-424, 805-821, and 872-875 or a fragment thereof, or a modified sequence of the sequence or the fragment thereof described above.

In some embodiments, each of the strands is independently 19 to 25 nucleotides in length.

In some embodiments, the antisense strand described above is 19 to 23 nucleotides in length.

In some embodiments, the sense strand described above is 19 to 23 nucleotides in length.

In some embodiments, the oligonucleotide described above comprises a 5' and/or 3'-overhang sequence that is one or more nucleotides in length, wherein the 5' and/or 3'-overhang sequence described above is present on the antisense strand and/or the sense strand. In one embodiment, the antisense strand of the oligonucleotide described above has an overhang of 1 to 10 nucleotides, for example, 1, 2, 3, 4, 5, 6, 7, 8, 9, or 10 nucleotides, at the 3' end or the 5' end. In one embodiment, the sense strand of the dsRNA has an overhang of 1 to 10 nucleotides, for example, 1, 2, 3, 4, 5, 6, 7, 8, 9, or 10 nucleotides, at the 3' end or the 5' end. In another embodiment, one or more nucleotides in the overhang are replaced with a nucleoside phosphorothioate.

In some embodiments, the antisense strand described above has an overhang.

In some embodiments, the sense strand described above has an overhang.

In some embodiments, the oligonucleotide described above comprises a 3'-overhang sequence that is two nucleotides in length. In some embodiments, the 3'-overhang sequence described above is present on the sense strand

described above; preferably, the overhang sequence described above is selected from: GG, GA, GC, UC, UG, UU, UA, CA, CC, CG, CU, AA, AG, AU, and AC.

In some embodiments, the 3'-overhang sequence described above is present on the antisense strand described above; preferably, the overhang sequence described above is selected from: UU, UC, UA, UG, GA, GG, GU, GC, TT, AG, AU, AA, AC, CA, CC, and U; more preferably, the overhang sequence described above is UU.

In some embodiments, the oligonucleotide described above comprises an antisense strand and a sense strand, each ranging from 19 to 23 nucleotides in length.

In some embodiments, the sense strand described above and the antisense strand described above form a duplex region.

In some embodiments, the sense strand described above and the antisense strand described above form a duplex structure with 19/21 pairing, 21/21 pairing, 21/23 pairing, or 23/23 pairing.

In some embodiments, the oligonucleotide described above comprises a 3'-overhang sequence that is two nucleotides in length, wherein the 3'-overhang sequence described above is present on the antisense strand described above, and wherein the sense strand described above is 19 nucleotides in length and the antisense strand described above is 21 nucleotides in length, such that the sense strand and the antisense strand described above form a duplex that is 19 nucleotides in length.

In some embodiments, the oligonucleotide described above comprises a 3'-overhang sequence that is two nucleotides in length, wherein the 3'-overhang sequence described above is present on both the antisense strand and the sense strand described above, and wherein the sense strand described above is 21 nucleotides in length and the antisense strand described above is 21 nucleotides in length, such that the sense strand and the antisense strand described above form a duplex that is 19 nucleotides in length.

In some embodiments, the oligonucleotide described above comprises a 3'-overhang sequence that is two nucleotides in length, wherein the 3'-overhang sequence described above is present on the antisense strand described above, and wherein the sense strand described above is 21 nucleotides in length and the antisense strand described above is 23 nucleotides in length, such that the sense strand and the antisense strand described above form a duplex that is 21 nucleotides in length.

In some embodiments, the oligonucleotide described above comprises a 3'-overhang sequence that is two nucleotides in length, wherein the 3'-overhang sequence described above is present on both the antisense strand and the sense strand described above, and wherein the sense strand described above is 23 nucleotides in length and the antisense strand described above is 23 nucleotides in length, such that the sense strand and the antisense strand described above form a duplex that is 21 nucleotides in length.

In some embodiments, the modified sequence of the sense strand described above comprises any one selected from the sequences set forth in SEQ ID NOs: 426-598, 822-838, and 876-886 described above.

In some embodiments, the modified sequence of the antisense strand described above comprises any one selected from the sequences set forth in SEQ ID NOs: 600-676, 678-788, 839-867, and 887-911 described above.

In some embodiments, the oligonucleotide or the pharmaceutically acceptable salt thereof described above is preferably prepared or synthesized in the form of a carboxylate, a sodium salt, a triethylamine salt, or other pharmaceutically acceptable salts.

In some embodiments, the oligonucleotide or the pharmaceutically acceptable salt thereof described above is more preferably a sodium salt or a triethylamine salt thereof.

In some embodiments, the sense strand described above comprises any one selected from the unmodified oligonucleotides set forth in 74, 101, 107, 108, 111-113, 129, 133, 135, 137, 148, 166, 178, 184, 789, 794, 798, 799, 800, 801, 802, 803, 804, and 870, or any one selected from the modified oligonucleotides set forth in SEQ ID NOs: 471, 477, 478, 481-483, 518, 536, 548, 554, 592-598, 822, 827, 831, 832, 833, 834, 835, 836, 837, and 838.

In some embodiments, the antisense strand described above comprises any one selected from the unmodified oligonucleotides set forth in SEQ ID NOs: 259, 281, 286, 311, 317, 318, 319, 334, 338, 340, 342, 353, 371, 383, 389, 805, 808, 811, 812, 813, 814, 815, 816, 817, 818, and 874, or any one selected from the modified oligonucleotides set forth in SEQ ID NOs: 643, 644, 645, 647, 648, 681, 678, 679, 680, 764, 610, 773, 774, 775, 776, 777, 778, 779, 780, 782, 783, 785, 839, 854, 857, 858, 859, 860, 861, 862, 863, and 864.

In some preferred embodiments, the oligonucleotide described above has a sense strand and an antisense strand, wherein the oligonucleotide described above has any one selected from the following combinations of sense and antisense strands:

(1) the sense strand comprises the sequence set forth in SEQ ID NO: 101, and the antisense strand comprises the sequence set forth in SEQ ID NO: 311;
(2) the sense strand comprises the sequence set forth in SEQ ID NO: 107, and the antisense strand comprises the sequence set forth in SEQ ID NO: 281;
(3) the sense strand comprises the sequence set forth in SEQ ID NO: 108, and the antisense strand comprises the

sequence set forth in SEQ ID NO: 286;
(4) the sense strand comprises the sequence set forth in SEQ ID NO: 111, and the antisense strand comprises the sequence set forth in SEQ ID NO: 317;
(5) the sense strand comprises the sequence set forth in SEQ ID NO: 112, and the antisense strand comprises the sequence set forth in SEQ ID NO: 318;
(6) the sense strand comprises the sequence set forth in SEQ ID NO: 113, and the antisense strand comprises the sequence set forth in SEQ ID NO: 319;
(7) the sense strand comprises the sequence set forth in SEQ ID NO: 129, and the antisense strand comprises the sequence set forth in SEQ ID NO: 334;
(8) the sense strand comprises the sequence set forth in SEQ ID NO: 133, and the antisense strand comprises the sequence set forth in SEQ ID NO: 338;
(9) the sense strand comprises the sequence set forth in SEQ ID NO: 135, and the antisense strand comprises the sequence set forth in SEQ ID NO: 340;
(10) the sense strand comprises the sequence set forth in SEQ ID NO: 137, and the antisense strand comprises the sequence set forth in SEQ ID NO: 342;
(11) the sense strand comprises the sequence set forth in SEQ ID NO: 148, and the antisense strand comprises the sequence set forth in SEQ ID NO: 353;
(12) the sense strand comprises the sequence set forth in SEQ ID NO: 166, and the antisense strand comprises the sequence set forth in SEQ ID NO: 371;
(13) the sense strand comprises the sequence set forth in SEQ ID NO: 178, and the antisense strand comprises the sequence set forth in SEQ ID NO: 383;
(14) the sense strand comprises the sequence set forth in SEQ ID NO: 184, and the antisense strand comprises the sequence set forth in SEQ ID NO: 389;
(15) the sense strand comprises the sequence set forth in SEQ ID NO: 113, and the antisense strand comprises the sequence set forth in SEQ ID NO: 808;
(16) the sense strand comprises the sequence set forth in SEQ ID NO: 113, and the antisense strand comprises the sequence set forth in SEQ ID NO: 811;
(17) the sense strand comprises the sequence set forth in SEQ ID NO: 113, and the antisense strand comprises the sequence set forth in SEQ ID NO: 818;
(18) the sense strand comprises the sequence set forth in SEQ ID NO: 789, and the antisense strand comprises the sequence set forth in SEQ ID NO: 805;
(19) the sense strand comprises the sequence set forth in SEQ ID NO: 794, and the antisense strand comprises the sequence set forth in SEQ ID NO: 805;
(20) the sense strand comprises the sequence set forth in SEQ ID NO: 798, and the antisense strand comprises the sequence set forth in SEQ ID NO: 805;
(21) the sense strand comprises the sequence set forth in SEQ ID NO: 799, and the antisense strand comprises the sequence set forth in SEQ ID NO: 812;
(22) the sense strand comprises the sequence set forth in SEQ ID NO: 800, and the antisense strand comprises the sequence set forth in SEQ ID NO: 813;
(23) the sense strand comprises the sequence set forth in SEQ ID NO: 801, and the antisense strand comprises the sequence set forth in SEQ ID NO: 814;
(24) the sense strand comprises the sequence set forth in SEQ ID NO: 802, and the antisense strand comprises the sequence set forth in SEQ ID NO: 815;
(25) the sense strand comprises the sequence set forth in SEQ ID NO: 803, and the antisense strand comprises the sequence set forth in SEQ ID NO: 816;
(26) the sense strand comprises the sequence set forth in SEQ ID NO: 804, and the antisense strand comprises the sequence set forth in SEQ ID NO: 817;
(27) the sense strand comprises the sequence set forth in SEQ ID NO: 868, and the antisense strand comprises the sequence set forth in SEQ ID NO: 872;
(28) the sense strand comprises the sequence set forth in SEQ ID NO: 869, and the antisense strand comprises the sequence set forth in SEQ ID NO: 873;
(29) the sense strand comprises the sequence set forth in SEQ ID NO: 870, and the antisense strand comprises the sequence set forth in SEQ ID NO: 874;
(30) the sense strand comprises the sequence set forth in SEQ ID NO: 871, and the antisense strand comprises the sequence set forth in SEQ ID NO: 875; and
(31) the sense strand comprises the sequence set forth in SEQ ID NO: 74, and the antisense strand comprises the sequence set forth in SEQ ID NO: 259;

wherein each of the strands is independently 19 to 25 nucleotides in length.

In some embodiments, the oligonucleotide described above comprises at least one modified nucleotide.

In some embodiments, at least one of the modified nucleotide(s) described above is selected from the group consisting of a deoxynucleotide, a 3'-terminal deoxythymidine (dT) nucleotide, a 2'-O-methyl-modified nucleotide, a 2'-fluoro-modified nucleotide, a 2'-deoxy-modified nucleotide, a locked nucleotide, a 2'-5'-linked ribonucleotide (3'-RNA), an unlocked nucleotide, a conformationally restricted nucleotide, a constrained ethyl nucleotide, an abasic nucleotide, a 2'-amino-modified nucleotide, a 2'-O-allyl-modified nucleotide, a 2'-O-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nucleotide comprising a non-natural base, a tetrahydropyran-modified nucleotide, a 1,5-anhydrohexitol-modified nucleotide, a cyclohexenyl-modified nucleotide, a nucleotide comprising a phosphorothioate group, a nucleotide comprising a methylphosphonate group, a nucleotide comprising a 5'-phosphate, a nucleotide comprising a 5'-phosphate mimic, a vinylphosphonate nucleotide, a thermolabile nucleotide, a glycol-modified nucleotide, a nucleotide comprising a 2'-phosphate, and a 2-O-(N-methylacetamide)-modified nucleotide, and a combination thereof.

In some embodiments, at least one of the modified nucleotide(s) described above is selected from the group consisting of LNA, HNA, CeNA, 2'-methoxyethyl, 2'-O-alkyl, 2'-O-allyl, 2'-C-allyl, 2'-fluoro, 2'-deoxy, 2'-hydroxy, and ethylene glycol, and a combination thereof.

In some embodiments, at least one of the modified nucleotide(s) described above is selected from the group consisting of a deoxynucleotide, a 2'-O-methyl-modified nucleotide, a 2'-fluoro-modified nucleotide, a 2'-deoxy-modified nucleotide, an ethylene glycol-modified nucleotide (GNA), a nucleotide comprising a 2'-phosphate, and a nucleotide comprising a phosphorothioate group, and a combination thereof.

In some embodiments, the oligonucleotide described above comprises at least one 2'-modified nucleotide. For example, the nucleotide may be one or more selected from a 2'-alkoxy-modified nucleotide, a 2'-substituted alkoxy-modified nucleotide, a 2'-alkyl-modified nucleotide, a 2'-substituted alkyl-modified nucleotide, a 2'-fluoro-modified nucleotide, a 2'-acylamino-modified nucleotide, a 2'-deoxy-modified nucleotide, a 2'-O-allyl-modified nucleotide, a 2'-O-alkyl-modified nucleotide, a 2'-hydroxy-modified nucleotide, a 2'-methoxyethyl-modified nucleotide, a 2'-amino-modified nucleotide, a 2'-substituted amino-modified nucleotide, a 2'-deoxynucleotide, a nucleotide comprising a 2'-phosphate, and a 2'-O-(N-methylacetamide)-modified nucleotide. For example, C1-C3 alkoxy (e.g., methoxy); substituted alkoxy (e.g., $C_1$-$C_3$ alkoxy-substituted $C_1$-$C_3$ alkoxy, such as methoxyethoxy); alkyl (e.g., $C_1$-$C_3$ alkyl, such as methyl); substituted alkyl (e.g., $C_1$-$C_3$ alkoxy-substituted $C_1$-$C_3$ alkyl, such as methoxymethyl or methoxyethyl); amino ($-NH_2$); or substituted amino (e.g., $C_1$-$C_3$ alkyl mono- or di-substituted amino, such as methylamino or ethylamino), but not limited thereto.

In some embodiments, the 2'-modified nucleotide described above is one or more selected from a 2'-alkoxy-modified nucleotide, a 2'-substituted alkoxy-modified nucleotide, a 2'-alkyl-modified nucleotide, a 2'-substituted alkyl-modified nucleotide, a 2'-amino-modified nucleotide, a 2'-substituted amino-modified nucleotide, and a 2'-deoxynucleotide.

In some embodiments, the 2'-modification described above is a modification selected from 2'-methoxy, 2'-acetylamino, 2'-aminoethyl, 2'-fluoro, 2'-O-methoxyethyl, and 2'-fluoro-β-d-arabinonucleic acid.

In some embodiments, the modification described above is a modification selected from a 5'-phosphate analog modification, 2'-methoxy ($CH_3O$-, m), 2'-fluoro (f), 2'-acetylamino ($CH_3CO$-NH-), and phosphorothioate (s).

In some embodiments, the 2'-modification described above is a 2'-methoxy modification.

In some embodiments, the 2'-modification described above is 2'-acetylamino.

In some embodiments, all nucleotides of the oligonucleotide described above are modified.

In some embodiments, the oligonucleotide described above may comprise a glycol nucleic acid (GNA) modification.

In some embodiments, the glycol nucleic acid (GNA) modification described above is selected from an adenosine-glycol nucleic acid, a cytidine-glycol nucleic acid, a thymidine-glycol nucleic acid, and a guanosine-glycol nucleic acid.

In some embodiments, the glycol nucleic acid (GNA) modification described above is selected from a thymidine-glycol nucleic acid S-isomer (Tgn) represented by formula (I), a cytidine-glycol nucleic acid S-isomer (Cgn) represented by formula (II), an adenosine-glycol nucleic acid S-isomer (Agn) represented by formula (III), and a guanosine-glycol nucleic acid S-isomer (Ggn) represented by formula (IV):

Tgn

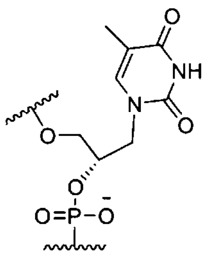

(I) ,

Cgn

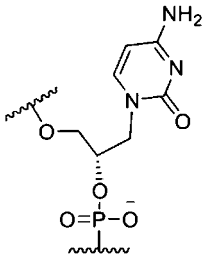

(II) ,

Agn

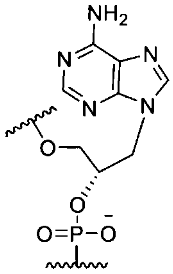

(III) , and

Ggn

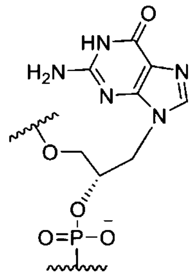

(IV) .

In some embodiments, the oligonucleotide described above may comprise a 2'-5'-phosphodiester linkage.

In some embodiments, the oligonucleotide described above comprises a modification selected from uridine-2'-phosphate (U-2'5') represented by formula (V), guanosine-2'-phosphate (G-2'5') represented by formula (VI), cytidine-2'-phosphate (C-2'5') represented by formula (VII), adenosine-2'-phosphate (A-2'5') represented by formula (VIII), and thymidine-2'-phosphate (T-2'5') represented by formula (IX):

(U-2'5')

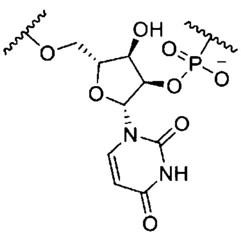

(V) ,

(G-2'5')

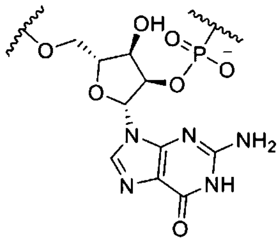

(VI) ,

(C-2'5')

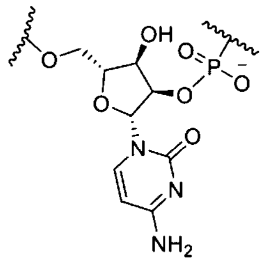

(VII) ,

(A-2'5')

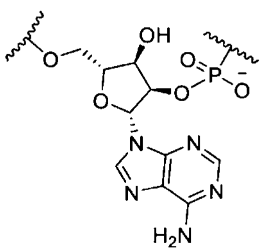

(VIII) , and

(T-2'5')

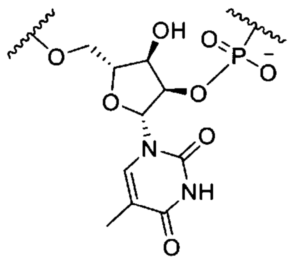

(IX) .

In some embodiments, the oligonucleotide described above further comprises a 5'-phosphate analog-modified nucleotide.

In some embodiments, the phosphate analog described above is an oxymethylphosphonate, a vinylphosphonate, or a malonylphosphonate.

In some embodiments, the 4'-carbon of the sugar of the 5'-nucleotide of the antisense strand described above comprises a phosphate analog.

In some embodiments, the 5'-phosphate analog-modified nucleotide described above is APU represented by formula (X); in some embodiments, the 5'-phosphate analog-modified nucleotide described above is VPUm represented by formula (XI):

APU

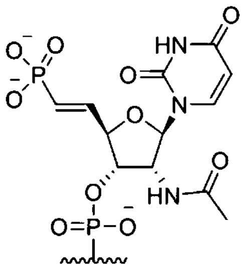

(X);

VPUm

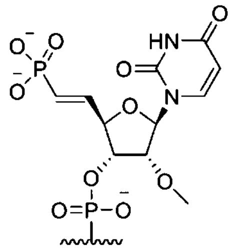

(XI).

In some embodiments, the oligonucleotide described above comprises at least one modified internucleotide linkage.

In some embodiments, the at least one modified internucleotide linkage described above is a phosphorothioate linkage. The phosphorothioate internucleotide linkage modification may occur at any position on any nucleotide in the sense strand, the antisense strand, or both strands. For example, the internucleotide linkage modification may occur on each nucleotide in the sense strand or the antisense strand; each internucleotide linkage modification may occur in an alternating pattern on the sense strand or the antisense strand; or the sense strand or the antisense strand may contain two types of internucleotide linkage modifications in an alternating pattern. The alternating pattern of the internucleotide linkage modifications on the sense strand may be identical to or different from that on the antisense strand, and the alternating pattern of the internucleotide linkage modifications on the sense strand may have an offset relative to the alternating pattern of the internucleotide linkages on the antisense strand. In one embodiment, the double-stranded RNAi agent comprises 6 to 8 phosphorothioate internucleotide linkages. In some embodiments, the antisense strand comprises two phosphorothioate internucleotide linkages at the 5' end and two phosphorothioate internucleotide linkages at the 3' end, and the sense strand comprises at least two phosphorothioate internucleotide linkages at the 5' end or the 3' end.

In some preferred embodiments, the oligonucleotide described above comprises any one selected from the following combinations of sense and antisense strands:

(1) the sense strand comprises the sequence set forth in SEQ ID NO: 477, and the antisense strand comprises the sequence set forth in SEQ ID NO: 643;
(2) the sense strand comprises the sequence set forth in SEQ ID NO: 471, and the antisense strand comprises the sequence set forth in SEQ ID NO: 644;
(3) the sense strand comprises the sequence set forth in SEQ ID NO: 478, and the antisense strand comprises the sequence set forth in SEQ ID NO: 645;
(4) the sense strand comprises the sequence set forth in SEQ ID NO: 482, and the antisense strand comprises the sequence set forth in SEQ ID NO: 647;
(5) the sense strand comprises the sequence set forth in SEQ ID NO: 483, and the antisense strand comprises the sequence set forth in SEQ ID NO: 648;
(6) the sense strand comprises the sequence set forth in SEQ ID NO: 481, and the antisense strand comprises the sequence set forth in SEQ ID NO: 681;
(7) the sense strand comprises the sequence set forth in SEQ ID NO: 471, and the antisense strand comprises the sequence set forth in SEQ ID NO: 678;
(8) the sense strand comprises the sequence set forth in SEQ ID NO: 478, and the antisense strand comprises the sequence set forth in SEQ ID NO: 679;
(9) the sense strand comprises the sequence set forth in SEQ ID NO: 483, and the antisense strand comprises the sequence set forth in SEQ ID NO: 680;
(10) the sense strand comprises the sequence set forth in SEQ ID NO: 594, and the antisense strand comprises the sequence set forth in SEQ ID NO: 764;
(11) the sense strand comprises the sequence set forth in SEQ ID NO: 593, and the antisense strand comprises the sequence set forth in SEQ ID NO: 610;
(12) the sense strand comprises the sequence set forth in SEQ ID NO: 592, and the antisense strand comprises the sequence set forth in SEQ ID NO: 773;
(13) the sense strand comprises the sequence set forth in SEQ ID NO: 592, and the antisense strand comprises the sequence set forth in SEQ ID NO: 774;
(14) the sense strand comprises the sequence set forth in SEQ ID NO: 592, and the antisense strand comprises the sequence set forth in SEQ ID NO: 775;
(15) the sense strand comprises the sequence set forth in SEQ ID NO: 595, and the antisense strand comprises the sequence set forth in SEQ ID NO: 776;

(16) the sense strand comprises the sequence set forth in SEQ ID NO: 596, and the antisense strand comprises the sequence set forth in SEQ ID NO: 777;
(17) the sense strand comprises the sequence set forth in SEQ ID NO: 597, and the antisense strand comprises the sequence set forth in SEQ ID NO: 778;
(18) the sense strand comprises the sequence set forth in SEQ ID NO: 598, and the antisense strand comprises the sequence set forth in SEQ ID NO: 779;
(19) the sense strand comprises the sequence set forth in SEQ ID NO: 518, and the antisense strand comprises the sequence set forth in SEQ ID NO: 780;
(20) the sense strand comprises the sequence set forth in SEQ ID NO: 536, and the antisense strand comprises the sequence set forth in SEQ ID NO: 782;
(21) the sense strand comprises the sequence set forth in SEQ ID NO: 548, and the antisense strand comprises the sequence set forth in SEQ ID NO: 783;
(22) the sense strand comprises the sequence set forth in SEQ ID NO: 554, and the antisense strand comprises the sequence set forth in SEQ ID NO: 785;
(23) the sense strand comprises the sequence set forth in SEQ ID NO: 592, and the antisense strand comprises the sequence set forth in SEQ ID NO: 854;
(24) the sense strand comprises the sequence set forth in SEQ ID NO: 592, and the antisense strand comprises the sequence set forth in SEQ ID NO: 857;
(25) the sense strand comprises the sequence set forth in SEQ ID NO: 592, and the antisense strand comprises the sequence set forth in SEQ ID NO: 864;
(26) the sense strand comprises the sequence set forth in SEQ ID NO: 822, and the antisense strand comprises the sequence set forth in SEQ ID NO: 839;
(27) the sense strand comprises the sequence set forth in SEQ ID NO: 827, and the antisense strand comprises the sequence set forth in SEQ ID NO: 839;
(28) the sense strand comprises the sequence set forth in SEQ ID NO: 831, and the antisense strand comprises the sequence set forth in SEQ ID NO: 839;
(29) the sense strand comprises the sequence set forth in SEQ ID NO: 598, and the antisense strand comprises the sequence set forth in SEQ ID NO: 840;
(30) the sense strand comprises the sequence set forth in SEQ ID NO: 832, and the antisense strand comprises the sequence set forth in SEQ ID NO: 841;
(31) the sense strand comprises the sequence set forth in SEQ ID NO: 832, and the antisense strand comprises the sequence set forth in SEQ ID NO: 842;
(32) the sense strand comprises the sequence set forth in SEQ ID NO: 832, and the antisense strand comprises the sequence set forth in SEQ ID NO: 848;
(33) the sense strand comprises the sequence set forth in SEQ ID NO: 832, and the antisense strand comprises the sequence set forth in SEQ ID NO: 849;
(34) the sense strand comprises the sequence set forth in SEQ ID NO: 832, and the antisense strand comprises the sequence set forth in SEQ ID NO: 850;
(35) the sense strand comprises the sequence set forth in SEQ ID NO: 833, and the antisense strand comprises the sequence set forth in SEQ ID NO: 858;
(36) the sense strand comprises the sequence set forth in SEQ ID NO: 834, and the antisense strand comprises the sequence set forth in SEQ ID NO: 859;
(37) the sense strand comprises the sequence set forth in SEQ ID NO: 835, and the antisense strand comprises the sequence set forth in SEQ ID NO: 860;
(38) the sense strand comprises the sequence set forth in SEQ ID NO: 836, and the antisense strand comprises the sequence set forth in SEQ ID NO: 861;
(39) the sense strand comprises the sequence set forth in SEQ ID NO: 837, and the antisense strand comprises the sequence set forth in SEQ ID NO: 862;
(40) the sense strand comprises the sequence set forth in SEQ ID NO: 838, and the antisense strand comprises the sequence set forth in SEQ ID NO: 863;
(41) the sense strand comprises the sequence set forth in SEQ ID NO: 882, and the antisense strand comprises the sequence set forth in SEQ ID NO: 894;
(42) the sense strand comprises the sequence set forth in SEQ ID NO: 882, and the antisense strand comprises the sequence set forth in SEQ ID NO: 902;
(43) the sense strand comprises the sequence set forth in SEQ ID NO: 884, and the antisense strand comprises the sequence set forth in SEQ ID NO: 896;
(44) the sense strand comprises the sequence set forth in SEQ ID NO: 884, and the antisense strand comprises the sequence set forth in SEQ ID NO: 903;

(45) the sense strand comprises the sequence set forth in SEQ ID NO: 592, and the antisense strand comprises the sequence set forth in SEQ ID NO: 905;
(46) the sense strand comprises the sequence set forth in SEQ ID NO: 592, and the antisense strand comprises the sequence set forth in SEQ ID NO: 907;
(47) the sense strand comprises the sequence set forth in SEQ ID NO: 882, and the antisense strand comprises the sequence set forth in SEQ ID NO: 910; and
(48) the sense strand comprises the sequence set forth in SEQ ID NO: 882, and the antisense strand comprises the sequence set forth in SEQ ID NO: 911;

wherein each of the strands is independently 19 to 25 nucleotides in length.

In some preferred embodiments, the oligonucleotide described above comprises any one selected from the following combinations of sense and antisense strands:

(1) the sense strand set forth in SEQ ID NO: 594 and the antisense strand set forth in SEQ ID NO: 764;
(2) the sense strand set forth in SEQ ID NO: 593 and the antisense strand set forth in SEQ ID NO: 610;
(3) the sense strand set forth in SEQ ID NO: 592 and the antisense strand set forth in SEQ ID NO: 773;
(4) the sense strand set forth in SEQ ID NO: 592 and the antisense strand set forth in SEQ ID NO: 774;
(5) the sense strand set forth in SEQ ID NO: 592 and the antisense strand set forth in SEQ ID NO: 775;
(6) the sense strand set forth in SEQ ID NO: 595 and the antisense strand set forth in SEQ ID NO: 776;
(7) the sense strand set forth in SEQ ID NO: 596 and the antisense strand set forth in SEQ ID NO: 777;
(8) the sense strand set forth in SEQ ID NO: 597 and the antisense strand set forth in SEQ ID NO: 778;
(9) the sense strand set forth in SEQ ID NO: 598 and the antisense strand set forth in SEQ ID NO: 779;
(10) the sense strand set forth in SEQ ID NO: 518 and the antisense strand set forth in SEQ ID NO: 780;
(11) the sense strand set forth in SEQ ID NO: 536 and the antisense strand set forth in SEQ ID NO: 782;
(12) the sense strand set forth in SEQ ID NO: 548 and the antisense strand set forth in SEQ ID NO: 783;
(13) the sense strand set forth in SEQ ID NO: 554 and the antisense strand set forth in SEQ ID NO: 785;
(14) the sense strand set forth in SEQ ID NO: 592 and the antisense strand set forth in SEQ ID NO: 854;
(15) the sense strand set forth in SEQ ID NO: 592 and the antisense strand set forth in SEQ ID NO: 857;
(16) the sense strand set forth in SEQ ID NO: 592 and the antisense strand set forth in SEQ ID NO: 864;
(17) the sense strand set forth in SEQ ID NO: 822 and the antisense strand set forth in SEQ ID NO: 839;
(18) the sense strand set forth in SEQ ID NO: 827 and the antisense strand set forth in SEQ ID NO: 839;
(19) the sense strand set forth in SEQ ID NO: 831 and the antisense strand set forth in SEQ ID NO: 839;
(20) the sense strand set forth in SEQ ID NO: 598 and the antisense strand set forth in SEQ ID NO: 840;
(21) the sense strand set forth in SEQ ID NO: 832 and the antisense strand set forth in SEQ ID NO: 841;
(22) the sense strand set forth in SEQ ID NO: 832 and the antisense strand set forth in SEQ ID NO: 842;
(23) the sense strand set forth in SEQ ID NO: 832 and the antisense strand set forth in SEQ ID NO: 848;
(24) the sense strand set forth in SEQ ID NO: 832 and the antisense strand set forth in SEQ ID NO: 849;
(25) the sense strand set forth in SEQ ID NO: 832 and the antisense strand set forth in SEQ ID NO: 850;
(26) the sense strand set forth in SEQ ID NO: 833 and the antisense strand set forth in SEQ ID NO: 858;
(27) the sense strand set forth in SEQ ID NO: 834 and the antisense strand set forth in SEQ ID NO: 859;
(28) the sense strand set forth in SEQ ID NO: 835 and the antisense strand set forth in SEQ ID NO: 860;
(29) the sense strand set forth in SEQ ID NO: 836 and the antisense strand set forth in SEQ ID NO: 861;
(30) the sense strand set forth in SEQ ID NO: 837 and the antisense strand set forth in SEQ ID NO: 862;
(31) the sense strand set forth in SEQ ID NO: 838 and the antisense strand set forth in SEQ ID NO: 863;
(32) the sense strand set forth in SEQ ID NO: 882 and the antisense strand set forth in SEQ ID NO: 894;
(33) the sense strand set forth in SEQ ID NO: 882 and the antisense strand set forth in SEQ ID NO: 902;
(34) the sense strand set forth in SEQ ID NO: 884 and the antisense strand set forth in SEQ ID NO: 896;
(35) the sense strand set forth in SEQ ID NO: 884 and the antisense strand set forth in SEQ ID NO: 903;
(36) the sense strand set forth in SEQ ID NO: 592 and the antisense strand set forth in SEQ ID NO: 905;
(37) the sense strand set forth in SEQ ID NO: 592 and the antisense strand set forth in SEQ ID NO: 907;
(38) the sense strand set forth in SEQ ID NO: 882 and the antisense strand set forth in SEQ ID NO: 910; and
(39) the sense strand set forth in SEQ ID NO: 882 and the antisense strand set forth in SEQ ID NO: 911.

In some preferred embodiments, the oligonucleotide described above comprises any one selected from the following combinations of sense and antisense strands:

(1) the sense strand set forth in SEQ ID NO: 594 and the antisense strand set forth in SEQ ID NO: 764;
(2) the sense strand set forth in SEQ ID NO: 592 and the antisense strand set forth in SEQ ID NO: 773;

(3) the sense strand set forth in SEQ ID NO: 592 and the antisense strand set forth in SEQ ID NO: 774;
(4) the sense strand set forth in SEQ ID NO: 592 and the antisense strand set forth in SEQ ID NO: 775;
(5) the sense strand set forth in SEQ ID NO: 595 and the antisense strand set forth in SEQ ID NO: 776;
(6) the sense strand set forth in SEQ ID NO: 598 and the antisense strand set forth in SEQ ID NO: 779;
(7) the sense strand set forth in SEQ ID NO: 518 and the antisense strand set forth in SEQ ID NO: 780;
(8) the sense strand set forth in SEQ ID NO: 592 and the antisense strand set forth in SEQ ID NO: 854;
(9) the sense strand set forth in SEQ ID NO: 592 and the antisense strand set forth in SEQ ID NO: 857;
(10) the sense strand set forth in SEQ ID NO: 592 and the antisense strand set forth in SEQ ID NO: 864;
(11) the sense strand set forth in SEQ ID NO: 822 and the antisense strand set forth in SEQ ID NO: 839;
(12) the sense strand set forth in SEQ ID NO: 827 and the antisense strand set forth in SEQ ID NO: 839;
(13) the sense strand set forth in SEQ ID NO: 831 and the antisense strand set forth in SEQ ID NO: 839;
(14) the sense strand set forth in SEQ ID NO: 598 and the antisense strand set forth in SEQ ID NO: 840;
(15) the sense strand set forth in SEQ ID NO: 832 and the antisense strand set forth in SEQ ID NO: 841;
(16) the sense strand set forth in SEQ ID NO: 832 and the antisense strand set forth in SEQ ID NO: 842;
(17) the sense strand set forth in SEQ ID NO: 832 and the antisense strand set forth in SEQ ID NO: 848;
(18) the sense strand set forth in SEQ ID NO: 832 and the antisense strand set forth in SEQ ID NO: 849;
(19) the sense strand set forth in SEQ ID NO: 832 and the antisense strand set forth in SEQ ID NO: 850;
(20) the sense strand set forth in SEQ ID NO: 833 and the antisense strand set forth in SEQ ID NO: 858;
(21) the sense strand set forth in SEQ ID NO: 834 and the antisense strand set forth in SEQ ID NO: 859;
(22) the sense strand set forth in SEQ ID NO: 835 and the antisense strand set forth in SEQ ID NO: 860;
(23) the sense strand set forth in SEQ ID NO: 836 and the antisense strand set forth in SEQ ID NO: 861;
(24) the sense strand set forth in SEQ ID NO: 837 and the antisense strand set forth in SEQ ID NO: 862;
(25) the sense strand set forth in SEQ ID NO: 838 and the antisense strand set forth in SEQ ID NO: 863;
(26) the sense strand set forth in SEQ ID NO: 882 and the antisense strand set forth in SEQ ID NO: 894;
(27) the sense strand set forth in SEQ ID NO: 882 and the antisense strand set forth in SEQ ID NO: 902;
(28) the sense strand set forth in SEQ ID NO: 884 and the antisense strand set forth in SEQ ID NO: 896;
(29) the sense strand set forth in SEQ ID NO: 884 and the antisense strand set forth in SEQ ID NO: 903;
(30) the sense strand set forth in SEQ ID NO: 592 and the antisense strand set forth in SEQ ID NO: 905;
(31) the sense strand set forth in SEQ ID NO: 592 and the antisense strand set forth in SEQ ID NO: 907;
(32) the sense strand set forth in SEQ ID NO: 882 and the antisense strand set forth in SEQ ID NO: 910; and
(33) the sense strand set forth in SEQ ID NO: 882 and the antisense strand set forth in SEQ ID NO: 911.

In some embodiments, at least one nucleotide of the oligonucleotide or the salt thereof described above is conjugated to one or more targeting ligands to form an siRNA conjugate. The siRNA conjugate described above contains the siRNA described above and a conjugated group conjugated to the siRNA. The term "oligonucleotide salt" refers to an oligonucleotide compound in a salt form. Oligonucleotide salts include salts of conjugated oligonucleotide compounds and salts of unconjugated oligonucleotide compounds. Oligonucleotide salts are advantageously present in a solid powder form.

In general, the conjugated group described above comprises at least one pharmaceutically acceptable targeting ligand and optionally a linker, and the siRNA described above, the linker described above, and the targeting ligand described above are linked sequentially. The targeting group may be a ligand conventionally used in the field of siRNA administration, such as the various ligands described in WO2009082607A2, the entire disclosure of which is incorporated herein by reference. In some embodiments, the number of the targeting ligands described above is 2 to 4. The siRNA molecule described above may be non-covalently or covalently conjugated to the conjugated group described above; for example, it may be covalently conjugated to the conjugated group described above. The conjugation site between the siRNA and the conjugated group may be at the 3' end or the 5' end of the sense strand or the antisense strand of the siRNA, or in the internal sequence of the siRNA. In some embodiments, the conjugation site between the siRNA and the conjugated group described above is at the 3' end or the 5' end of the sense strand of the siRNA. In some embodiments, the conjugation site between the siRNA and the conjugated group described above is at the 3' end or the 5' end of the antisense strand of the siRNA. In some preferred embodiments, the conjugation site between the siRNA and the conjugated group described above is at the 3' end of the sense strand of the siRNA.

In some embodiments, the targeting ligands comprise an asialoglycoprotein receptor ligand. In some embodiments, the asialoglycoprotein receptor ligand comprises or consists of one or more galactose derivatives. As used in the present disclosure, the term "galactose derivative" includes galactose and derivatives of galactose that have an affinity for the asialoglycoprotein receptor equal to or greater than that of galactose. Galactose derivatives include, but are not limited to, galactose, galactosamine, N-formylgalactosamine, N-acetylgalactosamine, N-propionyl-galactosamine, N-n-butyryl-galactosamine, and N-isobutyrylgalactosamine. Galactose derivatives and galactose derivative clusters that can be used for directing oligonucleotides and other molecules to the liver *in vivo* are known in the art. Galactose derivatives have been

used to direct molecules to hepatocytes *in vivo* through their binding to the asialoglycoprotein receptor (ASGPr) expressed on the surface of the hepatocytes. The binding of ASGPr ligand(s) to ASGPr(s) facilitates cell-specific targeting of hepatocytes and the endocytosis of molecules into hepatocytes. The ASGPr ligand may be a monomer (e.g., having a single galactose derivative) or a multimer (e.g., having multiple galactose derivatives). Galactose derivatives or galactose derivative clusters may be linked to the 3' end or the 5' end of the siRNA using methods known in the art.

In some embodiments, the pharmaceutically acceptable targeting ligands in the siRNA conjugate described above may be galactose or N-acetylgalactosamine (GalNAc), wherein the galactose or N-acetylgalactosamine molecules may be monovalent, bivalent, trivalent, or tetravalent. It should be understood that the "monovalent, bivalent, trivalent, or tetravalent" refers to that after an siRNA molecule and a conjugated group containing a galactose or N-acetylgalactosamine molecule as a targeting ligand form an siRNA conjugate, the molar ratio of the siRNA molecule to the galactose or N-acetylgalactosamine molecule in the siRNA conjugate is 1:1, 1:2, 1:3, or 1:4, respectively. In some embodiments, the pharmaceutically acceptable targeting ligands are N-acetylgalactosamine. In some embodiments, when the siRNA described in the present disclosure is conjugated to a conjugated group containing N-acetylgalactosamine, the N-acetylgalactosamine molecule is trivalent or tetravalent. In some embodiments, when the siRNA described in the present disclosure is conjugated to a conjugated group containing N-acetylgalactosamine, the N-acetylgalactosamine molecule is trivalent.

In some embodiments, the targeting ligands are selected from carbohydrates, amino sugars, cholesterol, polypeptides, and lipids.

In some embodiments, the targeting ligands comprise a N-acetylgalactosamine (GalNAc) moiety.

In some embodiments, the GalNAc moiety described above is a monovalent GalNAc moiety, a bivalent GalNAc moiety, a trivalent GalNAc moiety, or a tetravalent GalNAc moiety.

In some embodiments, the targeting ligands are A1 or L96. A1 is a GalNAc-targeting ligand represented by formula (XII), and L96 is N-[tris(GalNAc-alkyl)amidodecanoyl]-4-hydroxyprolinol Hyp-(GalNAc-alkyl)3 represented by formula (XIII).

A1

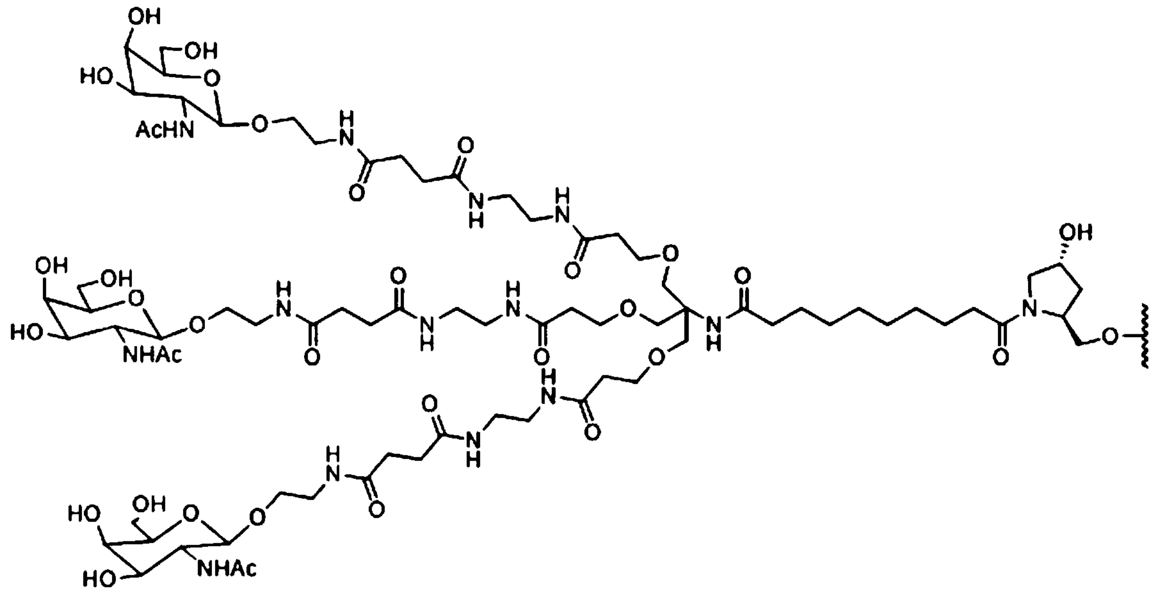

(XII)

L96

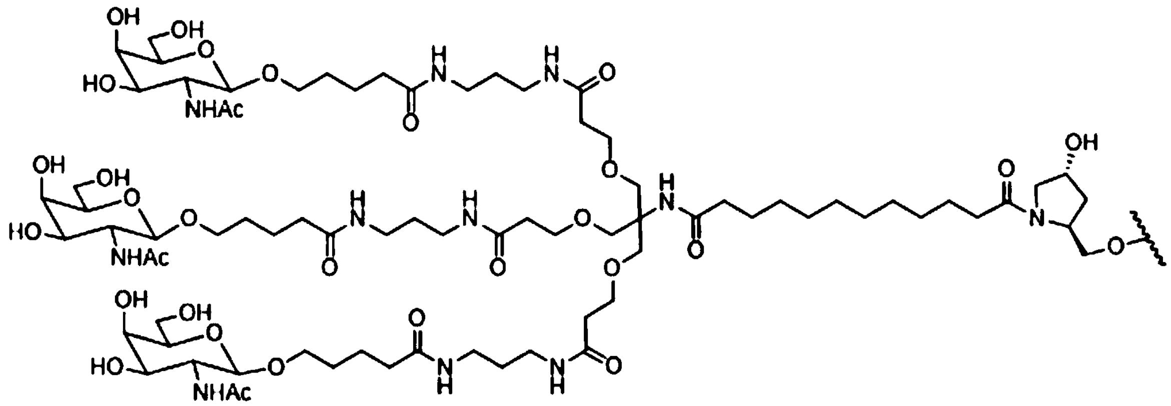

(XIII)

In another aspect, the present disclosure provides a composition, comprising the oligonucleotide or the pharmaceutically acceptable salt thereof described above, and optionally a pharmaceutically acceptable carrier.

In some embodiments, a dosage form of the composition described above is an oral agent, an intravenous injection, a subcutaneous injection, or an intramuscular injection.

In some preferred embodiments of the present disclosure, the dosage form of the composition described above is a subcutaneous injection.

In some embodiments, the combination described above further comprises an additional drug for treating or/and preventing an AGT-related disease.

In another aspect, the present disclosure provides use of the oligonucleotide or the pharmaceutically acceptable salt thereof, or the composition described above in the preparation of a medicament for treating or/and preventing an AGT-related disease. Various formulations have been developed to facilitate the use of oligonucleotides. For example, oligonucleotides can be delivered to a subject or cellular environment using formulations that minimize degradation, facilitate delivery and/or uptake, or provide another beneficial property to the oligonucleotide in the formulation. In some embodiments, provided herein is a composition comprising an oligonucleotide (e.g., a single-stranded or double-stranded oligonucleotide) for reducing AGT expression. Such compositions can be suitably formulated such that, when administered to a subject (either directly into the environment of target cells or systemically), a sufficient portion of the oligonucleotide enters the cells to reduce AGT expression. Any one of various suitable oligonucleotide formulations can be used to deliver oligonucleotides for reducing AGT, as disclosed herein. In some embodiments, the oligonucleotide or the conjugate thereof is formulated in a buffer solution, such as a phosphate-buffered saline solution, a liposome, a micellar structure, and a shell. The buffer solution may be selected from a solution of acetate, citrate, prolamin, carbonate, or phosphate, or any combination thereof. In some embodiments, the naked (i.e., without a delivery agent) oligonucleotide or the conjugate thereof is formulated in water or an aqueous solution (e.g., pH-adjusted water). In some embodiments, the naked oligonucleotide or the conjugate thereof is formulated in an alkaline buffered aqueous solution (e.g., PBS).

Formulations of oligonucleotides with cationic lipids can be used to facilitate the transfection of the oligonucleotides into cells. For example, cationic lipids such as lipofectin, cationic glycerol derivatives, and polycationic molecules (e.g., polylysine) can be used.

Accordingly, in some embodiments, the formulation comprises a lipid nanoparticle. In some embodiments, the excipient comprises a liposome, a lipid, a lipid complex, a microsphere, a microparticle, a nanosphere, or a nanoparticle, or may be otherwise formulated for administration to a cell, a tissue, an organ, or the body of a subject in need thereof.

In some embodiments, a formulation as disclosed herein comprises an excipient. In some embodiments, the excipient confers to a composition improved stability, improved absorption, improved solubility, and/or therapeutic enhancement of the active ingredient. In some embodiments, the excipient is a buffering agent (e.g., sodium citrate, sodium phosphate, a tris base, or sodium hydroxide) or a vehicle (e.g., a buffered solution, petrolatum, dimethyl sulfoxide, or mineral oil). In some embodiments, an oligonucleotide is lyophilized for extending its shelf-life and then made into a solution before use (e.g., administration to a subject). Accordingly, an excipient in a composition comprising any one of the oligonucleotides described herein may be a lyoprotectant (e.g., mannitol, lactose, polyethylene glycol, or polyvinylpyrrolidone) or a collapse temperature modifier (e.g., dextran, ficoll, or gelatin).

In some embodiments, the pharmaceutical composition is formulated to be compatible with its intended route of administration. Examples of routes of administration include parenteral, e.g., intravenous, intradermal, subcutaneous, oral (e.g., inhalation), transdermal (topical), transmucosal, and rectal administration. Typically, the route of administration is intravenous or subcutaneous.

Pharmaceutical compositions suitable for injectable use include sterile aqueous solutions (in the case of being water soluble) or dispersions and sterile powders for the extemporaneous preparation of sterile injectable solutions or dispersions. For intravenous or subcutaneous administration, suitable carriers include physiological saline, bacteriostatic

water, Cremophor EL.TM. (BASF, Parsippany, N.J.), or phosphate-buffered saline (PBS). The aforementioned carrier may be a solvent or dispersion medium containing, for example, water, ethanol, polyol (e.g., glycerol, propylene glycol, and liquid polyethylene glycol), and suitable mixtures thereof. In many cases, it is preferable to include isotonic agents, for example, sugars, polyols such as mannitol, sorbitol, and sodium chloride in the composition. Sterile injectable solutions can be prepared by incorporating a required amount of an oligonucleotide and one or a combination of the ingredients enumerated above, as required, in a selected solvent and then performing filter sterilization.

In some embodiments, the composition may comprise at least about 0.1% or more of a therapeutic agent (e.g., an oligonucleotide for reducing AGT expression), although the percentage of one or more active ingredients may be between about 1% and about 80% or more of the weight or volume of the total composition. Factors such as solubility, bioavailability, biological half-life, route of administration, product shelf life, and other pharmacological considerations will be contemplated by those skilled in the art of preparing such pharmaceutical formulations, and thus, a variety of dosages and treatment regimens may be desirable.

Even though many embodiments relate to liver-targeted delivery of any of the oligonucleotides disclosed herein, targeting other tissues is also contemplated.

In some embodiments, administration of an oligonucleotide as described herein results in a reduction in the level of AGT expression in a cell. In some embodiments, the reduction in the level of AGT expression may be a reduction to 1% or lower, 5% or lower, 10% or lower, 15% or lower, 20% or lower, 25% or lower, 30% or lower, 35% or lower, 40% or lower, 45% or lower, 50% or lower, 55% or lower, 60% or lower, 70% or lower, 80% or lower, or 90% or lower compared to an appropriate control level of AGT. The appropriate control level may be a level of AGT expression in a cell or population of cells that has not come into contact with an oligonucleotide as described herein. In some embodiments, the effect of delivery of an oligonucleotide to a cell according to a method disclosed herein is evaluated after a finite period of time. For example, blood AGT levels may be analyzed at least 8 hours, 12 hours, 18 hours, or 24 hours, or at least 1 week, 2 weeks, 3 weeks, 4 weeks, 5 weeks, 6 weeks, 7 weeks, or 8 weeks, after introduction of the aforementioned oligonucleotide into a cell.

In some embodiments, an oligonucleotide is delivered in the form of a transgene that is engineered to express in a cell the oligonucleotides of the present disclosure (e.g., in the form of an shRNA). In some embodiments, an oligonucleotide is delivered using a transgene that is engineered to express any of the oligonucleotides disclosed herein. The transgene may be delivered using a viral vector (e.g., adenovirus, retrovirus, vaccinia virus, poxvirus, adeno-associated virus, or herpes simplex virus) or a non-viral vector (e.g., a plasmid or synthetic mRNA). In some embodiments, the transgene may be directly injected into a subject.

In another aspect, the present disclosure provides a method for preventing at least one symptom in a subject having a disorder that will benefit from a reduction in AGT expression, such as an AGT-related disease, e.g., hypertension, e.g., borderlinehypertension (also known as prehypertension), primary hypertension (also known as essential hypertension or idiopathic hypertension), secondary hypertension (also known as inessential hypertension), hypertensive emergency (also known as malignant hypertension), hypertensive urgency, isolated systolic or diastolic hypertension, pregnancy-related hypertension (e.g., preeclampsia, eclampsia, and postpartum preeclampsia), diabetic hypertension, resistant hypertension, refractory hypertension, paroxysmal hypertension, renovascular hypertension (also known as renal hypertension), Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy (including peripheral vascular disease), diabetic nephropathy, diabetic retinopathy, chronic heart failure, cardiomyopathy, diabetic cardiomyopathy, glomerulosclerosis, coarctation of the aorta, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid disease, heart failure (e.g., left ventricular systolic dysfunction), myocardial infarction, angina pectoris, stroke, diabetes (e.g., diabetic nephropathy), nephropathy (e.g., chronic kidney disease or diabetic nephropathy, optionally in a pregnant environment), renal failure (e.g., chronic renal failure), cognitive disorder (e.g., Alzheimer's disease), and systemic sclerosis (e.g., scleroderma renal crisis). In particular embodiments, the AGT-related disease includes intrauterine growth retardation (IUGR) and fetal growth restriction. The method comprises administering to the subject a therapeutically effective amount of an iRNA agent (e.g., dsRNA) or vector of the present disclosure, thereby preventing at least one symptom in the subject having the disorder that will benefit from a reduction in AGT expression.

In certain aspects, the present disclosure provides a method for preventing in a subject a disease, disorder, symptom, or condition as described herein by administering to the subject a therapeutic agent (e.g., an oligonucleotide or a vector or transgene encoding same). In some embodiments, the subject to be treated is a subject who will therapeutically benefit from, for example, a reduction in the amount of AGT protein.

Methods described herein generally involve administering to a subject an effective amount (i.e., an amount that can produce a desirable therapeutic result) of an oligonucleotide. A therapeutically acceptable amount may be an amount that can treat a disease or disorder. The appropriate dosage for any subject will depend on certain factors, including the

subject's size, body surface area, and age, the particular composition to be administered, one or more active ingredients in the composition, time and route of administration, general health, and other drugs administered simultaneously.

In some embodiments, a subject is administered any one of the compositions disclosed herein enterally (e.g., orally, through a gastric feeding tube, through a duodenal feeding tube, via gastrostomy, or rectally), parenterally (e.g., subcutaneous injection, intravenous injection or infusion, intraarterial injection or infusion, or intramuscular injection), topically (e.g., epicutaneously, by inhalation, via eye drops, or through a mucous membrane), or by direct injection into a target organ (e.g., the liver of the subject). Typically, oligonucleotides disclosed herein are administered intravenously or subcutaneously.

In some embodiments, an oligonucleotide is administered at a dose ranging from about 0.001 mg/kg to about 200 mg/kg (e.g., about 0.1 mg/kg to about 100 mg/kg). In some embodiments, an oligonucleotide is administered to a subject at a dose ranging from about 0.1 mg/kg to about 50 mg/kg, preferably from about 0.1 mg/kg to about 20 mg/kg, from 0.3 mg/kg to about 18 mg/kg, from 0.5 mg/kg to about 15 mg/kg, or from 0.5 mg/kg to about 12 mg/kg, and more preferably from about 1 mg/kg to about 10 mg/kg.

In some embodiments, an oligonucleotide is administered at a fixed dose of about 50 mg to about 800 mg. In some embodiments, an oligonucleotide is administered to a subject at a fixed dose of about 50 mg to about 200 mg, about 200 mg to about 400 mg, or about 400 mg to about 800 mg. In some embodiments, an oligonucleotide is administered to a subject at a fixed dose of about 100 mg, about 200 mg, about 300 mg, about 400 mg, 500 mg, about 600 mg, about 700 mg, or about 800 mg.

As a group of non-limiting examples, an oligonucleotide of the present disclosure will typically be administered once a year, twice a year, quarterly (once every three months), every two months (once every two months), monthly, or weekly. In some embodiments, a fixed dose is administered to the subject once a month. In some embodiments, a fixed dose is administered to the subject once every six months.

In some embodiments, the subject is administered a fixed dose of about 150 mg about once every six months. In some embodiments, the subject is administered a fixed dose of about 300 mg about once every six months. In some embodiments, the subject is administered a fixed dose of about 300 mg about once every six months. In some embodiments, the subject is administered a fixed dose of about 600 mg about once every six months. In some embodiments, the subject is administered a fixed dose of about 800 mg about once every six months. In some embodiments, the subject is administered a fixed dose of about 800 mg about once every six months.

In some embodiments, the subject to be treated is a human (e.g., a human patient) or a non-human primate or other mammalian subject. Other exemplary subjects include domestic animals such as dogs and cats; livestock such as horses, cattle, pigs, sheep, goats, and chickens; and animals such as mice, rats, guinea pigs, and hamsters.

In another aspect, the present disclosure provides a method for inhibiting the expression of the angiotensinogen (AGT) gene in a subject, comprising administering to the subject a fixed dose of about 50 mg to about 800 mg of an oligonucleotide or a salt thereof.

In another aspect, the present disclosure provides a method for preventing or treating a subject having an angiotensinogen (AGT)-related disorder, comprising administering to the subject a fixed dose of about 50 mg to about 800 mg of an oligonucleotide or a salt thereof.

In another aspect, the present disclosure provides a method for lowering blood pressure levels in a subject, comprising administering to the subject a fixed dose of about 50 mg to about 800 mg of an oligonucleotide or a salt thereof.

In some embodiments, the method further comprises administering to the subject an additional therapeutic agent for treating hypertension. In some embodiments, the additional therapeutic agent is selected from a diuretic, an angiotensin-converting enzyme (ACE) inhibitor, an angiotensin II receptor antagonist, a β-blocker, a vasodilator, a calcium channel blocker, an aldosterone antagonist, an α2-agonist, a renin inhibitor, an α-blocker, a peripherally acting adrenergic agent, a selective D1 receptor partial agonist, a non-selective α-adrenergic antagonist, a synthetic steroidal anti-mineralocorticoid agent; a combination of any of the foregoing; and a hypertension therapeutic agent formulated as a combination of agents. In some embodiments, the additional therapeutic agent includes an angiotensin II receptor antagonist. In other embodiments, the angiotensin II receptor antagonist is selected from losartan, valsartan, olmesartan, eprosartan, and azilsartan.

For the purpose of clarity and a concise description, features are described herein as part of the same or separate embodiments; however, it will be appreciated that the scope of the present disclosure may include embodiments having combinations of all or some of the features described.

Below, the present disclosure is described in more detail with reference to specific examples; however, the examples are used for illustrative purposes only and are not intended to limit the present disclosure.

## Examples

Where the sources of reagents are not specifically given herein, these reagents are available from any molecular biology reagent supplier, and their quality/purity criteria apply to molecular biology.

Abbreviations for nucleotide monomers used in nucleic acid sequence representations. It will be appreciated that these monomers, when present in oligonucleotides, are linked to each other by 5'-3' phosphodiester linkages unless otherwise specified. In addition, it should be appreciated that when a nucleotide contains a 2'-fluoro modification, fluorine replaces the hydroxyl group at that position in the parent nucleotide (that is, it is a 2'-deoxy-2'-fluoro nucleotide).

Table A. Abbreviations for nucleotide monomers used for nucleic acid sequence representations

| Abbreviation | Nucleotide |
|---|---|
| A | Adenosine-3'-phosphate |
| Af | 2'-Fluoroadenosine-3'-phosphate |
| Afs | 2'-Fluoroadenosine-3'-phosphorothioate |
| As | Adenosine-3'-phosphorothioate |
| C | Cytidine-3'-phosphate |
| Cf | 2'-Fluorocytidine-3'-phosphate |
| Cfs | 2'-Fluorocytidine-3'-phosphorothioate |
| Cs | Cytidine-3'-phosphorothioate |
| G | Guanosine-3'-phosphate |
| Gf | 2'-Fluoroguanosine-3'-phosphate |
| Gfs | 2'-Fluoroguanosine-3'-phosphorothioate |
| Gs | Guanosine-3'-phosphorothioate |
| U | Uridine-3'-phosphate |
| Uf | 2'-Fluorouridine-3'-phosphate |
| Ufs | 2'-Fluorouridine-3'-phosphorothioate |
| Us | Uridine-3'-phosphorothioate |
| Am | 2'-O-Methyladenosine-3'-phosphate |
| Ams | 2'-O-Methyladenosine-3'-phosphorothioate |
| Cm | 2'-O-Methylcytidine-3'-phosphate |
| Cms | 2'-O-Methylcytidine-3'-phosphorothioate |
| Gm | 2'-O-Methylguanosine-3'-phosphate |
| Gms | 2'-O-Methylguanosine-3'-phosphorothioate |
| Um | 2'-O-Methyluridine-3'-phosphate |
| Ums | 2'-O-Methyluridine-3'-phosphorothioate |
| s | Phosphorothioate linkage |
| P | Phosphate |
| dA | 2'-Deoxyadenosine-3'-phosphate |
| dAs | 2'-Deoxyadenosine-3'-phosphorothioate |
| dC | 2'-Deoxycytidine-3'-phosphate |
| dCs | 2'-Deoxycytidine-3'-phosphorothioate |
| dG | 2'-Deoxyguanosine-3'-phosphate |
| dGs | 2'-Deoxyguanosine-3'-phosphorothioate |
| dT | 2'-Deoxythymidine-3'-phosphate |
| dTs | 2'-Deoxythymidine-3'-phosphorothioate |
| dU | 2'-Deoxyuridine-3'-phosphate |
| dUs | 2'-Deoxyuridine-3'-phosphorothioate |

(continued)

| Abbreviation | Nucleotide |
|---|---|
| (Agn) | Adenosine-glycol nucleic acid (GNA) |
| (Cgn) | Cytidine-glycol nucleic acid (GNA) |
| (Ggn) | Guanosine-glycol nucleic acid (GNA) |
| (Tgn) | Thymidine-glycol nucleic acid (GNA) |
| I | Inosine-3'-phosphate |
| Im | 2'-O-Methylinosine-3'-phosphate |
| Ims | 2'-O-Methylinosine-3'-phosphorothioate |
| If | 2'-Fluoroinosine-3'-phosphate |
| Ifs | 2'-Fluoroinosine-3'-phosphorothioate |

**Preparation of targeting ligands**

(i) L96 was prepared with reference to the method described in Patent No. CN104717982B.
(ii) A1 was prepared as follows:

(1) Synthesis of intermediate E1

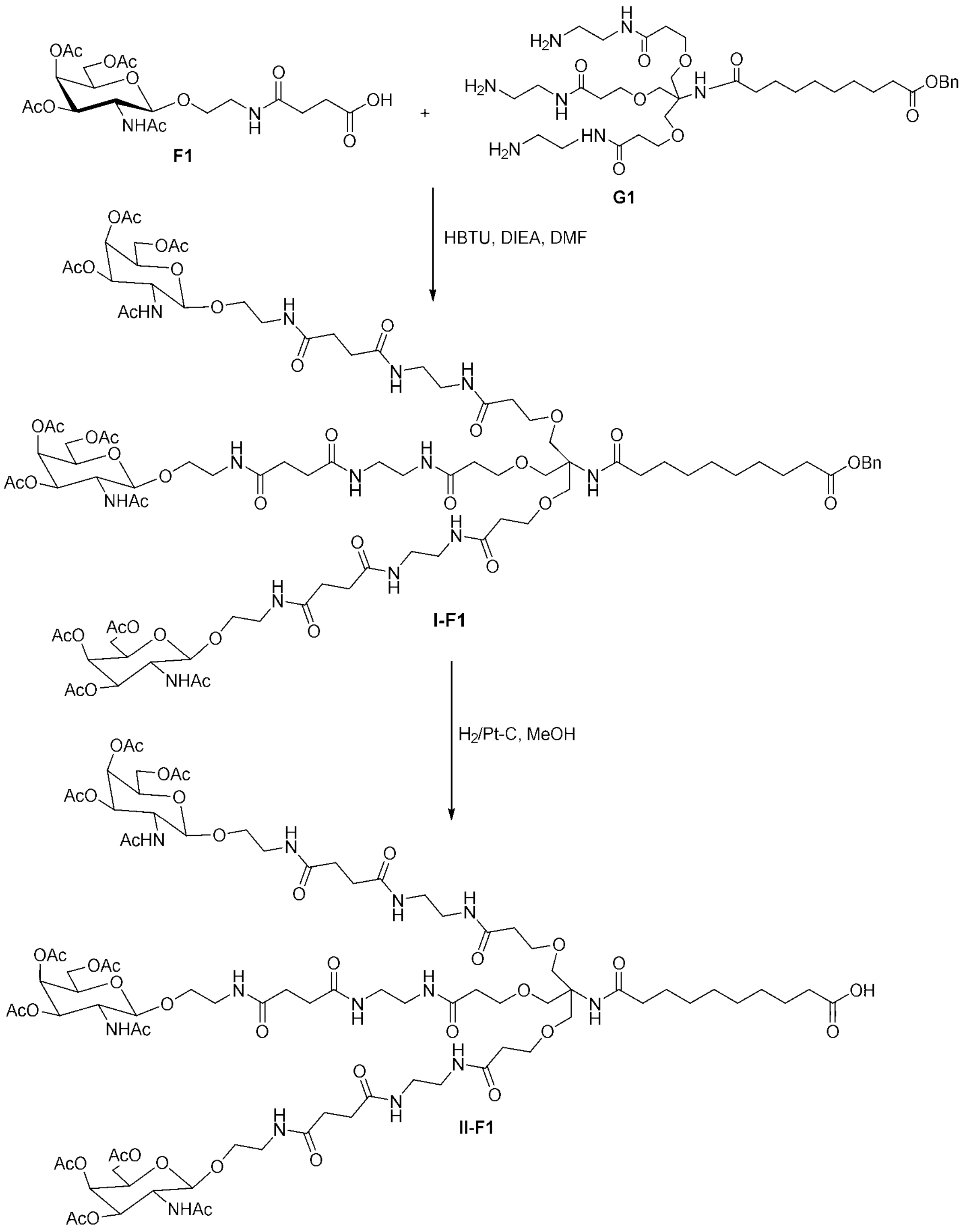


F1 + G1 → I-F1 → II-F1

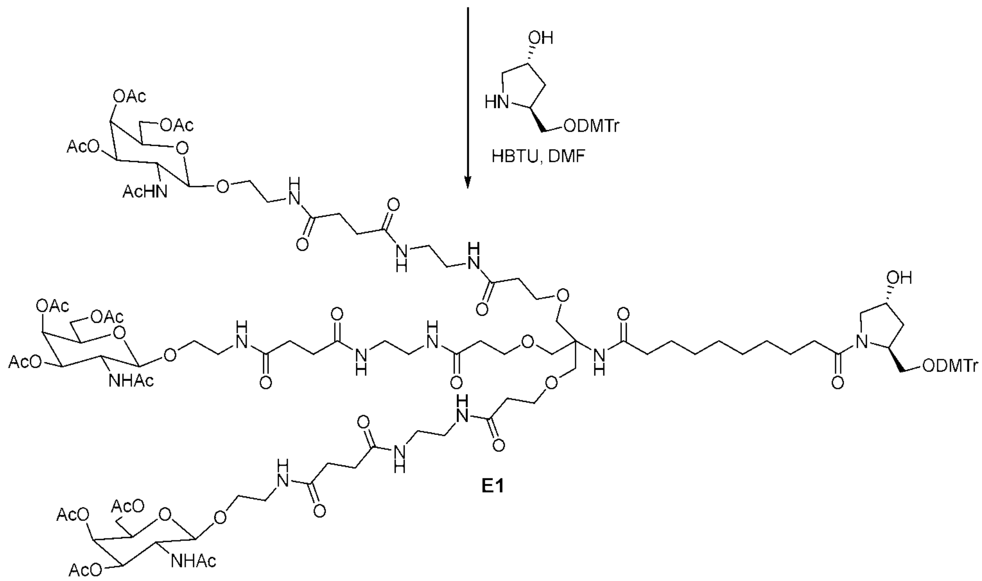


E1

F1 (199.46 mg, 0.41 mmol) was dissolved in DMF and activated by adding HBTU (155.39 mg, 0.41 mmol), and DIEA (67.81 L, 0.41 mmol) was then added. After 30 min of stirring, intermediate G1 (100.0 mg, 0.14 mmol) was added to the reaction system. After the mixture was stirred overnight, TLC analysis showed that the reaction was complete. After the reaction mixture was concentrated under reduced pressure, the residue was separated by column chromatography (PE:EA = 10:1) to give intermediate I-F1 (174.98 mg, yield: 58%). Intermediate I-F1 was debenzylated in a hydrogen atmosphere with palladium on carbon as a catalyst to give intermediate II-F1 (156.34, 95%). HBTU-activated intermediate II-F1 was coupled to (3R,5S)-5-[[bis(4-methoxyphenyl)phenylmethoxy] methyl]-3-pyrrolidinol in DMF to give intermediate E1; $^1$HNMR (400 MHz, DMSO-$D_6$): $\delta$ 7.91-7.86 (m, 8H), 7.51-7.45 (m, 6H), 7.40-7.32 (m, 10H), 7.02-6.95 (m, 3H), 6.03-5.89 (m, 4H), 5.31 (d, 3H), 5.07-4.99 (m, 4H), 4.43-4.37 (m, 3H), 4.23-4.19 (m, 4H), 4.17-4.13 (m, 6H), 4.05-4.00 (m, 6H), 3.88-3.81 (m, 6H), 3.77 (s, 6H), 3.69-3.66 (m, 9H), 3.50-3.47 (m, 12H), 3.40-3.37 (m, 6H), 3.20-3.16 (m, 16H), 2.60-2.57 (m, 2H), 2.46 (s, 9H), 2.30 (s, 9H), 2.10 (m, 6H), 2.07 (s, 9H), 1.98 (s, 9H), 1.56-1.45 (m, 8H), 1.32-1.27 (m, 4H). HRMS $[M+H]^+$ m/z 2467 (calcd for $C_{115}H_{168}N_{14}O_{45}$, 2466).

(2) Synthesis of solid-phase support A1 with protecting group

E1

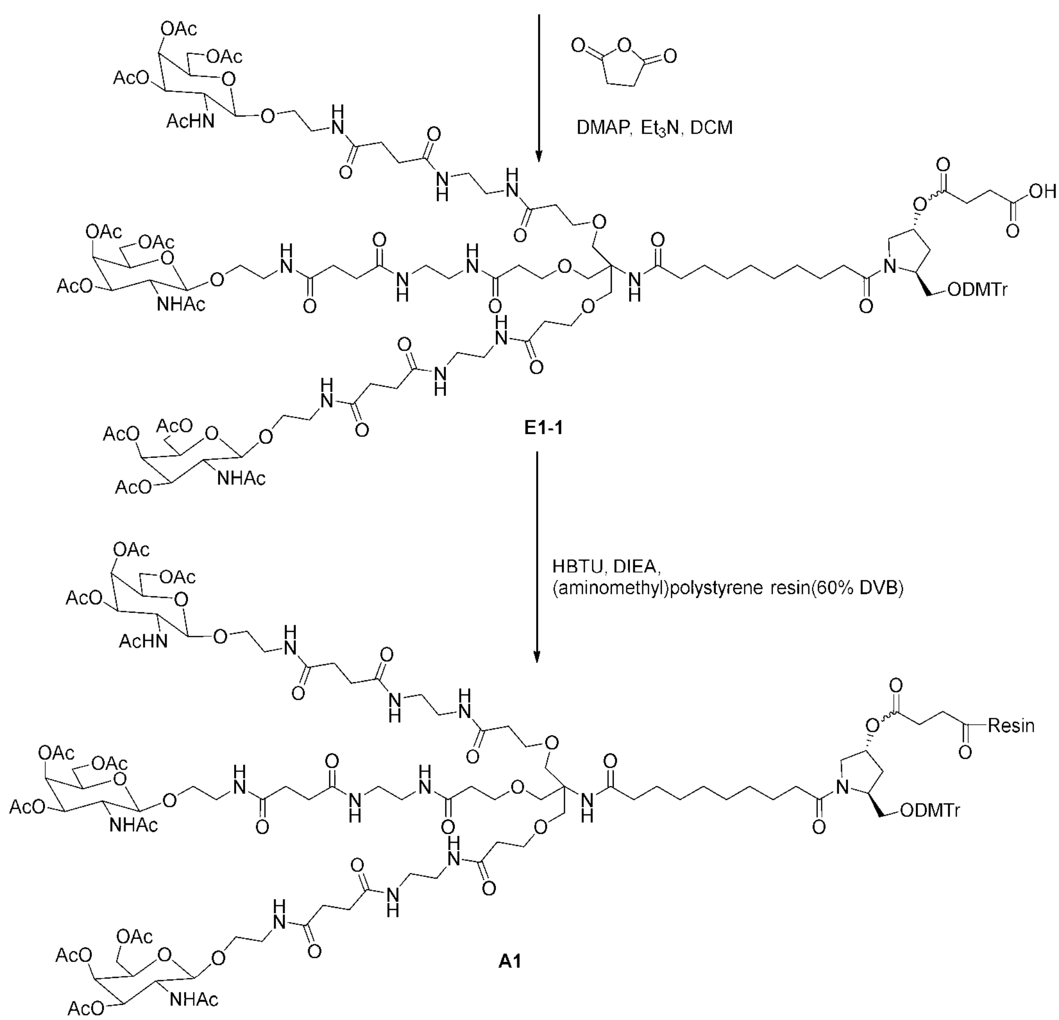


E1-1

A1

Intermediate E1 was treated with succinic anhydride in the presence of DMAP and $Et_3N$ to give hemisuccinate intermediate E1-1 in quantitative yield. Hemisuccinate intermediate E1-1 was coupled to the amino group of (aminomethyl)polystyrene resin cross-linked with 60% divinylbenzene (DVB) (amine content: 250 mol/g) to give solid-phase support A1 with a protecting group (loading: 100 mol/g).

## Preparation of oligonucleotides

(1) Preparation of siRNAs

siRNA sequences were obtained by separately synthesizing sense strands (SS) and antisense strands (AS) on the solid-phase support and subjecting the strands to deprotection, cleavage, purification, annealing, purification, and lyophilization.

Solid-phase synthesis (FIG. 2): The sense and antisense strands were separately synthesized on the solid-phase support using the phosphoramidite technique and an automated oligonucleotide synthesizer. The synthesizer was, for example, AKTA Oligopilot (Cytiva), Dr. Oligo 192XLc (Kunshan Biolytic Precision Instrument Co., Ltd.). Solid-phase synthesis started from the 3' end of a sequence. According to the sequence, monomers were sequentially coupled to form the sequence. The coupling of each phosphoramidite monomer included four chemical steps: 1) decapping or deprotection (removing the hydroxyl protecting group); 2) coupling; 3) oxidation; and 4) capping. The phosphoramidite monomers, reagents, and purification consumables used were all commercially available reagents and consumables. For example, a variety of phosphoramidite monomers (e.g., 5'-O-(4,4'-dimethoxytrityl)-2'-O-methyl-uridine-3'-CE-phosphoramidite) were purchased from Shanghai Hongene Biotech Corporation, and reaction reagents (e.g., 40 wt% methylamine in water and 28 wt% ammonium hydroxide in water) were purchased from Sigma-Aldrich LLC. The siRNA synthesis and purification methods used herein are described in, e.g., US20130178612A1 and US2015100197A1. The synthesis

method for sequences containing VPUm and APU structures is described in, e.g., J. Med. Chem. 2018, 61, 734-744.

(2) Preparation of double-stranded RNA (dsRNA) agents

(a) Synthesis of sense strands

The solid-phase phosphoramidite method is a well-established oligonucleotide synthesis method. Reactions were conducted in a stainless steel synthesis column using a computer-controlled synthesizer. The synthesis of a sense strand started with a solid-phase support loaded with a targeting ligand (e.g., L96 and A1) or directly started with a solid-phase support. By controlling different tubes in the solid-phase synthesizer and injecting different starting materials (in 3'-to-5' order according to the sequence), reagents, and solvents, phosphoramidite nucleoside monomers were linked one by one. The reaction process included cycles of four steps: a DMT protecting group removal reaction, a condensation reaction, an oxidation or thionation reaction, and a capping reaction, and each cycle resulted in linking to the previous nucleotide unit. An oligonucleotide sequence of 19 or 21 nucleotide units was obtained. After the synthesis was completed, the protecting group (2-cyanoethyl) was removed on the solid-phase synthesis column, and the synthesized sequence was then cleaved from the solid-phase support via ammonolysis. After filtration, the filter cake was washed with ethanol, and the filtrate and washings were collected and concentrated to give a sense strand crude product. The crude product was purified by chromatography (SOURCE 15Q) and lyophilized to give the target product sense strand. In the synthesizer, siRNA sense strand conjugates were synthesized with a solid-phase support loaded with a targeting ligand (e.g., L96) as a start, and siRNAs were synthesized directly with a solid-phase support as a start.

(b) Synthesis of antisense strands

The synthesis of an antisense strand was similar to that of a sense strand. By controlling different tubes in the solid-phase synthesizer and injecting different starting materials (in 3'-to-5' order according to the sequence), reagents, and solvents, phosphoramidite nucleoside monomers were linked one by one. The reaction process included cycles of four steps: a DMT protecting group removal reaction, a condensation reaction, an oxidation or thionation reaction, and a capping reaction, and each cycle resulted in linking to the previous nucleotide unit. An oligonucleotide sequence of 21 or 23 nucleotide units was obtained. After the synthesis was completed, the protecting group (2-cyanoethyl) was removed on the solid-phase synthesis column, and the synthesized sequence was then cleaved from the solid-phase support via ammonolysis. After filtration, the filter cake was washed with ethanol, and the filtrate and washings were collected and concentrated to give an antisense strand crude product. The crude product was purified by chromatography (SOURCE 15Q), ultrafiltered, and lyophilized to give the target product antisense strand siRNA.

(c) Preparation of double-stranded siRNAs

An AS strand and a SS strand were separately dissolved in water for injection, and the solutions were mixed in a determined ratio (1.01:1.0-1.2:1.0). The mixture was incubated at 30-50 °C for 30-90 min and cooled to room temperature. After lyophilization, a double-stranded siRNA product was obtained.

By using the same method, the double-stranded siRNA agents in Tables 2, 3, and 4 below were prepared.

**Example 1. *In Vitro* Activity Screening of AGT-siRNAs in Liver Cell Line**

First, candidate oligonucleotide sequences complementary to the human AGT mRNA (NM_000029.3, Table 1) were generated using a computer-based algorithm. Some of these sequences were also complementary to the cynomolgus monkey AGT mRNA (XM_005443695.2, Table 1) or had no more than 2 mismatches. Some of these sequences were designed as double-stranded siRNAs in which the sense strand and the antisense strand were 19/21 paired, and the antisense strand had two overhangs complementary to the mRNA sequence; in certain cases, the overhangs of the antisense strand were non-complementary UU. Some of these sequences were designed as double-stranded siRNAs in which the sense strand and the antisense strand were 21/23 paired, and the antisense strand had two overhangs complementary to the mRNA sequence. Some of these sequences were designed as double-stranded siRNAs in which the sense strand and the antisense strand were 21/21 or 23/23 paired. The base at position 1 of the 5' end of the antisense strand (the last position of the 3' end of the sense strand) of some of the complementarily paired sequences was replaced with a base that did not match the AGT mRNA.

Table 1. Human and cynomolgus monkey AGT mRNA sequences

| Species | GenBank RefSeq # |
|---|---|
| Human | NM_000029.3 |
| Cynomolgus monkey | XM_005443695.2 |

Certain oligonucleotides might contain glycol nucleic acid (GNA) modifications. In certain oligonucleotides, an individual ribonucleic acid was replaced with (Tgn), which is a thymidine-glycol nucleic acid (GNA) S-isomer represented by formula (I). In certain oligonucleotides, an individual ribonucleic acid was replaced with (Cgn), which is a cytidine-glycol nucleic acid (GNA) S-isomer represented by formula (II). In certain oligonucleotides, an individual ribonucleic acid was replaced with (Agn), which is an adenosine-glycol nucleic acid (GNA) S-isomer represented by formula (III). In certain oligonucleotides, an individual ribonucleic acid was replaced with (Ggn), which is a guanosine-glycol nucleic acid (GNA) S-isomer represented by formula (IV). 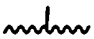 means linking to the remainder of the oligonucleotide. The structural formulas of Tgn, Cgn, Agn, and Ggn are as follows:

Tgn

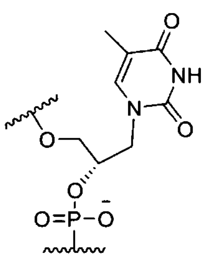

(I) ,

Cgn

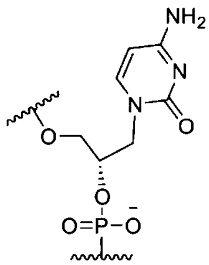

(II) ,

Agn

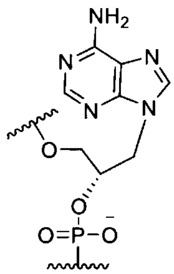

(III) , and

Ggn

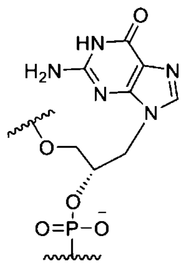

(IV) .

Certain oligonucleotide sequences might contain 2'-5'-phosphodiester linkages, and for a pair of adjacent nucleoside units, 2'-5' was bonded to 5'-2'. In certain oligonucleotides, an individual ribonucleic acid was replaced with (U-2'5'), which is uridine-2'-phosphate represented by formula (V). In certain oligonucleotides, an individual ribonucleic acid was replaced with (G-2'5'), which is guanosine-2'-phosphate represented by formula (VI). In certain oligonucleotides, an individual ribonucleic acid was replaced with (C-2'5'), which is cytidine-2'-phosphate represented by formula (VII). In certain oligonucleotides, an individual ribonucleic acid was replaced with (A-2'5'), which is adenosine-2'-phosphate represented by formula (VIII). In certain oligonucleotides, an individual ribonucleic acid was replaced with (T-2'5'), which is thymidine-2'-phosphate represented by formula (IX).

(U-2'5')

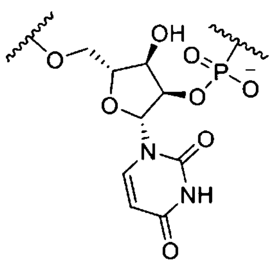

(V) ,

(G-2'5')

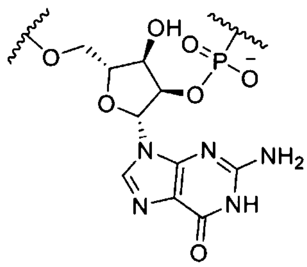

(VI) ,

(C-2'5')

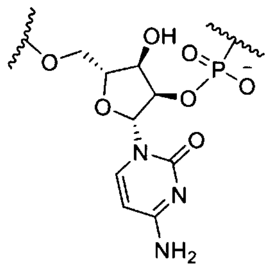

(VII) ,

(A-2'5')

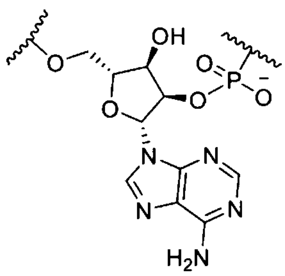

(VIII) , and

(T-2'5')

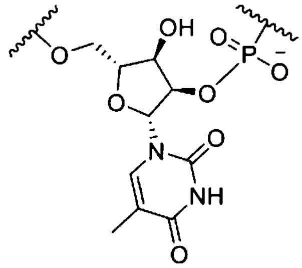

(IX) .

In Tables 2, 3, and 4, "G", "C", "A", "U", "T", and "I" typically represent nucleotides having guanine, cytosine, adenine, uracil, thymine, and hypoxanthine as bases, respectively. The naked sequences in Tables 2, 3, and 4 refer to unmodified oligonucleotide sequences.

For nucleotide modifications: m stands for 2'-methoxy; f stands for 2'-deoxy-2'-fluoro; s stands for phosphorothioate; APU is 5'-phosphate analog-modified uridylic acid represented by formula (X) (2'-acetylamino-5'-vinylphosphonate-uridylic acid); and VPUm is 5'-phosphate analog-modified uridylic acid represented by formula (XI) (2'-methoxy-5'-vinylphosphonate-uridylic acid):

APU

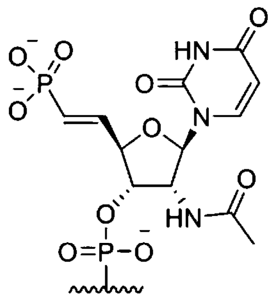

(X) and

VPUm

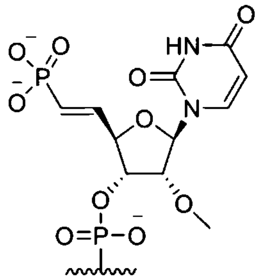

(XI) .

A1 is a GalNAc-targeting ligand represented by formula (XII), and L96 is N-[tris(GalNAc-alkyl)amidodecanoyl]-4-hydroxyprolinol (Hyp-(GalNAc-alkyl)3) represented by formula (XIII):

A1

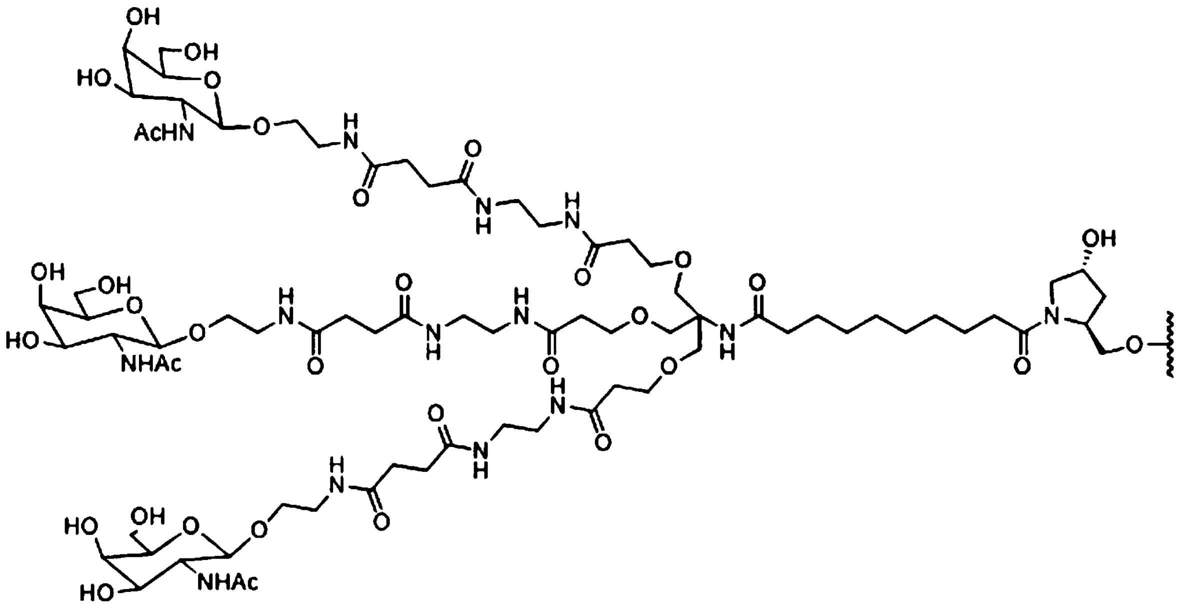

(XII)

L96

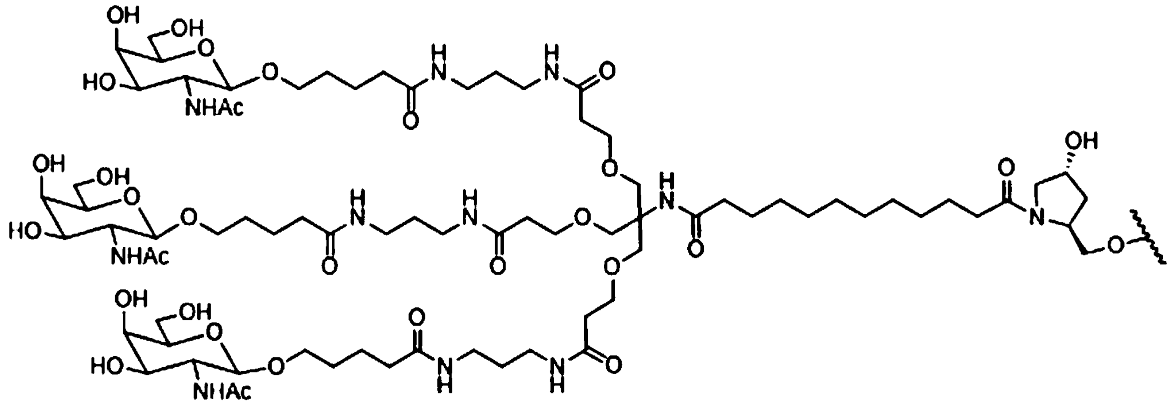

(XIII)

Table 2. Naked oligonucleotide sequences

| Sequence No. | Sequence of sense strand | Naked sequence SEQ ID NO | Sequence of antisense strand | Naked sequence SEQ ID NO |
|---|---|---|---|---|
| AL0181001 | GUCAUCCACAAUGAGAGUACA | 1 | UGUACUCUCAUUGUGGAUGACGA | 220 |
| AL0181002 | CUUCCACCUCGUCAUCCACAA | 2 | UUGUGGAUGACGAGGUGGAAGGG | 221 |
| AL0181003 | CCACCUCGUCAUCCACAAUGA | 3 | UCAUUGUGGAUGACGAGGUGGAA | 222 |
| AL0181004 | CCUCGUCAUCCACAAUGAGAA | 4 | UUCUCAUUGUGGAUGACGAGGUG | 223 |
| AL0181005 | CGUCAUCCACAAUGAGAGUAA | 5 | UUACUCUCAUUGUGGAUGACGAG | 224 |
| AL0181006 | AUCCACAAUGAGAGUACCUGA | 6 | UCAGGUACUCUCAUUGUGGAUGA | 225 |
| AL0181007 | CCACAAUGAGAGUACCUGUGA | 7 | UCACAGGUACUCUCAUUGUGGAU | 226 |
| AL0181008 | CAAUGAGAGUACCUGUGAGCA | 8 | UGCUCACAGGUACUCUCAUUGUG | 227 |
| AL0181009 | GACACCGAAGACAAGUUGAGA | 9 | UCUCAACUUGUCUUCGGUGUCAA | 228 |
| AL0181010 | GUCGGGAUGCUGGCCAACUUA | 10 | UAAGUUGGCCAGCAUCCCGACCA | 229 |
| AL0181011 | GGGAUGCUGGCCAACUUCUUA | 11 | UAAGAAGUUGGCCAGCAUCCCGA | 230 |
| AL0181012 | ACUUCUUGGGCUUCCGUAUAU | 12 | AUAUACGGAAGCCCAAGAAGUUG | 231 |
| AL0181013 | AUGGCAUGCACAGUGAGCUAU | 13 | AUAGCUCACUGUGCAUGCCAUAU | 232 |
| AL0181014 | GGCAUGCACAGUGAGCUAUGA | 14 | UCAUAGCUCACUGUGCAUGCCAU | 233 |
| AL0181015 | GCUGACAGGCUACAGGCAAUA | 15 | UAUUGCCUGUAGCCUGUCAGCUG | 234 |
| AL0181016 | GUGUUCCUUGGAAGGACAAGA | 16 | UCUUGUCCUUCCAAGGAACACCC | 235 |
| AL0181017 | GAUGCGCACAAGGUCCUGUCU | 17 | AGACAGGACCUUGUGCGCAUCCA | 236 |
| AL0181018 | GGCUGUACAGGGCCUGCUAGU | 18 | ACUAGCAGGCCCUGUACAGCCUG | 237 |
| AL0181019 | UUGCUGCUGAGAAGAUUGACA | 19 | UGUCAAUCUUCUCAGCAGCAACA | 238 |
| AL0181020 | GAGAAGAUUGACAGGUUCAUG | 20 | CAUGAACCUGUCAAUCUUCUCAG | 239 |
| AL0181021 | GGACAGCACCCUGGCUUUCAA | 21 | UUGAAAGCCAGGGUGCUGUCCAC | 240 |
| AL0181022 | ACAGCACCCUGGCUUUCAACA | 22 | UGUUGAAAGCCAGGGUGCUGUCC | 241 |
| AL0181023 | AGGAGUUCUGGGUGGACAACA | 23 | UGUUGUCCACCCAGAACUCCUGG | 242 |
| AL0181024 | GGUGGACAACAGCACCUCAGU | 24 | ACUGAGGUGCUGUUGUCCACCCA | 243 |
| AL0181025 | GUGGACAACAGCACCUCAGUA | 25 | UACUGAGGUGCUGUUGUCCACCC | 244 |
| AL0181026 | GCCUCACUAUGCCUCUGACCU | 26 | AGGUCAGAGGCAUAGUGAGGCUG | 245 |

(continued)

| Sequence No. | Sequence of sense strand | Naked sequence SEQ ID NO | Sequence of antisense strand | Naked sequence SEQ ID NO |
|---|---|---|---|---|
| AL0181027 | GGACAAGGUGGAGGGUCUCAA | 27 | UUGAGACCCUCCACCUUGUCCAG | 246 |
| AL0181028 | GACAAGGUGGAGGGUCUCACU | 28 | AGUGAGACCCUCCACCUUGUCCA | 247 |
| AL0181029 | ACAAGGUGGAGGGUCUCACUU | 29 | AAGUGAGACCCUCCACCUUGUCC | 248 |
| AL0181030 | CUCACUUUCCAGCAAAACUCA | 30 | UGAGUUUUGCUGGAAAGUGAGAC | 249 |
| AL0181031 | ACUCCCUCAACUGGAUGAAGA | 31 | UCUUCAUCCAGUUGAGGGAGUUU | 250 |
| AL0181032 | CCCUCAACUGGAUGAAGAAAA | 32 | UUUUCUUCAUCCAGUUGAGGGAG | 251 |
| AL0181033 | CCUCAACUGGAUGAAGAAACU | 33 | AGUUUCUUCAUCCAGUUGAGGGA | 252 |
| AL0181034 | CAACUGGAUGAAGAAACUAUA | 34 | UAUAGUUUCUUCAUCCAGUUGAG | 253 |
| AL0181035 | AACUGGAUGAAGAAACUAUCU | 35 | AGAUAGUUUCUUCAUCCAGUUGA | 254 |
| AL0181036 | GGUGCUGCAAGGAUCUUAUGA | 36 | UCAUAAGAUCCUUGCAGCACCAG | 255 |
| AL0181037 | GCUGCAAGGAUCUUAUGACCU | 37 | AGGUCAUAAGAUCCUUGCAGCAC | 256 |
| AL0181038 | GGUGCUGAACAGCAUUUUUUU | 38 | AAAAAAAUGCUGUUCAGCACCUC | 257 |
| AL0181039 | GUGCUGAACAGCAUUUUUUUU | 39 | AAAAAAAAUGCUGUUCAGCACCU | 258 |
| AL0181040 | GCUGAACAGCAUUUUUUUUGA | 40 | UCAAAAAAAAUGCUGUUCAGCAC | 259 |
| AL0181041 | GAACAGCAUUUUUUUUGAGCU | 41 | AGCUCAAAAAAAAUGCUGUUCAG | 260 |
| AL0181042 | CAGCAUUUUUUUUGAGCUUGA | 42 | UCAAGCUCAAAAAAAAUGCUGUU | 261 |
| AL0181043 | GAGCUUGAAGCGGAUGAGAGA | 43 | UCUCUCAUCCGCUUCAAGCUCAA | 262 |
| AL0181044 | GCUUGAAGCGGAUGAGAGAGA | 44 | UCUCUCUCAUCCGCUUCAAGCUC | 263 |
| AL0181045 | AUGAGAGAGAGCCCACAGAGU | 45 | ACUCUGUGGGCUCUCUCUCAUCC | 264 |
| AL0181046 | GAGAGAGAGCCCACAGAGUCU | 46 | AGACUCUGUGGGCUCUCUCUCAU | 265 |
| AL0181047 | AGAGAGAGCCCACAGAGUCUA | 47 | UAGACUCUGUGGGCUCUCUCUCA | 266 |
| AL0181048 | GAGUCUACCCAACAGCUUAAA | 48 | UUUAAGCUGUUGGGUAGACUCUG | 267 |
| AL0181049 | AGUCUACCCAACAGCUUAACA | 49 | UGUUAAGCUGUUGGGUAGACUCU | 268 |
| AL0181050 | CAUUCCUGUUUGCUGUGUAUA | 50 | UAUACACAGCAAACAGGAAUGGG | 269 |
| AL0181051 | CCUGUUUGCUGUGUAUGAUCA | 51 | UGAUCAUACACAGCAAACAGGAA | 270 |
| AL0181052 | GUUUGCUGUGUAUGAUCAAAA | 52 | UUUUGAUCAUACACAGCAAACAG | 271 |

(continued)

| Sequence No. | Sequence of sense strand | Naked sequence SEQ ID NO | Sequence of antisense strand | Naked sequence SEQ ID NO |
|---|---|---|---|---|
| AL0181053 | GCAAAGGCCAGCAGCAGAUAA | 53 | UUAUCUGCUGCUGGCCUUUGCCU | 272 |
| AL0181054 | CAGCGAUGUGUCACCCCCAGU | 54 | ACUGGGGGUGACACAUCGCUGAU | 273 |
| AL0181055 | GCGAUGUGUCACCCCCAGUCU | 55 | AGACUGGGGGUGACACAUCGCUG | 274 |
| AL0181056 | GAUGUGUCACCCCCAGUCUCA | 56 | UGAGACUGGGGGUGACACAUCGC | 275 |
| AL0181057 | GUGUCACCCCCAGUCUCCCAA | 57 | UUGGGAGACUGGGGGUGACACAU | 276 |
| AL0181058 | GUCACCCCCAGUCUCCCACCU | 58 | AGGUGGGAGACUGGGGGUGACAC | 277 |
| AL0181059 | CACCCCCAGUCUCCCACCUUU | 59 | AAAGGUGGGAGACUGGGGGUGAC | 278 |
| AL0181060 | CCCCCAGUCUCCCACCUUUUA | 60 | UAAAAGGUGGGAGACUGGGGGUG | 279 |
| AL0181061 | CCCAGUCUCCCACCUUUUCUU | 61 | AAGAAAAGGUGGGAGACUGGGGG | 280 |
| AL0181062 | CAGUCUCCCACCUUUUCUUCU | 62 | AGAAGAAAAGGUGGGAGACUGGG | 281 |
| AL0181063 | UCUCCCACCUUUUCUUCUAAU | 63 | AUUAGAAGAAAAGGUGGGAGACU | 282 |
| AL0181064 | CACCUUUUCUUCUUAAUGAGU | 64 | ACUCAUUAAGAAGAAAAGGUGGG | 283 |
| AL0181065 | CCUUUUCUUCUAAUGAGUCGA | 65 | UCGACUCAUUAGAAGAAAAGGUG | 284 |
| AL0181066 | AGCCGUUUCUCCUUGGUCUAA | 66 | UUAGACCAAGGAGAAACGGCUGC | 285 |
| AL0181067 | GCCGUUUCUCCUUGGUCUAAA | 67 | UUUAGACCAAGGAGAAACGGCUG | 286 |
| AL0181068 | CCAGUUUGCUGGGUUUAUUUU | 68 | AAAAUAAACCCAGCAAACUGGGA | 287 |
| AL0181069 | CAGUUUGCUGGGUUUAUUUUA | 69 | UAAAAUAAACCCAGCAAACUGGG | 288 |
| AL0181070 | GUCCACAAUGAGAGUACCUGA | 70 | UCAGGUACUCUCAUUGUGGACGA | 289 |
| AL0181071 | GGCAUGCACACUGAGCUAUGA | 71 | UCAUAGCUCACUGUGCAUGCCAU | 233 |
| AL0181072 | GAGAAGAUUGACAGGUUCAUA | 72 | UAUGAACCUGUCAAUCUUCUCAG | 290 |
| AL0181073 | GGACAGCACCGUGGCUUUCAA | 73 | UUGAAAGCCAGGGUGCUGUCCAC | 240 |
| AL0181074 | GCUGAACAGCGUUUUUUUUGA | 74 | UCAAAAAAAAUGCUGUUCAGCAC | 259 |
| AL0181075 | CCUUUUCUUCGAAUGAGUCGA | 75 | UCGACUCAUUAGAAGAAAAGGUG | 284 |
| AL0181076 | AGCCGUUUCUGCUUGGUCUAA | 76 | UUAGACCAAGGAGAAACGGCUGC | 285 |
| AL0181077 | GCCGUUUCUGCUUGGUCUAAA | 77 | UUUAGACCAAGGAGAAACGGCUG | 286 |
| AL0181078 | CGUUGUUCUGGGUACUACAGA | 78 | UCUGUAGUACCCAGAACAACGGC | 291 |

(continued)

| Sequence No. | Sequence of sense strand | Naked sequence SEQ ID NO | Sequence of antisense strand | Naked sequence SEQ ID NO |
|---|---|---|---|---|
| AL0181079 | GCCACCUCGUCAUCCACAAUA | 79 | UAUUGUGGAUGACGAGGUGGCAG | 292 |
| AL0181080 | CACCUCGUCAUCCACAAUGAA | 80 | UUCAUUGUGGAUGACGAGGUGGA | 293 |
| AL0181081 | AUCCACAAUGCGAGUACCUGA | 81 | UCAGGUACUCUCAUUGUGGAUGA | 225 |
| AL0181082 | UCCACAAUGAGAGUACCUGUA | 82 | UACAGGUACUCUCAUUGUGGAUG | 294 |
| AL0181083 | GAGCUUGAAGGGGAUGAGAGA | 83 | UCUCUCAUCCGCUUCAAGCUCAA | 262 |
| AL0181084 | GAGAGAGAGCGCACAGAGUCU | 84 | AGACUCUGUGGGCUCUCUCUCAU | 265 |
| AL0181085 | GUUUUUUGAGCUUGAAGCGGA | 85 | UCCGCUUCAAGCUCAAAAAACAU | 295 |
| AL0181086 | GUUUUGAGCUUGAAGCGGAUA | 86 | UAUCCGCUUCAAGCUCAAAACAA | 296 |
| AL0181087 | GUUUGAGCUUGAAGCGGAUGA | 87 | UCAUCCGCUUCAAGCUCAAACAA | 297 |
| AL0181088 | CCAGUCUCCCACCUUUUCUUA | 88 | UAAGAAAAGGUGGGAGACUGGGG | 298 |
| AL0181089 | CCCCAGUCUCCCACCUUUUCU | 89 | AGAAAAGGUGGGAGACUGGGGAG | 299 |
| AL0181090 | GCGACUUUGAGCUGGAAAGCA | 90 | UGCUUUCCAGCUCAAAGUCGCCU | 300 |
| AL0181091 | GCAGCCGUUUCUCCUUGGUCU | 91 | AGACCAAGGAGAAACGGCUGCUU | 301 |
| AL0181092 | CCGUUUCUCCUUGGUCUAAGU | 92 | ACUUAGACCAAGGAGAAACGGCU | 302 |
| AL0181093 | GUCCACAAUGAGAGUACCUGA | 70 | UCAGGUACUCUCAUUGUGGACGA | 289 |
| AL0181094 | GUCCACAAUGAGAGUACCUGU | 93 | ACAGGUACUCUCAUUGUGGACGA | 303 |
| AL0181095 | AUCCACAAUGAGAGUACCUGU | 94 | ACAGGUACUCUCAUUGUGGAUGA | 304 |
| AL0181096 | GCAUCCACAAUGAGAGUACCU | 95 | AGGUACUCUCAUUGUGGAUGCCG | 305 |
| AL0181097 | CCCAGUCUCCCACCUUUUCUA | 96 | UAGAAAAGGUGGGAGACUGGGGG | 306 |
| AL0181098 | CAGUCUCCCACCUUUUCUUCA | 97 | UGAAGAAAAGGUGGGAGACUGGG | 307 |
| AL0181099 | GGUCUCCCACCUUUUCUUCUA | 98 | UAGAAGAAAAGGUGGGAGACCGG | 308 |
| AL0181100 | GUCUCCCACCUUUUCUUCUAA | 99 | UUAGAAGAAAAGGUGGGAGACUG | 309 |
| AL0181101 | CAGCCGUUUCUCCUUGGUCUA | 100 | UAGACCAAGGAGAAACGGCUGCU | 310 |
| AL0181102 | GGCCGUUUCUCCUUGGUCUAA | 101 | UUAGACCAAGGAGAAACGGCCGC | 311 |
| AL0181103 | GCCGUUUCUCCUUGGUCUAAG | 102 | CUUAGACCAAGGAGAAACGGCUG | 312 |
| AL0181104 | CGUUUCUCCUUGGUCUAAGUG | 103 | CACUUAGACCAAGGAGAAACGGC | 313 |

(continued)

| Sequence No. | Sequence of sense strand | Naked sequence SEQ ID NO | Sequence of antisense strand | Naked sequence SEQ ID NO |
|---|---|---|---|---|
| AL0181105 | CGUUUCUCCUUGGUCUAAGUA | 104 | UACUUAGACCAAGGAGAAACGGC | 314 |
| AL0181106 | GUUUCUCCUUGGUCUAAGUGU | 105 | ACACUUAGACCAAGGAGAAACGG | 315 |
| AL0181107 | GUUUCUCCUUGGUCUAAGUGA | 106 | UCACUUAGACCAAGGAGAAACGG | 316 |
| AL0181108 | CAGUCUCCCAGCUUUUCUUCU | 107 | AGAAGAAAAGGUGGGAGACUGGG | 281 |
| AL0181109 | GGCCGUUUCUCCUUGGUCUAA | 101 | UUAGACCAAGGAGAAACGGCCGC | 311 |
| AL0181110 | GCCGUUUCUCGUUGGUCUAAA | 108 | UUUAGACCAAGGAGAAACGGCUG | 286 |
| AL0181111 | GGCCGUUUCUGCUUGGUCUAA | 109 | UUAGACCAAGGAGAAACGGCCGC | 311 |
| AL0181112 | GGCCGUUUCUACUUGGUCUAA | 110 | UUAGACCAAGGAGAAACGGCCGC | 311 |
| AL0181113 | CCGUUUCUCCUUGGUCUAAGA | 111 | UCUUAGACCAAGGAGAAACGGCU | 317 |
| AL0181114 | GCGUUUCUCCUUGGUCUAAGU | 112 | ACUUAGACCAAGGAGAAACGCCU | 318 |
| AL0181115 | GCGUUUCUCCUUGGUCUAAGA | 113 | UCUUAGACCAAGGAGAAACGCCU | 319 |
| AL0181116 | GCGUUUCUCCGUGGUCUAAGU | 114 | ACUUAGACCAAGGAGAAACGCCU | 318 |
| AL0181117 | CCGUUUCUCCUUGGUCUAAGA | 111 | UCUUAGACCAAGGAGAAACGGCU | 317 |
| AL0181118 | UCCCACCUUUUCUUCUAAU | 115 | AUUAGAAGAAAAGGUGGGAGA | 320 |
| AL0181119 | GCCCACCUUUUCUUCUAAA | 116 | UUUAGAAGAAAAGGUGGGCGA | 321 |
| AL0181120 | GCAUCCACAAUGAGAGUAA | 117 | UUACUCUCAUUGUGGAUGCCG | 322 |
| AL0181121 | GCGUCAUCCACAAUGAGAA | 118 | UUCUCAUUGUGGAUGACGCGG | 323 |
| AL0181122 | CCUCGUCAUCCACAAUGAA | 119 | UUCAUUGUGGAUGACGAGGUG | 324 |
| AL0181123 | CACCUCGUCAUCCACAAUA | 120 | UAUUGUGGAUGACGAGGUGGA | 325 |
| AL0181124 | GUCCACAAUGAGAGUACCA | 121 | UGGUACUCUCAUUGUGGACGA | 326 |
| AL0181125 | GCCACAAUGAGAGUACCUA | 122 | UAGGUACUCUCAUUGUGGCUG | 327 |
| AL0181126 | CACAAUGAGAGUACCUGUA | 123 | UACAGGUACUCUCAUUGUGGA | 328 |
| AL0181127 | GCAAUGAGAGUACCUGUGA | 124 | UCACAGGUACUCUCAUUGCGG | 329 |
| AL0181128 | CAAUGAGAGUACCUGUGAA | 125 | UUCACAGGUACUCUCAUUGUG | 330 |
| AL0181129 | GAUGAGAGUACCUGUGAGA | 126 | UCUCACAGGUACUCUCAUCGU | 331 |
| AL0181130 | GGAGAGUACCUGUGAGCAA | 127 | UUGCUCACAGGUACUCUCCUU | 332 |

(continued)

| Sequence No. | Sequence of sense strand | Naked sequence SEQ ID NO | Sequence of antisense strand | Naked sequence SEQ ID NO |
|---|---|---|---|---|
| AL0181131 | GAGAGUACCUGUGAGCAGA | 128 | UCUGCUCACAGGUACUCUCAU | 333 |
| AL0181134 | CUCCCACCUUUUCUUCUAA | 129 | UUAGAAGAAAAGGUGGGAGAC | 334 |
| AL0181135 | GCUCCCACCUUUUCUUCUA | 130 | UAGAAGAAAAGGUGGGAGCCU | 335 |
| AL0181136 | GUCUCCCACCUUUUCUUCA | 131 | UGAAGAAAAGGUGGGAGACUG | 336 |
| AL0181137 | GGUCUCCCACCUUUUCUUA | 132 | UAAGAAAAGGUGGGAGACCGG | 337 |
| AL0181138 | CCCACCUUUUCUUCUAAUA | 133 | UAUUAGAAGAAAAGGUGGGAG | 338 |
| AL0181139 | CCACCUUUUCUUCUAAUGA | 134 | UCAUUAGAAGAAAAGGUGGGA | 339 |
| AL0181140 | CACCUUUUCUUCUAAUGAA | 135 | UUCAUUAGAAGAAAAGGUGGG | 340 |
| AL0181141 | GCCUUUUCUUCUAAUGAGA | 136 | UCUCAUUAGAAGAAAAGGCGG | 341 |
| AL0181142 | CCUUUUCUUCUAAUGAGUA | 137 | UACUCAUUAGAAGAAAAGGUG | 342 |
| AL0181143 | CUUUUCUUCUAAUGAGUCA | 138 | UGACUCAUUAGAAGAAAAGGU | 343 |
| AL0181144 | AGCACCUGAAUUUCUGUUU | 139 | AAACAGAAAUUCAGGUGCUUG | 344 |
| AL0181145 | GUCUGUAAUACCUUAGUUU | 140 | AAACUAAGGUAUUACAGACAC | 345 |
| AL0181146 | CAAAUGCACCUCCCAGUUU | 141 | AAACUGGGAGGUGCAUUUGUG | 346 |
| AL0181147 | UGGACAGCACCCUGGCUUU | 142 | AAAGCCAGGGUGCUGUCCACA | 347 |
| AL0181148 | CUUCUAAUGAGUCGACUUU | 143 | AAAGUCGACUCAUUAGAAGAA | 348 |
| AL0181149 | AGGUGGAGGGUCUCACUUU | 144 | AAAGUGAGACCCUCCACCUUG | 349 |
| AL0181150 | GAACCAUAGCUGGUUAUUU | 145 | AAAUAACCAGCUAUGGUUCCG | 350 |
| AL0181151 | UUGCCUUCGGUUUGUAUUU | 146 | AAAUACAAACCGAAGGCAAUG | 351 |
| AL0181152 | GGUUUGUAUUUAGUGUCUU | 147 | AAGACACUAAAUACAAACCGA | 352 |
| AL0181153 | CAAGUUGAGAACAAAAAUU | 148 | AAUUUUUGUUCUCAACUUGAA | 353 |
| AL0181154 | GUUUGUGAAACAAAAAAGU | 149 | ACUUUUUUGUUUCACAAACAA | 354 |
| AL0181155 | UGGACAAGGUGGAGGGUCU | 150 | AGACCCUCCACCUUGUCCAGG | 355 |
| AL0181156 | CUCACUUUCCAGCAAAACU | 151 | AGUUUUGCUGGAAAGUGAGAC | 356 |
| AL0181157 | ACUUCUUGGGCUUCCGUAU | 152 | AUACGGAAGCCCAAGAAGUUG | 357 |
| AL0181158 | GUCUUGAAUGUAAGAACAU | 153 | AUGUUCUUACAUUCAAGACAC | 358 |

(continued)

| Sequence No. | Sequence of sense strand | Naked sequence SEQ ID NO | Sequence of antisense strand | Naked sequence SEQ ID NO |
|---|---|---|---|---|
| AL0181159 | UUCCUGUUUGCUGUGUAUG | 154 | CAUACACAGCAAACAGGAAUG | 359 |
| AL0181160 | GGCAAGAACCAGUGUUUAG | 155 | CUAAACACUGGUUCUUGCCUC | 360 |
| AL0181161 | GAUUGACAGGUUCAUGCAG | 156 | CUGCAUGAACCUGUCAAUCUU | 361 |
| AL0181162 | GUAUAUAUGGCAUGCACAG | 157 | CUGUGCAUGCCAUAUAUACGG | 362 |
| AL0181163 | CCAUAGCUGGUUAUUUCUC | 158 | GAGAAAUAACCAGCUAUGGUU | 363 |
| AL0181164 | AUGUAAGAACAUGACCUCC | 159 | GGAGGUCAUGUUCUUACAUUC | 364 |
| AL0181165 | CCGUAUAUAUGGCAUGCAC | 160 | GUGCAUGCCAUAUAUACGGAA | 365 |
| AL0181166 | GUGUCUUGAAUGUAAGAAC | 161 | GUUCUUACAUUCAAGACACUA | 366 |
| AL0181167 | AGGCAAGAACCAGUGUUUA | 162 | UAAACACUGGUUCUUGCCUCC | 367 |
| AL0181168 | CAUUCCUGUUUGCUGUGUA | 163 | UACACAGCAAACAGGAAUGGG | 368 |
| AL0181169 | GGGUUUUAAAAUUAAAGUA | 164 | UACUUUAAUUUUAAAACCCAA | 369 |
| AL0181170 | CUUCUUGGGCUUCCGUAUA | 165 | UAUACGGAAGCCCAAGAAGUU | 370 |
| AL0181171 | GUUUUAAAAUUAAAGUAUA | 166 | UAUACUUUAAUUUUAAAACCC | 371 |
| AL0181172 | UAUAUGGCAUGCACAGUGA | 167 | UCACUGUGCAUGCCAUAUAUA | 372 |
| AL0181173 | CUUGAAUGUAAGAACAUGA | 168 | UCAUGUUCUUACAUUCAAGAC | 373 |
| AL0181174 | GAUCUUAUGACCUGCAGGA | 169 | UCCUGCAGGUCAUAAGAUCCU | 374 |
| AL0181175 | GGUGGACAACAGCACCUCA | 170 | UGAGGUGCUGUUGUCCACCCA | 375 |
| AL0181176 | AGAUUGACAGGUUCAUGCA | 171 | UGCAUGAACCUGUCAAUCUUC | 376 |
| AL0181177 | GUUCAUGCAGGCUGUGACA | 172 | UGUCACAGCCUGCAUGAACCU | 377 |
| AL0181178 | CAGGAGUUCUGGGUGGACA | 173 | UGUCCACCCAGAACUCCUGGG | 378 |
| AL0181179 | CGUAUAUAUGGCAUGCACA | 174 | UGUGCAUGCCAUAUAUACGGA | 379 |
| AL0181180 | ACUCCCUCAACUGGAUGAA | 175 | UUCAUCCAGUUGAGGGAGUUU | 380 |
| AL0181181 | GGGUCUCACUUUCCAGCAA | 176 | UUGCUGGAAAGUGAGACCCUC | 381 |
| AL0181182 | AGGAGUUCUGGGUGGACAA | 177 | UUGUCCACCCAGAACUCCUGG | 382 |
| AL0181183 | GACCAGCUUGUUUGUGAAA | 178 | UUUCACAAACAAGCUGGUCGG | 383 |
| AL0181184 | CCUGUGAGCAGCUGGCAAA | 179 | UUUGCCAGCUGCUCACAGGUA | 384 |

(continued)

| Sequence No. | Sequence of sense strand | Naked sequence SEQ ID NO | Sequence of antisense strand | Naked sequence SEQ ID NO |
|---|---|---|---|---|
| AL0181185 | GGUCUCACUUUCCAGCAAA | 180 | UUUGCUGGAAAGUGAGACCCU | 385 |
| AL0181186 | CAAAAAUUGGGUUUUAAAA | 181 | UUUUAAAACCCAAUUUUUGUU | 386 |
| AL0181187 | GCUUGUUUGUGAAACAAAA | 182 | UUUUGUUUCACAAACAAGCUG | 387 |
| AL0181188 | AAGACGUUUAUUACUAACA | 183 | UGUUAGUAAUAAACGUCUUGC | 388 |
| AL0181189 | CGACCAGCUUGUUUGUGAA | 184 | UUCACAAACAAGCUGGUCGGU | 389 |
| AL0181190 | CUUGUUUGUGAAACAAAAA | 185 | UUUUUGUUUCACAAACAAGCU | 390 |
| AL0181191 | ACAACUUCUCGGUGACUCA | 186 | UGAGUCACCGAGAAGUUGUCC | 391 |
| AL0181192 | UGGUCGGGAUGCUGGCCAA | 187 | UUGGCCAGCAUCCCGACCAUU | 392 |
| AL0181193 | CUGAAUUUCUGUUUGAAUG | 188 | CAUUCAAACAGAAAUUCAGGU | 393 |
| AL0181194 | AGGAUCUUAUGACCUGCAG | 189 | CUGCAGGUCAUAAGAUCCUUG | 394 |
| AL0181195 | GAGAAGAUUGACAGGUUCA | 190 | UGAACCUGUCAAUCUUCUCAG | 395 |
| AL0181196 | GUUCCAAAAAGAAUUCCAA | 191 | UUGGAAUUCUUUUUGGAACAG | 396 |
| AL0181197 | UGUAAGAACAUGACCUCCG | 192 | CGGAGGUCAUGUUCUUACAUU | 397 |
| AL0181198 | GGCUGUACAGGGCCUGCUA | 193 | UAGCAGGCCCUGUACAGCCUG | 398 |
| AL0181199 | CAACUGGAUGAAGAAACUA | 194 | UAGUUUCUUCAUCCAGUUGAG | 399 |
| AL0181200 | CCCUCAACUGGAUGAAGAA | 195 | UUCUUCAUCCAGUUGAGGGAG | 400 |
| AL0181201 | UACGUGAAAGAUGCAAGCA | 196 | UGCUUGCAUCUUUCACGUAUU | 401 |
| AL0181202 | GCAAAGGCCAGCAGCAGAU | 197 | AUCUGCUGCUGGCCUUUGCCU | 402 |
| AL0181203 | GCAAGGAUCUUAUGACCUG | 198 | CAGGUCAUAAGAUCCUUGCAG | 403 |
| AL0181204 | GCUGACAGGCUACAGGCAA | 199 | UUGCCUGUAGCCUGUCAGCUG | 404 |
| AL0181205 | CCCUUGUGUUAGUAAUAAA | 200 | UUUAUUACUAACACAAGGGAG | 405 |
| AL0181206 | GCACCUGAAUUUCUGUUUG | 201 | CAAACAGAAAUUCAGGUGCUU | 406 |
| AL0181207 | GAGCACAGCAUGAGGCCAG | 202 | CUGGCCUCAUGCUGUGCUCAG | 407 |
| AL0181208 | CUCCCUUGUGUUAGUAAUA | 203 | UAUUACUAACACAAGGGAGAA | 408 |
| AL0181209 | ACAAUACGUGAAAGAUGCA | 204 | UGCAUCUUUCACGUAUUGUUC | 409 |
| AL0181210 | GAACAAUACGUGAAAGAUG | 205 | CAUCUUUCACGUAUUGUUCAA | 410 |

(continued)

| Sequence No. | Sequence of sense strand | Naked sequence SEQ ID NO | Sequence of antisense strand | Naked sequence SEQ ID NO |
|---|---|---|---|---|
| AL0181211 | UUUCUCCCUUGUGUUAGUA | 206 | UACUAACACAAGGGAGAAAUA | 411 |
| AL0181212 | CCAAUGCCGGGAAGCCCAA | 207 | UUGGGCUUCCCGGCAUUGGCC | 412 |
| AL0181213 | UGCAAGCACCUGAAUUUCU | 208 | AGAAAUUCAGGUGCUUGCAUC | 413 |
| AL0181214 | GGUGCUGCAAGGAUCUUAU | 209 | AUAAGAUCCUUGCAGCACCAG | 414 |
| AL0181215 | CCACACAGCUGACAGGCUA | 210 | UAGCCUGUCAGCUGUGUGGUC | 415 |
| AL0181216 | UUGAACAAUACGUGAAAGA | 211 | UCUUUCACGUAUUGUUCAAAA | 416 |
| AL0181217 | GACACCGAAGACAAGUUGA | 212 | UCAACUUGUCUUCGGUGUCAA | 417 |
| AL0181218 | GAGCCAGUGUGGACAGCAC | 213 | GUGCUGUCCACACUGGCUCCC | 418 |
| AL0181219 | UGGAAGGACAAGAACUGCA | 214 | UGCAGUUCUUGUCCUUCCAAG | 419 |
| AL0181220 | CAACUUCUCGGUGACUCAA | 215 | UUGAGUCACCGAGAAGUUGUC | 420 |
| AL0181221 | AACGUCUUGCCACAAUAAG | 216 | CUUAUUGUGGCAAGACGUUUA | 421 |
| AL0181222 | CUGAGAAGAUUGACAGGUU | 217 | AACCUGUCAAUCUUCUCAGCA | 422 |
| AL0181223 | CCAGUUUGCUGGGUUUAUU | 218 | AAUAAACCCAGCAAACUGGGA | 423 |
| AL0181224 | ACUUCUCGGUGACUCAAGU | 219 | ACUUGAGUCACCGAGAAGUUG | 424 |
| AL0181225 | GUCCCACCUUUUCUUCUAA | 789 | UUAGAAGAAAAGGUGGGACAC | 805 |
| AL0181226 | GUCCCACCAUUUCUUCUAA | 790 | UUAGAAGAAAAGGUGGGACAC | 805 |
| AL0181227 | GUCCCACCCUUUCUUCUAA | 791 | UUAGAAGAAAAGGUGGGACAC | 805 |
| AL0181228 | GUCCCACCGUUUCUUCUAA | 792 | UUAGAAGAAAAGGUGGGACAC | 805 |
| AL0181229 | GUCCCACCUAUUCUUCUAA | 793 | UUAGAAGAAAAGGUGGGACAC | 805 |
| AL0181230 | GUCCCACCUCUUCUUCUAA | 794 | UUAGAAGAAAAGGUGGGACAC | 805 |
| AL0181231 | GUCCCACCUGUUCUUCUAA | 795 | UUAGAAGAAAAGGUGGGACAC | 805 |
| AL0181232 | GUCCCACCUUAUCUUCUAA | 796 | UUAGAAGAAAAGGUGGGACAC | 805 |
| AL0181233 | GUCCCACCUUCUCUUCUAA | 797 | UUAGAAGAAAAGGUGGGACAC | 805 |
| AL0181234 | GUCCCACCUUGUCUUCUAA | 798 | UUAGAAGAAAAGGUGGGACAC | 805 |
| AL0181235 | GCGUUUCUCCUUGGUCUAAGA | 113 | UCUUAGACCAAIGAGAAACGCCU | 806 |
| AL0181236 | GCGUUUCUCCUUGGUCUAAGA | 113 | UCUUAGACCAAGIAGAAACGCCU | 807 |

(continued)

| Sequence No. | Sequence of sense strand | Naked sequence SEQ ID NO | Sequence of antisense strand | Naked sequence SEQ ID NO |
|---|---|---|---|---|
| AL0181237 | GCGUUUCUCCUUGGUCUAAGA | 113 | UCUUAGACCAAGGAIAAACGCCU | 808 |
| AL0181238 | GCGUUUCUCCUUGGUCUAAGA | 113 | UCUUAGACCAAGGAGAIACGCCU | 809 |
| AL0181239 | GCGUUUCUCCUUGGUCUAAGA | 113 | UCUUAGACCAAGGAGAAICGCCU | 810 |
| AL0181240 | GCGUUUCUCCUUGGUCUAAGA | 113 | UCUUAGACCAAGGAGAAACICCU | 811 |
| AL0181241 | GCCACCUUUUCUUCUAAUA | 799 | UAUUAGAAGAAAAGGUGGCAG | 812 |
| AL0181242 | GACCUUUUCUUCUAAUGAA | 800 | UUCAUUAGAAGAAAAGGUCGG | 813 |
| AL0181243 | CAAGUUGAGAACAAAAAUA | 801 | UAUUUUUGUUCUCAACUUGAA | 814 |
| AL0181244 | GGGCAAGAACCAGUGUUUA | 802 | UAAACACUGGUUCUUGCCCCC | 815 |
| AL0181245 | GGACCAGCUUGUUUGUGAA | 803 | UUCACAAACAAGCUGGUCCGU | 816 |
| AL0181246 | CACCAGCUUGUUUGUGAAA | 804 | UUUCACAAACAAGCUGGUGGG | 817 |
| AL0181247 | GCGUUUCUCCUUGGUCUAAGA | 113 | UCUUAGACCAIGGAGAAACGCCU | 818 |
| AL0181248 | GCGUUUCUCCUUGGUCUAAGA | 113 | UCUUAGACCIAGGAGAAACGCCU | 819 |
| AL0181249 | GCGUUUCUCCUUGGUCUAAGA | 113 | UCUUAGICCAAGGAGAAACGCCU | 820 |
| AL0181250 | GCGUUUCUCCUUGGUCUAAGA | 113 | UCUUIGACCAAGGAGAAACGCCU | 821 |
| AL0181251 | GCACCUCGUCAUCCACAAUGA | 868 | UCAUUGUGGAUGACGAGGUGCAA | 872 |
| AL0181252 | GUGCUGCUGAGAAGAUUGACA | 869 | UGUCAAUCUUCUCAGCAGCACCA | 873 |
| AL0181253 | GAGAGAGAGCCCACAGAGUCA | 870 | UGACUCUGUGGGCUCUCUCUCAU | 874 |
| AL0181254 | GCUUUUCUUCUAAUGAGUCGA | 871 | UCGACUCAUUAGAAGAAAAGCUG | 875 |

Table 3. Modified oligonucleotides

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185001 | GmsUmsCmAmUmCmCfAmCfAfAfUmGmAmGmAmGmUmAmCmAm | 425/1 | UmsGfsUmAmCmUfCmUmCmAmUmUmGmUfGmGfAmUmGmAmCmsGmsAm | 599/220 |
| AL0185002 | CmsCmsAmCmCmUmCfGmUfCfAfUmCmCmAmCmAmAmUmGmAm | 426/3 | UmsCfsAmUmUmGfUmGmGfAmUmGmAmCfGmAfGmGmUmGmGmsAmsAm | 600/222 |
| AL0185003 | CmsCmsUmCmGmUmCfAmUfCfCfAmCmAmAmUmGmAmGmAmAm | 427/4 | UmsUfsCmUmCmAfUmUmGfUmGmGmAmUfGmAfCmGmAmGmGmsUmsGm | 601/223 |
| AL0185004 | AmsUmsCmCmAmCmAfAmUfGfAfGmAmGmUmAmCmCmUmGmAm | 428/6 | UmsCfsAmGmGmUfAmCmUfCmUmCmAmUfUmGfUmGmGmAmUmsGmsAm | 602/225 |
| AL0185005 | GmsGmsCmAmUmGmCfAmCfAfGfUmGmAmGmCmUmAmUmGmAm | 429/14 | UmsCfsAmUmAmGfCmUmCfAmCmUmGmUfGmCfAmUmGmCmCmsAmsUm | 603/233 |
| AL0185006 | GmsAmsGmAmAmGmAfUmUfGfAfCmAmGmGmUmUmCmAmUmGm | 430/20 | CmsAfsUmGmAmAfCmCmUfGmUmCmAmAfUmCfUmUmCmUmCmsAmsGm | 604/239 |
| AL0185007 | GmsGmsAmCmAmGmCfAmCfCfCfUmGmGmCmUmUmUmCmAmAm | 431/21 | UmsUfsGmAmAmAfGmCmCfAmGmGmGmUfGmCfUmGmUmCmCmsAmsCm | 605/240 |
| AL0185008 | GmsCmsUmGmAmAmCfAmGfCfAfUmUmUmUmUmUmUmUmGmAm | 432/40 | UmsCfsAmAmAmAfAmAmAfAmUmGmCmUfGmUfUmCmAmGmCmsAmsCm | 606/259 |
| AL0185009 | GmsAmsGmCmUmUmGfAmAfGfCfGmGmAmUmGmAmGmAmGmAm | 433/43 | UmsCfsUmCmUmCfAmUmCfCmGmCmUmUfCmAfAmGmCmUmCmsAmsAm | 607/262 |
| AL0185010 | GmsAmsGmAmGmAmGfAmGfCfCfCmAmCmAmGmAmGmUmCmUm | 434/46 | AmsGfsAmCmUmCfUmGmUfGmGmGmCmUfCmUfCmUmCmUmCmsAmsUm | 608/265 |
| AL0185011 | CmsCmsCmAmGmUmCfUmCfCfCfAmCmCmUmUmUmUmCmUmUm | 435/61 | AmsAfsGmAmAmAfAmGmGfUmGmGmGmAfGmAfCmUmGmGmGmsGmsGm | 609/280 |
| AL0185012 | CmsAmsGmUmCmUmCfCmCfAfCfCmUmUmUmUmCmUmUmCmUm | 436/62 | AmsGfsAmAmGmAfAmAmAfGmGmUmGmGfGmAfGmAmCmUmGmsGmsGm | 610/281 |
| AL0185013 | CmsCmsUmUmUmUmCfUmUfCfUfAmAmUmGmAmGmUmCmGmAm | 437/65 | UmsCfsGmAmCmUfCmAmUfUmAmGmAmAfGmAfAmAmAmGmGmsUmsGm | 611/284 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185014 | AmsGmsCmCmGmUmUfUmCfUfCfCmUmUmGmGmUmCmUmAmAm | 438/66 | UmsUfsAmGmAmCfCmAmAfGmGmAmGmAfAmAfCmGmGmCmUmsGmsCm | 612/285 |
| AL0185015 | GmsCmsCmGmUmUmUfCmUfCfCfUmUmGmGmUmCmUmAmAmAm | 439/67 | UmsUfsUmAmGmAfCmCmAfAmGmGmAmGfAmAfAmCmGmGmCmsUmsGm | 613/286 |
| AL0185016 | GmsUmsCmCmAmCmAfAmUfGfAfGmAmGmUmAmCmCmUmGmAm | 440/70 | UmsCfsAmGmGmUfAmCmUfCmUmCmAmUfUmGfUmGmGmAmCmsGmsAm | 614/289 |
| AL0185017 | GmsGmsCmAmUmGmCfAmCfAfCfUmGmAmGmCmUmAmUmGmAm | 441/71 | UmsCfsAmUmAmGfCmUmCfAmCmUmGmUfGmCfAmUmGmCmCmsAmsUm | 603/233 |
| AL0185018 | GmsAmsGmAmAmGmAfUmUfGfAfCmAmGmGmUmUmCmAmUmAm | 442/72 | UmsAfsUmGmAmAfCmCmUfGmUmCmAmAfUmCfUmUmCmUmCmsAmsGm | 615/290 |
| AL0185019 | GmsGmsAmCmAmGmCfAmCfCfGfUmGmGmCmUmUmUmCmAmAm | 443/73 | UmsUfsGmAmAmAfGmCmCfAmGmGmGmUfGmCfUmGmUmCmCmsAmsCm | 605/240 |
| AL0185020 | GmsCmsUmGmAmAmCfAmGfCfGfUmUmUmUmUmUmUmGmAm | 444/74 | UmsCfsAmAmAmAfAmAmAfAmUmGmCmUfGmUfUmCmAmGmCmsAmsCm | 606/259 |
| AL0185021 | CmsCmsUmUmUmUmCfUmUfCfGfAmAmUmGmAmGmUmCmGmAm | 445/75 | UmsCfsGmAmCmUfCmAmUfUmAmGmAmAfGmAfAmAmAmGmGmsUmsGm | 611/284 |
| AL0185022 | AmsGmsCmCmGmUmUfUmCfUfGfCmUmUmGmGmUmCmUmAmAm | 446/76 | UmsUfsAmGmAmCfCmAmAfGmGmAmGmAfAmAfCmGmGmCmUmsGmsCm | 612/285 |
| AL0185023 | GmsCmsCmGmUmUmUfCmUfGfCfUmUmGmGmUmCmUmAmAmAm | 447/77 | UmsUfsUmAmGmAfCmCmAfAmGmGmAmGfAmAfAmCmGmGmCmsUmsGm | 613/286 |
| AL0185024 | CmsGmsUmUmGmUmUfCmUfGfGfGmUmAmCmUmAmCmAmGmAm | 448/78 | UmsCfsUmGmUmAfGmUmAfCmCmCmAmGfAmAfCmAmAmCmGmsGmsCm | 616/291 |
| AL0185025 | GmsCmsCmAmCmCmUfCmGfUfCfAmUmCmCmAmCmAmAmUmAm | 449/79 | UmsAfsUmUmGmUfGmGmAfUmGmAmCmGfAmGfGmUmGmGmCmsAmsGm | 617/292 |
| AL0185026 | CmsAmsCmCmUmCmGfUmCfAfUfCmCmAmCmAmAmUmGmAmAm | 450/80 | UmsUfsCmAmUmUfGmUmGfGmAmUmGmAfCmGfAmGmGmUmGmsGmsAm | 618/293 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185027 | AmsUmsCmCmAmCmAfAmUfGfCfGmAmGmUmAmCmCmUmGmAm | 451/81 | UmsCfsAmGmGmUfAmCmUfCmUmCmAmUfUmGfUmGmGmAmUmsGmsAm | 602/225 |
| AL0185028 | UmsCmsCmAmCmAmAfUmGfAfGfAmGmUmAmCmCmUmGmUmAm | 452/82 | UmsAfsCmAmGmGfUmAmCfUmCmUmCmAfUmUfGmUmGmGmAmsUmsGm | 619/294 |
| AL0185029 | GmsAmsGmCmUmUmGfAmAfGfGfGmGmAmUmGmAmGmAmGmAm | 453/83 | UmsCfsUmCmUmCfAmUmCfCmGmCmUmUfCmAfAmGmCmUmCmsAmsAm | 607/262 |
| AL0185030 | GmsAmsGmAmGmAmGfAmGfCfGfCmAmCmAmGmAmGmUmCmUm | 454/84 | AmsGfsAmCmUmCfUmGmUfGmGmGmCmUfCmUfCmUmCmUmCmsAmsUm | 608/265 |
| AL0185031 | GmsUmsUmUmUmUmUfGmAfGfCfUmUmGmAmAmGmCmGmGmAm | 455/85 | UmsCfsCmGmCmUfUmCmAfAmGmCmUmCfAmAfAmAmAmAmCmsAmsUm | 620/295 |
| AL0185032 | GmsUmsUmUmUmGmAfGmCfUfUfGmAmAmGmCmGmGmAmUmAm | 456/86 | UmsAfsUmCmCmGfCmUmUfCmAmAmGmCfUmCfAmAmAmAmCmsAmsAm | 621/296 |
| AL0185033 | GmsUmsUmUmGmAmGfCmUfUfGfAmAmGmCmGmGmAmUmGmAm | 457/87 | UmsCfsAmUmCmCfGmCmUfUmCmAmAmGfCmUfCmAmAmAmCmsAmsAm | 622/297 |
| AL0185034 | CmsCmsAmGmUmCmUfCmCfCfAfCmCmUmUmUmUmCmUmUmAm | 458/88 | UmsAfsAmGmAmAfAmAmGfGmUmGmGmGfAmGfAmCmUmGmGmsGmsGm | 623/298 |
| AL0185035 | CmsCmsCmCmAmGmUfCmUfCfCfCmAmCmCmUmUmUmUmCmUm | 459/89 | AmsGfsAmAmAmAfGmGmUfGmGmGmAmGfAmCfUmGmGmGmGmsAmsGm | 624/299 |
| AL0185036 | GmsCmsGmAmCmUmUfUmGfAfGfCmUmGmGmAmAmAmGmCmAm | 460/90 | UmsGfsCmUmUmUfCmCmAfGmCmUmCmAfAmAfGmUmCmGmCmsCmsUm | 625/300 |
| AL0185037 | GmsCmsAmGmCmCmGfUmUfUfCfUmCmCmUmUmGmGmUmCmUm | 461/91 | AmsGfsAmCmCmAfAmGmGfAmGmAmAmAfCmGfGmCmUmGmCmsUmsUm | 626/301 |
| AL0185038 | CmsCmsGmUmUmUmCfUmCfCfUfUmGmGmUmCmUmAmAmGmUm | 462/92 | AmsCfsUmUmAmGfAmCmCfAmAmGmGmAfGmAfAmAmCmGmGmsCmsUm | 627/302 |
| AL0185039 | GmsUmsCmCmAmCmAfAmUfGfAfGmAmGmUmAmCmCmUmGmAm | 440/70 | UmsCfsAmGmGmUfAmCmUfCmUmCmAmUfUmGfUmGmGmAmCmsGmsAm | 614/289 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185040 | GmsUmsCmCmAmCmAfAmUfGfAfGmAmGmUmAmCmCmUmGmUm | 463/93 | AmsCfsAmGmGmUfAmCmUfCmUmCmAmUfUmGfUmGmGmAmCmsGmsAm | 628/303 |
| AL0185041 | AmsUmsCmCmAmCmAfAmUfGfAfGmAmGmUmAmCmCmUmGmUm | 464/94 | AmsCfsAmGmGmUfAmCmUfCmUmCmAmUfUmGfUmGmGmAmUmsGmsAm | 629/304 |
| AL0185042 | GmsCmsAmUmCmCmAfCmAfAfUfGmAmGmAmGmUmAmCmCmUm | 465/95 | AmsGfsGmUmAmCfUmCmUfCmAmUmUmGfUmGfGmAmUmGmCmsCmsGm | 630/305 |
| AL0185043 | CmsCmsCmAmGmUmCfUmCfCfCfAmCmCmUmUmUmUmCmUmAm | 466/96 | UmsAfsGmAmAmAfAmGmGfUmGmGmGmAfGmAfCmUmGmGmGmsGmsGm | 631/306 |
| AL0185044 | CmsAmsGmUmCmUmCfCmCfAfCfCmUmUmUmUmCmUmUmCmAm | 467/97 | UmsGfsAmAmGmAfAmAmAfGmGmUmGmGfGmAfGmAmCmUmGmsGmsGm | 632/307 |
| AL0185045 | GmsGmsUmCmUmCmCfCmAfCfCfUmUmUmUmCmUmUmCmUmAm | 468/98 | UmsAfsGmAmAmGfAmAmAfAmGmGmUmGfGmGfAmGmAmCmCmsGmsGm | 633/308 |
| AL0185046 | GmsUmsCmUmCmCmCfAmCfCfUfUmUmUmCmUmUmCmUmAmAm | 469/99 | UmsUfsAmGmAmAfGmAmAfAmAmGmGmUfGmGfGmAmGmAmCmsUmsGm | 634/309 |
| AL0185047 | CmsAmsGmCmCmGmUfUmUfCfUfCmCmUmUmGmGmUmCmUmAm | 470/100 | UmsAfsGmAmCmCfAmAmGfGmAmGmAmAfAmCfGmGmCmUmGmsCmsUm | 635/310 |
| AL0185048 | GmsGmsCmCmGmUmUfUmCfUfCfCmUmUmGmGmUmCmUmAmAm | 471/101 | UmsUfsAmGmAmCfCmAmAfGmGmAmGmAfAmAfCmGmGmCmCmsGmsCm | 636/311 |
| AL0185049 | GmsCmsCmGmUmUmUfCmUfCfCfUmUmGmGmUmCmUmAmAmGm | 472/102 | CmsUfsUmAmGmAfCmCmAfAmGmGmAmGfAmAfAmCmGmGmCmsUmsGm | 637/312 |
| AL0185050 | CmsGmsUmUmUmCmUfCmCfUfUfGmGmUmCmUmAmAmGmUmGm | 473/103 | CmsAfsCmUmUmAfGmAmCfCmAmAmGmGfAmGfAmAmAmCmGmsGmsCm | 638/313 |
| AL0185051 | CmsGmsUmUmUmCmUfCmCfUfUfGmGmUmCmUmAmAmGmUmAm | 474/104 | UmsAfsCmUmUmAfGmAmCfCmAmAmGmGfAmGfAmAmAmCmGmsGmsCm | 639/314 |
| AL0185052 | GmsUmsUmUmCmUmCfCmUfUfGfGmUmCmUmAmAmGmUmGmUm | 475/105 | AmsCfsAmCmUmUfAmGmAfCmCmAmAmGfGmAfGmAmAmAmCmsGmsGm | 640/315 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185053 | GmsUmsUmUmCmUmCfCmUfUfGfGmUmCmUmAmAmGmUmGmAm | 476/106 | UmsCfsAmCmUmUfAmGmAfCmCmAmAmGfGmAfGmAmAmAmCmsGmsGm | 641/316 |
| AL0185054 | GmsUmsCmAmUmCmCfAmCfAfAfUmGmAmGmAmGmUmAmCmAm | 425/1 | UmsGfsUmAmCmUfCmUmCfAmUmUmGmUfGmGfAmUmGmAmCmsGmsAm | 642/220 |
| AL0185055 | CmsAmsGmUmCmUmCfCmCfAfGfCmUmUmUmUmCmUmUmCmUm | 477/107 | AmsGfsAmAmGmAfAmAmAmGmGmUmGmGfGmAfGmAmCmUmGmsGmsGm | 643/281 |
| AL0185056 | GmsGmsCmCmGmUmUfUmCfUfCfCmUmUmGmGmUmCmUmAmAm | 471/101 | UmsUfsAmGmAmCfCmAmAmGmGmAmGmAfAmAfCmGmGmCmCmsGmsCm | 644/311 |
| AL0185057 | GmsCmsCmGmUmUmUfCmUfCfGfUmUmGmGmUmCmUmAmAmAm | 478/108 | UmsUfsUmAmGmAfCmCmAmAmGmGmAmGfAmAfAmCmGmGmCmsUmsGm | 645/286 |
| AL0185058 | GmsGmsCmCmGmUmUfUmCfUfGfCmUmUmGmGmUmCmUmAmAm | 479/109 | UmsUfsAmGmAmCfCmAmAmGmGmAmGmAfAmAfCmGmGmCmCmsGmsCm | 644/311 |
| AL0185059 | GmsGmsCmCmGmUmUfUmCfUfAfCmUmUmGmGmUmCmUmAmAm | 480/110 | UmsUfsAmGmAmCfCmAmAmGmGmAmGmAfAmAfCmGmGmCmCmsGmsCm | 644/311 |
| AL0185060 | CmsCmsGmUmUmUmCfUmCfCfUfUmGmGmUmCmUmAmAmGmAm | 481/111 | UmsCfsUmUmAmGfAmCmCmAmAmGmGmAfGmAfAmAmCmGmGmsCmsUm | 646/317 |
| AL0185061 | GmsCmsGmUmUmUmCfUmCfCfUfUmGmGmUmCmUmAmAmGmUm | 482/112 | AmsCfsUmUmAmGfAmCmCmAmAmGmGmAfGmAfAmAmCmGmCmsCmsUm | 647/318 |
| AL0185062 | GmsCmsGmUmUmUmCfUmCfCfUfUmGmGmUmCmUmAmAmGmAm | 483/113 | UmsCfsUmUmAmGfAmCmCmAmAmGmGmAfGmAfAmAmCmGmCmsCmsUm | 648/319 |
| AL0185063 | GmsCmsGmUmUmUmCfUmCfCfGfUmGmGmUmCmUmAmAmGmUm | 484/114 | AmsCfsUmUmAmGfAmCmCmAmAmGmGmAfGmAfAmAmCmGmCmsCmsUm | 647/318 |
| AL0185064 | CmsCmsGmUmUmUmCfUmCfCfUfUmGmGmUmCmUmAmAmGmAm | 481/111 | (VPUm)CfsUmUmAmGfAmCmCmAmAmGmGmAfGmAfAmAmCmGmGmsCmsUm | 649/317 |
| AL0185065 | UmsCmsCmCmAfCmCfUfUfUmUmCmUmUmCmUmAmAmUm | 485/115 | AmsUfsUmAmGmAfAmGmAmAmAmAmGmGfUmGfGmGmAmsGmsAm | 650/320 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185066 | GmsCmsCmCmAfCmCfUfUfUmUmCmUmUmCmUmAmAmAm | 486/116 | UmsUfsUmAmGmAfAmGmAmAmAmAmGmGfUmGfGmGmCmsGmsAm | 651/321 |
| AL0185067 | GmsCmsAmUmCfCmAfCfAfAmUmGmAmGmAmGmUmAmAm | 487/117 | UmsUfsAmCmUmCfUmCmAmUmUmGmUmGfGmAfUmGmCmsCmsGm | 652/322 |
| AL0185068 | GmsCmsGmUmCfAmUfCfCfAmCmAmAmUmGmAmGmAmAm | 488/118 | UmsUfsCmUmCmAfUmUmGmUmGmGmAmUfGmAfCmGmCmsGmsGm | 653/323 |
| AL0185069 | CmsCmsUmCmGfUmCfAfUfCmCmAmCmAmAmUmGmAmAm | 489/119 | UmsUfsCmAmUmUfGmUmGmGmAmUmGmAfCmGfAmGmGmsUmsGm | 654/324 |
| AL0185070 | CmsAmsCmCmUfCmGfUfCfAmUmCmCmAmCmAmAmUmAm | 490/120 | UmsAfsUmUmGmUfGmGmAmUmGmAmCmGfAmGfGmUmGmsGmsAm | 655/325 |
| AL0185071 | GmsUmsCmCmAfCmAfAfUfGmAmGmAmGmUmAmCmCmAm | 491/121 | UmsGfsGmUmAmCfUmCmUmCmAmUmUmGfUmGfGmAmCmsGmsAm | 656/326 |
| AL0185072 | GmsCmsCmAmCfAmAfUfGfAmGmAmGmUmAmCmCmUmAm | 492/122 | UmsAfsGmGmUmAfCmUmCmUmCmAmUmUfGmUfGmGmCmsUmsGm | 657/327 |
| AL0185073 | CmsAmsCmAmAfUmGfAfGfAmGmUmAmCmCmUmGmUmAm | 493/123 | UmsAfsCmAmGmGfUmAmCmUmCmUmCmAfUmUfGmUmGmsGmsAm | 658/328 |
| AL0185074 | GmsCmsAmAmUfGmAfGfAfGmUmAmCmCmUmGmUmGmAm | 494/124 | UmsCfsAmCmAmGfGmUmAmCmUmCmUmCfAmUfUmGmCmsGmsGm | 659/329 |
| AL0185075 | CmsAmsAmUmGfAmGfAfGfUmAmCmCmUmGmUmGmAmAm | 495/125 | UmsUfsCmAmCmAfGmGmUmAmCmUmCmUfCmAfUmUmGmsUmsGm | 660/330 |
| AL0185076 | GmsAmsUmGmAfGmAfGfUfAmCmCmUmGmUmGmAmGmAm | 496/126 | UmsCfsUmCmAmCfAmGmGmUmAmCmUmCfUmCfAmUmCmsGmsUm | 661/331 |
| AL0185077 | GmsGmsAmGmAfGmUfAfCfCmUmGmUmGmAmGmCmAmAm | 497/127 | UmsUfsGmCmUmCfAmCmAmGmGmUmAmCfUmCfUmCmCmsUmsUm | 662/332 |
| AL0185078 | GmsAmsGmAmGfUmAfCfCfUmGmUmGmAmGmCmAmGmAm | 498/128 | UmsCfsUmGmCmUfCmAmCmAmGmGmUmAfCmUfCmUmCmsAmsUm | 663/333 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185081 | CmsUmsCmCmCfAmCfCfUfUmUmUmCmUmUmCmUmAmAm | 499/129 | UmsUfsAmGmAmAfGmAmAmAmAmGmGmUfGmGfGmAmGmsAmsCm | 664/334 |
| AL0185082 | GmsCmsUmCmCfCmAfCfCfUmUmUmUmCmUmUmCmUmAm | 500/130 | UmsAfsGmAmAmGfAmAmAmAmGmGmUmGfGmGfAmGmCmsCmsUm | 665/335 |
| AL0185083 | GmsUmsCmUmCfCmCfAfCfCmUmUmUmUmCmUmUmCmAm | 501/131 | UmsGfsAmAmGmAfAmAmAmGmGmUmGmGfGmAfGmAmCmsUmsGm | 666/336 |
| AL0185084 | GmsGmsUmCmUfCmCfCfAfCmCmUmUmUmUmCmUmUmAm | 502/132 | UmsAfsAmGmAmAfAmAmGmGmUmGmGmGfAmGfAmCmCmsGmsGm | 667/337 |
| AL0185085 | CmsCmsCmAmCfCmUfUfUfUmCmUmUmCmUmAmAmUmAm | 503/133 | UmsAfsUmUmAmGfAmAmGmAmAmAmAmGfGmUfGmGmGmsAmsGm | 668/338 |
| AL0185086 | CmsCmsAmCmCfUmUfUfUfCmUmUmCmUmAmAmUmGmAm | 504/134 | UmsCfsAmUmUmAfGmAmAmGmAmAmAmAfGmGfUmGmGmsGmsAm | 669/339 |
| AL0185087 | CmsAmsCmCmUfUmUfUfCfUmUmCmUmAmAmUmGmAmAm | 505/135 | UmsUfsCmAmUmUfAmGmAmAmGmAmAmAfAmGfGmUmGmsGmsGm | 670/340 |
| AL0185088 | GmsCmsCmUmUfUmUfCfUfUmCmUmAmAmUmGmAmGmAm | 506/136 | UmsCfsUmCmAmUfUmAmGmAmAmGmAmAfAmAfGmGmCmsGmsGm | 671/341 |
| AL0185089 | CmsCmsUmUmUfUmCfUfUfCmUmAmAmUmGmAmGmUmAm | 507/137 | UmsAfsCmUmCmAfUmUmAmGmAmAmGmAfAmAfAmGmGmsUmsGm | 672/342 |
| AL0185090 | CmsUmsUmUmUfCmUfUfCfUmAmAmUmGmAmGmUmCmAm | 508/138 | UmsGfsAmCmUmCfAmUmUmAmGmAmAmGfAmAfAmAmGmsGmsUm | 786/343 |
| AL0185091 | GmsGmsCmCmGmUmUfUmCfUfCfCmUmUmGmGmUmCmUmAmAm | 471/101 | (APU)sUfsAmGmAmCfCmAmAmGmGmAmGmAfAmAfCmGmGmCmCmsGmsCm | 673/311 |
| AL0185092 | GmsCmsCmGmUmUmUfCmUfCfGfUmUmGmGmUmCmUmAmAmAm | 478/108 | (APU)sUfsUmAmGmAfCmCmAmAmGmGmAmGfAmAfAmCmGmGmCmsUmsGm | 674/286 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185093 | GmsCmsGmUmUmUmCfUmCfCfUfUmGmGmUmCmUmAmAmGmAm | 483/113 | (APU)sCfsUmUmAmGfAmCmCmAmAmGmGmAfGmAfAmAmCmGmCmsCmsUm | 675/319 |
| AL0185094 | CmsCmsGmUmUmUmCfUmCfCfUfUmGmGmUmCmUmAmAmGmAm | 481/111 | (APU)sCfsUmUmAmGfAmCmCmAmAmGmGmAfGmAfAmAmCmGmGmsCmsUm | 676/317 |
| AL0185095 | GmsUmsCmAmUmCmCfAmCfAfAfUmGmAmGmAmGmUmAmCmAm | 425/1 | UmsGfsUmAmCm(Tgn)CmUmCmAmUmUmGmUfGmGfAmUmGmAmCmsGmsAm | 677/220 |
| AL0185096 | GmsGmsCmCmGmUmUfUmCfUfCfCmUmUmGmGmUmCmUmAmAm | 471/101 | (VPUm)sUfsAmGmAmCfCmAmAmGmGmAmGmAfAmAfCmGmGmCmCmsGmsCm | 678/311 |
| AL0185097 | GmsCmsCmGmUmUmUfCmUfCfGfUmUmGmGmUmCmUmAmAmAm | 478/108 | (VPUm)sUfsUmAmGmAfCmCmAmAmGmGmAmGfAmAfAmCmGmGmCmsUmsGm | 679/286 |
| AL0185098 | GmsCmsGmUmUmUmCfUmCfCfUfUmGmGmUmCmUmAmAmGmAm | 483/113 | (VPUm)sCfsUmUmAmGfAmCmCmAmAmGmGmAfGmAfAmAmCmGmCmsCmsUm | 680/319 |
| AL0185099 | CmsCmsGmUmUmUmCfUmCfCfUfUmGmGmUmCmUmAmAmGmAm | 481/111 | (VPUm)sCfsUmUmAmGfAmCmCmAmAmGmGmAfGmAfAmAmCmGmGmsCmsUm | 681/317 |
| AL0185100 | AmsGmsCmAmCmCmUfGfAfAmUmUmUmCmUmGmUmUmUm | 509/139 | AmsAfsAmCmAmGfAmAmAmUmUmCmAmGfGmUfGmCmUmsUmsGm | 682/344 |
| AL0185101 | GmsUmsCmUmGmUmAfAfUfAmCmCmUmUmAmGmUmUmUm | 510/140 | AmsAfsAmCmUmAfAmGmGmUmAmUmUmAfCmAfGmAmCmsAmsCm | 683/345 |
| AL0185102 | CmsAmsAmAmUmGmCfAfCfCmUmCmCmCmAmGmUmUmUm | 511/141 | AmsAfsAmCmUmGfGmGmAmGmGmUmGmCfAmUfUmUmGmsUmsGm | 684/346 |
| AL0185103 | UmsGmsGmAmCmAmGfCfAfCmCmCmUmGmGmCmUmUmUm | 512/142 | AmsAfsAmGmCmCfAmGmGmGmUmGmCmUfGmUfCmCmAmsCmsAm | 685/347 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185104 | CmsUmsUmCmUmAmAfUfGfAmGmUmCmGmAmCmUmUmUm | 513/143 | AmsAfsAmGmUmCfGmAmCmUmCmAmUmUfAmGfAmAmGmsAmsAm | 686/348 |
| AL0185105 | AmsGmsGmUmGmGmAfGfGfGmUmCmUmCmAmCmUmUmUm | 514/144 | AmsAfsAmGmUmGfAmGmAmCmCmCmUmCfCmAfCmCmUmsUmsGm | 687/349 |
| AL0185106 | GmsAmsAmCmCmAmUfAfGfCmUmGmGmUmUmAmUmUmUm | 515/145 | AmsAfsAmUmAmAfCmCmAmGmCmUmAmUfGmGfUmUmCmsCmsGm | 688/350 |
| AL0185107 | UmsUmsGmCmCmUmUfCfGfGmUmUmUmGmUmAmUmUmUm | 516/146 | AmsAfsAmUmAmCfAmAmAmCmCmGmAmAfGmGfCmAmAmsUmsGm | 689/351 |
| AL0185108 | GmsGmsUmUmUmGmUfAfUfUmUmAmGmUmGmUmCmUmUm | 517/147 | AmsAfsGmAmCmAfCmUmAmAmAmUmAmCfAmAfAmCmCmsGmsAm | 690/352 |
| AL0185109 | CmsAmsAmGmUmUmGfAfGfAmAmCmAmAmAmAmAmUmUm | 518/148 | AmsAfsUmUmUmUfUmGmUmUmCmUmCmAfAmCfUmUmGmsAmsAm | 691/353 |
| AL0185110 | GmsUmsUmUmGmUmGfAfAfAmCmAmAmAmAmAmAmGmUm | 519/149 | AmsCfsUmUmUmUfUmUmGmUmUmUmCmAfCmAfAmAmCmsAmsAm | 692/354 |
| AL0185111 | UmsGmsGmAmCmAmAfGfGfUmGmGmAmGmGmGmUmCmUm | 520/150 | AmsGfsAmCmCmCfUmCmCmAmCmCmUmUfGmUfCmCmAmsGmsGm | 693/355 |
| AL0185112 | CmsUmsCmAmCmUmUfUfCfCmAmGmCmAmAmAmAmCmUm | 521/151 | AmsGfsUmUmUmUfGmCmUmGmGmAmAmAfGmUfGmAmGmsAmsCm | 694/356 |
| AL0185113 | AmsCmsUmUmCmUmUfGfGfGmCmUmUmCmCmGmUmAmUm | 522/152 | AmsUfsAmCmGmGfAmAmGmCmCmCmAmAfGmAfAmGmUmsUmsGm | 695/357 |
| AL0185114 | GmsUmsCmUmUmGmAfAfUfGmUmAmAmGmAmAmCmAmUm | 523/153 | AmsUfsGmUmUmCfUmUmAmCmAmUmUmCfAmAfGmAmCmsAmsCm | 696/358 |
| AL0185115 | UmsUmsCmCmUmGmUfUfUfGmCmUmGmUmGmUmAmUmGm | 524/154 | CmsAfsUmAmCmAfCmAmGmCmAmAmAmCfAmGfGmAmAmsUmsGm | 697/359 |
| AL0185116 | GmsGmsCmAmAmGmAfAfCfCmAmGmUmGmUmUmUmAmGm | 525/155 | CmsUfsAmAmAmCfAmCmUmGmGmUmUmCfUmUfGmCmCmsUmsCm | 698/360 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185117 | GmsAmsUmUmGmAmCfAfGfGmUmUmCmAmUmGmCmAmGm | 526/156 | CmsUfsGmCmAmUfGmAmAmCmCmUmGmUfCmAfAmUmCmsUmsUm | 699/361 |
| AL0185118 | GmsUmsAmUmAmUmAfUfGfGmCmAmUmGmCmAmCmAmGm | 527/157 | CmsUfsGmUmGmCfAmUmGmCmCmAmUmAfUmAfUmAmCmsGmsGm | 700/362 |
| AL0185119 | CmsCmsAmUmAmGmCfUfGfGmUmUmAmUmUmUmCmUmCm | 528/158 | GmsAfsGmAmAmAfUmAmAmCmCmAmGmCfUmAfUmGmGmsUmsUm | 701/363 |
| AL0185120 | AmsUmsGmUmAmAmGfAfAfCmAmUmGmAmCmCmUmCmCm | 529/159 | GmsGfsAmGmGmUfCmAmUmGmUmUmCmUfUmAfCmAmUmsUmsCm | 702/364 |
| AL0185121 | CmsCmsGmUmAmUmAfUfAfUmGmGmCmAmUmGmCmAmCm | 530/160 | GmsUfsGmCmAmUfGmCmCmAmUmAmUmAfUmAfCmGmGmsAmsAm | 703/365 |
| AL0185122 | GmsUmsGmUmCmUmUfGfAfAmUmGmUmAmAmGmAmAmCm | 531/161 | GmsUfsUmCmUmUfAmCmAmUmUmCmAmAfGmAfCmAmCmsUmsAm | 704/366 |
| AL0185123 | AmsGmsGmCmAmAmGfAfAfCmCmAmGmUmGmUmUmUmAm | 532/162 | UmsAfsAmAmCmAfCmUmGmGmUmUmCmUfUmGfCmCmUmsCmsCm | 705/367 |
| AL0185124 | CmsAmsUmUmCmCmUfGfUfUmUmGmCmUmGmUmGmUmAm | 533/163 | UmsAfsCmAmCmAfGmCmAmAmAmCmAmGfGmAfAmUmGmsGmsGm | 706/368 |
| AL0185125 | GmsGmsGmUmUmUmUfAfAfAmAmUmUmAmAmAmGmUmAm | 534/164 | UmsAfsCmUmUmUfAmAmUmUmUmUmAmAfAmAfCmCmCmsAmsAm | 707/369 |
| AL0185126 | CmsUmsUmCmUmUmGfGfGfCmUmUmCmCmGmUmAmUmAm | 535/165 | UmsAfsUmAmCmGfGmAmAmGmCmCmCmAfAmGfAmAmGmsUmsUm | 708/370 |
| AL0185127 | GmsUmsUmUmUmAmAfAfAfUmUmAmAmAmGmUmAmUmAm | 536/166 | UmsAfsUmAmCmUfUmUmAmAmUmUmUmUfAmAfAmAmCmsCmsCm | 709/371 |
| AL0185128 | UmsAmsUmAmUmGmGfCfAfUmGmCmAmCmAmGmUmGmAm | 537/167 | UmsCfsAmCmUmGfUmGmCmAmUmGmCmCfAmUfAmUmAmsUmsAm | 710/372 |
| AL0185129 | CmsUmsUmGmAmAmUfGfUfAmAmGmAmAmCmAmUmGmAm | 538/168 | UmsCfsAmUmGmUfUmCmUmUmAmCmAmUfUmCfAmAmGmsAmsCm | 711/373 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185130 | GmsAmsUmCmUmUmAfUfGfAmCmCmUmGmCmAmGmGmAm | 539/169 | UmsCfsCmUmGmCfAmGmGmUmCmAmUmAfAmGfAmUmCmsCmsUm | 712/374 |
| AL0185131 | GmsGmsUmGmGmAmCfAfAfCmAmGmCmAmCmCmUmCmAm | 540/170 | UmsGfsAmGmGmUfGmCmUmGmUmUmGmUfCmCfAmCmCmsCmsAm | 713/375 |
| AL0185132 | AmsGmsAmUmUmGmAfCfAfGmGmUmUmCmAmUmGmCmAm | 541/171 | UmsGfsCmAmUmGfAmAmCmCmUmGmUmCfAmAfUmCmUmsUmsCm | 714/376 |
| AL0185133 | GmsUmsUmCmAmUmGfCfAfGmGmCmUmGmUmGmAmCmAm | 542/172 | UmsGfsUmCmAmCfAmGmCmCmUmGmCmAfUmGfAmAmCmsCmsUm | 715/377 |
| AL0185134 | CmsAmsGmGmAmGmUfUfCfUmGmGmGmUmGmGmAmCmAm | 543/173 | UmsGfsUmCmCmAfCmCmCmAmGmAmAmCfUmCfCmUmGmsGmsGm | 716/378 |
| AL0185135 | CmsGmsUmAmUmAmUfAfUfGmGmCmAmUmGmCmAmCmAm | 544/174 | UmsGfsUmGmCmAfUmGmCmCmAmUmAmUfAmUfAmCmGmsGmsAm | 717/379 |
| AL0185136 | AmsCmsUmCmCmCmUfCfAfAmCmUmGmGmAmUmGmAmAm | 545/175 | UmsUfsCmAmUmCfCmAmGmUmUmGmAmGfGmGfAmGmUmsUmsUm | 718/380 |
| AL0185137 | GmsGmsGmUmCmUmCfAfCfUmUmUmCmCmAmGmCmAmAm | 546/176 | UmsUfsGmCmUmGfGmAmAmAmGmUmGmAfGmAfCmCmCmsUmsCm | 719/381 |
| AL0185138 | AmsGmsGmAmGmUmUfCfUfGmGmGmUmGmGmAmCmAmAm | 547/177 | UmsUfsGmUmCmCfAmCmCmCmAmGmAmAfCmUfCmCmUmsGmsGm | 720/382 |
| AL0185139 | GmsAmsCmCmAmGmCfUfUfGmUmUmUmGmUmGmAmAmAm | 548/178 | UmsUfsUmCmAmCfAmAmAmCmAmAmGmCfUmGfGmUmCmsGmsGm | 721/383 |
| AL0185140 | CmsCmsUmGmUmGmAfGfCfAmGmCmUmGmGmCmAmAmAm | 549/179 | UmsUfsUmGmCmCfAmGmCmUmGmCmUmCfAmCfAmGmGmsUmsAm | 722/384 |
| AL0185141 | GmsGmsUmCmUmCmAfCfUfUmUmCmCmAmGmCmAmAmAm | 550/180 | UmsUfsUmGmCmUfGmGmAmAmAmGmUmGfAmGfAmCmCmsCmsUm | 723/385 |
| AL0185142 | CmsAmsAmAmAmAmUfUfGfGmGmUmUmUmUmAmAmAmAm | 551/181 | UmsUfsUmUmAmAfAmAmCmCmCmAmAmUfUmUfUmUmGmsUmsUm | 724/386 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185143 | GmsCmsUmUmGmUmUfUfGfUmGmAmAmAmCmAmAmAmAm | 552/182 | UmsUfsUmUmGmUfUmUmCmAmCmAmAmAfCmAfAmGmCmsUmsGm | 725/387 |
| AL0185144 | AmsAmsGmAmCmGmUfUfUfAmUmUmAmCmUmAmAmCmAm | 553/183 | UmsGfsUmUmAmGfUmAmAmUmAmAmAmCfGmUfCmUmUmsGmsCm | 726/388 |
| AL0185145 | CmsGmsAmCmCmAmGfCfUfUmGmUmUmUmGmUmGmAmAm | 554/184 | UmsUfsCmAmCmAfAmAmCmAmAmGmCmUfGmGfUmCmGmsGmsUm | 727/389 |
| AL0185146 | CmsUmsUmGmUmUmUfGfUfGmAmAmAmCmAmAmAmAmAm | 555/185 | UmsUfsUmUmUmGfUmUmUmCmAmCmAmAfAmCfAmAmGmsCmsUm | 728/390 |
| AL0185147 | AmsCmsAmAmCmUmUfCfUfCmGmGmUmGmAmCmUmCmAm | 556/186 | UmsGfsAmGmUmCfAmCmCmGmAmGmAmAfGmUfUmGmUmsCmsCm | 729/391 |
| AL0185148 | UmsGmsGmUmCmGmGfGfAfUmGmCmUmGmGmCmCmAmAm | 557/187 | UmsUfsGmGmCmCfAmGmCmAmUmCmCmCfGmAfCmCmAmsUmsUm | 730/392 |
| AL0185149 | CmsUmsGmAmAmUmUfUfCfUmGmUmUmUmGmAmAmUmGm | 558/188 | CmsAfsUmUmCmAfAmAmCmAmGmAmAmAfUmUfCmAmGmsGmsUm | 731/393 |
| AL0185150 | AmsGmsGmAmUmCmUfUfAfUmGmAmCmCmUmGmCmAmGm | 559/189 | CmsUfsGmCmAmGfGmUmCmAmUmAmAmGfAmUfCmCmUmsUmsGm | 732/394 |
| AL0185151 | GmsAmsGmAmAmGmAfUfUfGmAmCmAmGmGmUmUmCmAm | 560/190 | UmsGfsAmAmCmCfUmGmUmCmAmAmUmCfUmUfCmUmCmsAmsGm | 733/395 |
| AL0185152 | GmsUmsUmCmCmAmAfAfAfAmGmAmAmUmUmCmCmAmAm | 561/191 | UmsUfsGmGmAmAfUmUmCmUmUmUmUmUfGmGfAmAmCmsAmsGm | 734/396 |
| AL0185153 | UmsGmsUmAmAmGmAfAfCfAmUmGmAmCmCmUmCmCmGm | 562/192 | CmsGfsGmAmGmGfUmCmAmUmGmUmUmCfUmUfAmCmAmsUmsUm | 735/397 |
| AL0185154 | GmsGmsCmUmGmUmAfCfAfGmGmGmCmCmUmGmCmUmAm | 563/193 | UmsAfsGmCmAmGfGmCmCmCmUmGmUmAfCmAfGmCmCmsUmsGm | 736/398 |
| AL0185155 | CmsAmsAmCmUmGmGfAfUfGmAmAmGmAmAmAmCmUmAm | 564/194 | UmsAfsGmUmUmUfCmUmUmCmAmUmCmCfAmGfUmUmGmsAmsGm | 737/399 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185156 | CmsCmsCmUmCmAmAfCfUfGmGmAmUmGmAmAmGmAmAm | 565/195 | UmsUfsCmUmUmCfAmUmCmCmAmGmUmUfGmAfGmGmGmsAmsGm | 738/400 |
| AL0185157 | UmsAmsCmGmUmGmAfAfAfGmAmUmGmCmAmAmGmCmAm | 566/196 | UmsGfsCmUmUmGfCmAmUmCmUmUmUmCfAmCfGmUmAmsUmsUm | 739/401 |
| AL0185158 | GmsCmsAmAmAmGmGfCfCfAmGmCmAmGmCmAmGmAmUm | 567/197 | AmsUfsCmUmGmCfUmGmCmUmGmGmCmCfUmUfUmGmCmsCmsUm | 740/402 |
| AL0185159 | GmsCmsAmAmGmGmAfUfCfUmUmAmUmGmAmCmCmUmGm | 568/198 | CmsAfsGmGmUmCfAmUmAmAmGmAmUmCfCmUfUmGmCmsAmsGm | 741/403 |
| AL0185160 | GmsCmsUmGmAmCmAfGfGfCmUmAmCmAmGmGmCmAmAm | 569/199 | UmsUfsGmCmCmUfGmUmAmGmCmCmUmGfUmCfAmGmCmsUmsGm | 742/404 |
| AL0185161 | CmsCmsCmUmUmGmUfGfUfUmAmGmUmAmAmUmAmAmAm | 570/200 | UmsUfsUmAmUmUfAmCmUmAmAmCmAmCfAmAfGmGmGmsAmsGm | 743/405 |
| AL0185162 | GmsCmsAmCmCmUmGfAfAfUmUmUmCmUmGmUmUmUmGm | 571/201 | CmsAfsAmAmCmAfGmAmAmAmUmUmCmAfGmGfUmGmCmsUmsUm | 744/406 |
| AL0185163 | GmsAmsGmCmAmCmAfGfCfAmUmGmAmGmGmCmCmAmGm | 572/202 | CmsUfsGmGmCmCfUmCmAmUmGmCmUmGfUmGfCmUmCmsAmsGm | 745/407 |
| AL0185164 | CmsUmsCmCmCmUmUfGfUfGmUmUmAmGmUmAmAmUmAm | 573/203 | UmsAfsUmUmAmCfUmAmAmCmAmCmAmAfGmGfGmAmGmsAmsAm | 746/408 |
| AL0185165 | AmsCmsAmAmUmAmCfGfUfGmAmAmAmGmAmUmGmCmAm | 574/204 | UmsGfsCmAmUmCfUmUmUmCmAmCmGmUfAmUfUmGmUmsUmsCm | 747/409 |
| AL0185166 | GmsAmsAmCmAmAmUfAfCfGmUmGmAmAmAmGmAmUmGm | 575/205 | CmsAfsUmCmUmUfUmCmAmCmGmUmAmUfUmGfUmUmCmsAmsAm | 748/410 |
| AL0185167 | UmsUmsUmCmUmCmCfCfUfUmGmUmGmUmUmAmGmUmAm | 576/206 | UmsAfsCmUmAmAfCmAmCmAmAmGmGmGfAmGfAmAmAmsUmsAm | 749/411 |
| AL0185168 | CmsCmsAmAmUmGmCfCfGfGmGmAmAmGmCmCmCmAmAm | 577/207 | UmsUfsGmGmGmCfUmUmCmCmCmGmGmCfAmUfUmGmGmsCmsCm | 750/412 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185169 | UmsGmsCmAmAmGmCfAfCfCmUmGmAmAmUmUmUmCmUm | 578/208 | AmsGfsAmAmAmUfUmCmAmGmGmUmGmCfUmUfGmCmAmsUmsCm | 751/413 |
| AL0185170 | GmsGmsUmGmCmUmGfCfAfAmGmGmAmUmCmUmUmAmUm | 579/209 | AmsUfsAmAmGmAfUmCmCmUmUmGmCmAfGmCfAmCmCmsAmsGm | 752/414 |
| AL0185171 | CmsCmsAmCmAmCmAfGfCfUmGmAmCmAmGmGmCmUmAm | 580/210 | UmsAfsGmCmCmUfGmUmCmAmGmCmUmGfUmGfUmGmGmsUmsCm | 753/415 |
| AL0185172 | UmsUmsGmAmAmCmAfAfUfAmCmGmUmGmAmAmAmGmAm | 581/211 | UmsCfsUmUmUmCfAmCmGmUmAmUmUmGfUmUfCmAmAmsAmsAm | 754/416 |
| AL0185173 | GmsAmsCmAmCmCmGfAfAfGmAmCmAmAmGmUmUmGmAm | 582/212 | UmsCfsAmAmCmUfUmGmUmCmUmUmCmGfGmUfGmUmCmsAmsAm | 755/417 |
| AL0185174 | GmsAmsGmCmCmAmGfUfGfUmGmGmAmCmAmGmCmAmCm | 583/213 | GmsUfsGmCmUmGfUmCmCmAmCmAmCmUfGmGfCmUmCmsCmsCm | 756/418 |
| AL0185175 | UmsGmsGmAmAmGmGfAfCfAmAmGmAmAmCmUmGmCmAm | 584/214 | UmsGfsCmAmGmUfUmCmUmUmGmUmCmCfUmUfCmCmAmsAmsGm | 757/419 |
| AL0185176 | CmsAmsAmCmUmUmCfUfCfGmGmUmGmAmCmUmCmAmAm | 585/215 | UmsUfsGmAmGmUfCmAmCmCmGmAmGmAfAmGfUmUmGmsUmsCm | 758/420 |
| AL0185177 | AmsAmsCmGmUmCmUfUfGfCmCmAmCmAmAmUmAmAmGm | 586/216 | CmsUfsUmAmUmUfGmUmGmGmCmAmAmGfAmCfGmUmUmsUmsAm | 759/421 |
| AL0185178 | CmsUmsGmAmGmAmAfGfAfUmUmGmAmCmAmGmGmUmUm | 587/217 | AmsAfsCmCmUmGfUmCmAmAmUmCmUmUfCmUfCmAmGmsCmsAm | 760/422 |
| AL0185179 | CmsCmsAmGmUmUmUfGfCfUmGmGmGmUmUmUmAmUmUm | 588/218 | AmsAfsUmAmAmAfCmCmCmAmGmCmAmAfAmCfUmGmGmsGmsAm | 761/423 |
| AL0185180 | AmsCmsUmUmCmUmCfGfGfUmGmAmCmUmCmAmAmGmUm | 589/219 | AmsCfsUmUmGmAfGmUmCmAmCmCmGmAfGmAfAmGmUmsUmsGm | 762/424 |
| AL0185181 | CmsCmsGmUmUmUmCmUmCfCfUfUmGmGmUmCmUmAmAmGmAm | 590/111 | (APU)sCfsUmUmAmGfAmCmCfAmAmGmGmAfGmAfAmAmCmGmGmsCmsUm | 787/317 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185182 | GmsCmsCmGmUmUmUmCmUfCfGfUmUmGmGmUmCmUmAmAmAm | 591/108 | (APU)sUfsUmAmGmAfCmCmAfAmGmGmAmGfAmAfAmCmGmGmCmsUmsGm | 788/286 |
| AL0185183 | GmsCmsGmUmUmUmCmUmCfCfUfUmGmGmUmCmUmAmAmGmAm | 592/113 | (APU)sCfsUmUmAmGfAmCmCfAmAmGmGmAfGmAfAmAmCmGmCmsCmsUm | 774/319 |
| AL0185184 | CmsAmsGmUmCmUmCmCmCfAfGfCmUmUmUmUmCmUmUmCmUm | 593/107 | AmsGfsAmAmGmAfAmAmAfGmGmUmGmGfGmAfGmAmCmUmGmsGmsGm | 610/281 |
| AL0185185 | GmsCmsGmUmUmUmCmUmCfCfUfUmGmGmUmCmUmAmAmGmUm | 594/112 | AmsCfsUmUmAmGfAmCmCmAmAmGmGmAfGmAfAmAmCmGmCmsCmsUm | 647/318 |
| AL0185186 | GmsCmsGmUmUmUmCmUmCfCfUfUmGmGmUmCmUmAmAmGmUm | 594/112 | AmsCfsUmUmAmGfAmCfCmAmAmGmGmAfGmAfAmAmCmGmCmsCmsUm | 763/318 |
| AL0185187 | GmsCmsGmUmUmUmCmUmCfCfUfUmGmGmUmCmUmAmAmGmUm | 594/112 | AmsCfsUmUmAmGfAmCmCfAmAmGmGmAfGmAfAmAmCmGmCmsCmsUm | 764/318 |
| AL0185188 | CmsUmsCmCmCfAmCfCfUfUmUmUmCmUmUmCmUmAmAm | 499/129 | (VPUm)sUfsAmGmAmAfGmAmAmAmAmGmGmUfGmGfGmAmGmsAmsCm | 765/334 |
| AL0185189 | CmsCmsCmAmCfCmUfUfUfUmCmUmUmCmUmAmAmUmAm | 503/133 | (VPUm)sAfsUmUmAmGfAmAmGmAmAmAmAmGfGmUfGmGmGmsAmsGm | 766/338 |
| AL0185190 | CmsAmsCmCmUfUmUfUfCfUmUmCmUmAmAmUmGmAmAm | 505/135 | (VPUm)sUfsCmAmUmUfAmGmAmAmGmAmAmAfAmGfGmUmGmsGmsGm | 767/340 |
| AL0185191 | CmsCmsUmUmUfUmCfUfUfCmUmAmAmUmGmAmGmUmAm | 507/137 | (VPUm)sAfsCmUmCmAfUmUmAmGmAmAmGmAfAmAfAmGmGmsUmsGm | 768/342 |
| AL0185192 | CmsUmsCmCmCfAmCfCfUfUmUmUmCmUmUmCmUmAmAm | 499/129 | (APU)sUfsAmGmAmAfGmAmAmAmAmGmGmUfGmGfGmAmGmsAmsCm | 769/334 |
| AL0185193 | CmsCmsCmAmCfCmUfUfUfUmCmUmUmCmUmAmAmUmAm | 503/133 | (APU)sAfsUmUmAmGfAmAmGmAmAmAmAmGfGmUfGmGmGmsAmsGm | 770/338 |
| AL0185194 | CmsAmsCmCmUfUmUfUfCfUmUmCmUmAmAmUmGmAmAm | 505/135 | (APU)sUfsCmAmUmUfAmGmAmAmGmAmAmAfAmGfGmUmGmsGmsGm | 771/340 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185195 | CmsCmsUmUmUfUmCfUfUfCmUmAmAmUmGmAmGmUmAm | 507/137 | (APU)sAfsCmUmCmAfUmUmAmGmAmAmGmAfAmAfAmGmGmsUmsGm | 772/342 |
| AL0185196 | CmsAmsGmUmCmUmCmCmCfAfGfCmUmUmUmUmCmUmUmCmUm | 593/107 | AmsGfsAmAmGmAfAmAmAfGmGmUmGmGfGmAfGmAmCmUmGmsGmsGm | 610/281 |
| AL0185197 | GmsCmsGmUmUmUmCmUmCfCfUfUmGmGmUmCmUmAmAmGmAm | 592/113 | UmsCfsUmUmAmGfAmCmCfAmAmGmGmAfGmAfAmAmCmGmCmsCmsUm | 773/319 |
| AL0185198 | GmsCmsGmUmUmUmCmUmCfCfUfUmGmGmUmCmUmAmAmGmAm | 592/113 | (APU)sCfsUmUmAmGfAmCmCfAmAmGmGmAfGmAfAmAmCmGmCmsCmsUm | 774/319 |
| AL0185199 | GmsCmsGmUmUmUmCmUmCfCfUfUmGmGmUmCmUmAmAmGmAm | 592/113 | (VPUm)sCfsUmUmAmGfAmCmCfAmAmGmGmAfGmAfAmAmCmGmCmsCmsUm | 775/319 |
| AL0185200 | CmsUmsCmCmCmAmCfCfUfUmUmUmCmUmUmCmUmAmAm | 595/129 | (APU)sUfsAmGmAmAfGmAmAfAmAmGmGmUfGmGfGmAmGmsAmsCm | 776/334 |
| AL0185201 | CmsCmsCmAmCmCmUfUfUfUmCmUmUmCmUmAmAmUmAm | 596/133 | (APU)sAfsUmUmAmGfAmAmGfAmAmAmAmGfGmUfGmGmGmsAmsGm | 777/338 |
| AL0185202 | CmsAmsCmCmUmUmUfUfCfUmUmCmUmAmAmUmGmAmAm | 597/135 | (APU)sUfsCmAmUmUfAmGmAfAmGmAmAmAfAmGfGmUmGmsGmsGm | 778/340 |
| AL0185203 | CmsCmsUmUmUmUmCfUfUfCmUmAmAmUmGmAmGmUmAm | 598/137 | (APU)sAfsCmUmCmAfUmUmAfGmAmAmGmAfAmAfAmGmGmsUmsGm | 779/342 |
| AL0185204 | CmsAmsAmGmUmUmGfAfGfAmAmCmAmAmAmAmAmUmUm | 518/148 | AmsAfsUmUmUmUfUmGmUfUmCmUmCmAfAmCfUmUmGmsAmsAm | 780/353 |
| AL0185205 | AmsGmsGmCmAmAmGfAfAfCmCmAmGmUmGmUmUmUmAm | 532/162 | (APU)sAfsAmAmCmAfCmUmGfGmUmUmCmUfUmGfCmCmUmsCmsCm | 781/367 |
| AL0185206 | GmsUmsUmUmUmAmAfAfAfUmUmAmAmAmGmUmAmUmAm | 536/166 | (APU)sAfsUmAmCmUfUmUmAfAmUmUmUmUfAmAfAmAmCmsCmsCm | 782/371 |
| AL0185207 | GmsAmsCmCmAmGmCfUfUfGmUmUmUmGmUmGmAmAmAm | 548/178 | (APU)sUfsUmCmAmCfAmAmAfCmAmAmGmCfUmGfGmUmCmsGmsGm | 783/383 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185208 | GmsGmsUmCmUmCmAfCfUfUmUm CmCmAmGmCmAmAmAm | 550/180 | (APU)sUfsUmGmCmUfGmGmAfAmAm GmUmGfAmGfAmCmCmsCmsUm | 784/385 |
| AL0185209 | CmsGmsAmCmCmAmGfCfUfUmGm UmUmUmGmUmGmAmAm | 554/184 | (APU)sUfsCmAmCmAfAmAmCfAmAm GmCmUfGmGfUmCmGmsGmsUm | 785/389 |
| AL0185210 | GmsUmsCmCmCmAmCfCfUfUmUm UmCmUmUmCmUmAmAm | 822/789 | (VPUm)sUfsAmGmAmAfGmAmAfAmA mGmGmUfGmGfGmAmCmsAmsCm | 839/805 |
| AL0185211 | GmsUmsCmCmCmAmCfCfAfUmUm UmCmUmUmCmUmAmAm | 823/790 | (VPUm)sUfsAmGmAmAfGmAmAfAmA mGmGmUfGmGfGmAmCmsAmsCm | 839/805 |
| AL0185212 | GmsUmsCmCmCmAmCfCfCfUmUm UmCmUmUmCmUmAmAm | 824/791 | (VPUm)sUfsAmGmAmAfGmAmAfAmA mGmGmUfGmGfGmAmCmsAmsCm | 839/805 |
| AL0185213 | GmsUmsCmCmCmAmCfCfGfUmUm UmCmUmUmCmUmAmAm | 825/792 | (VPUm)sUfsAmGmAmAfGmAmAfAmA mGmGmUfGmGfGmAmCmsAmsCm | 839/805 |
| AL0185214 | GmsUmsCmCmCmAmCfCfUfAmUm UmCmUmUmCmUmAmAm | 826/793 | (VPUm)sUfsAmGmAmAfGmAmAfAmA mGmGmUfGmGfGmAmCmsAmsCm | 839/805 |
| AL0185215 | GmsUmsCmCmCmAmCfCfUfCmUm UmCmUmUmCmUmAmAm | 827/794 | (VPUm)sUfsAmGmAmAfGmAmAfAmA mGmGmUfGmGfGmAmCmsAmsCm | 839/805 |
| AL0185216 | GmsUmsCmCmCmAmCfCfUfGmUm UmCmUmUmCmUmAmAm | 828/795 | (VPUm)sUfsAmGmAmAfGmAmAfAmA mGmGmUfGmGfGmAmCmsAmsCm | 839/805 |
| AL0185217 | GmsUmsCmCmCmAmCfCfUfUmAm UmCmUmUmCmUmAmAm | 829/796 | (VPUm)sUfsAmGmAmAfGmAmAfAmA mGmGmUfGmGfGmAmCmsAmsCm | 839/805 |
| AL0185218 | GmsUmsCmCmCmAmCfCfUfUmCm UmCmUmUmCmUmAmAm | 830/797 | (VPUm)sUfsAmGmAmAfGmAmAfAmA mGmGmUfGmGfGmAmCmsAmsCm | 839/805 |
| AL0185219 | GmsUmsCmCmCmAmCfCfUfUmGm UmCmUmUmCmUmAmAm | 831/798 | (VPUm)sUfsAmGmAmAfGmAmAfAmA mGmGmUfGmGfGmAmCmsAmsCm | 839/805 |
| AL0185220 | CmsCmsUmUmUmUmCfUfUfCmUm AmAmUmGmAmGmUmAm | 598/137 | (VPUm)sAfsCmUmCmAfUmUmAfGmA mAmGmAfAmAfAmGmGmsUmsGm | 840/342 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185221 | CmsCmsUmUmUmUmCfUmUfCfUmAmAmUmGmAmGmUmAm | 832/137 | (VPUm)sAfsCmUmCmAfUmUfAmGmAmAfGmAfAmAfAmGfGmsUmsGm | 841/342 |
| AL0185222 | CmsCmsUmUmUmUmCfUmUfCfUmAmAmUmGmAmGmUmAm | 832/137 | (VPUm)sAfsCm(Tgn)CmAfUmUfAmGmAmAfGmAfAmAfAmGfGmsUmsGm | 842/342 |
| AL0185223 | CmsCmsUmUmUmUmCfUmUfCfUmAmAmUmGmAmGmUmAm | 832/137 | (VPUm)sAfsCmUm(Cgn)AfUmUfAmGmAmAfGmAfAmAfAmGfGmsUmsGm | 843/342 |
| AL0185224 | CmsCmsUmUmUmUmCfUmUfCfUmAmAmUmGmAmGmUmAm | 832/137 | (VPUm)sAfsCmUmCm(Agn)UmUfAmGmAmAfGmAfAmAfAmGfGmsUmsGm | 844/342 |
| AL0185225 | CmsCmsUmUmUmUmCfUmUfCfUmAmAmUmGmAmGmUmAm | 832/137 | (VPUm)sAfsCmUmCmAf(Tgn)UfAmGmAmAfGmAfAmAfAmGfGmsUmsGm | 845/342 |
| AL0185226 | CmsCmsUmUmUmUmCfUmUfCfUmAmAmUmGmAmGmUmAm | 832/137 | (VPUm)sAfsCmUmCmAfUm(Tgn)AmGmAmAfGmAfAmAfAmGfGmsUmsGm | 846/342 |
| AL0185227 | CmsCmsUmUmUmUmCfUmUfCfUmAmAmUmGmAmGmUmAm | 832/137 | (VPUm)sAfsCm(U-2'5')CmAfUmUfAmGmAmAfGmAfAmAfAmGfGmsUmsGm | 847/342 |
| AL0185228 | CmsCmsUmUmUmUmCfUmUfCfUmAmAmUmGmAmGmUmAm | 832/137 | (VPUm)sAfsCmUm(C-2'5')AfUmUfAmGmAmAfGmAfAmAfAmGfGmsUmsGm | 848/342 |
| AL0185229 | CmsCmsUmUmUmUmCfUmUfCfUmAmAmUmGmAmGmUmAm | 832/137 | (VPUm)sAfsCmUmCm(A-2'5')UmUfAmGmAmAfGmAfAmAfAmGfGmsUmsGm | 849/342 |
| AL0185230 | CmsCmsUmUmUmUmCfUmUfCfUmAmAmUmGmAmGmUmAm | 832/137 | (VPUm)sAfsCmUmCmAf(U-2'5')UfAmGmAmAfGmAfAmAfAmGfGmsUmsGm | 850/342 |
| AL0185231 | CmsCmsUmUmUmUmCfUmUfCfUmAmAmUmGmAmGmUmAm | 832/137 | (VPUm)sAfsCmUmCmAfUm(U-2'5')AmGmAmAfGmAfAmAfAmGfGmsUmsGm | 851/342 |
| AL0185232 | GmsCmsGmUmUmUmCmUmCfCfUfUmGmGmUmCmUmAmAmGmAm | 592/113 | (VPUm)sCfsUmUmAmGfAmCmCfAmAmImGmAfGmAfAmAmCmGmCmsCmsUm | 852/806 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185233 | GmsCmsGmUmUmUmCmUmCfCfUf UmGmGmUmCmUmAmAmGmAm | 592/113 | (VPUm)sCfsUmUmAmGfAmCmCfAmA mGmImAfGmAfAmAmCmGmCmsCmsU m | 853/807 |
| AL0185234 | GmsCmsGmUmUmUmCmUmCfCfUf UmGmGmUmCmUmAmAmGmAm | 592/113 | (VPUm)sCfsUmUmAmGfAmCmCfAmA mGmGmAfImAfAmAmCmGmCmsCmsU m | 854/808 |
| AL0185235 | GmsCmsGmUmUmUmCmUmCfCfUf UmGmGmUmCmUmAmAmGmAm | 592/113 | (VPUm)sCfsUmUmAmGfAmCmCfAmA mGmGmAfGmAfImAmCmGmCmsCmsU m | 855/809 |
| AL0185236 | GmsCmsGmUmUmUmCmUmCfCfUf UmGmGmUmCmUmAmAmGmAm | 592/113 | (VPUm)sCfsUmUmAmGfAmCmCfAmA mGmGmAfGmAfAmImCmGmCmsCmsU m | 856/810 |
| AL0185237 | GmsCmsGmUmUmUmCmUmCfCfUf UmGmGmUmCmUmAmAmGmAm | 592/113 | (VPUm)sCfsUmUmAmGfAmCmCfAmA mGmGmAfGmAfAmAmCmImCmsCmsU m | 857/811 |
| AL0185238 | GmsCmsCmAmCmCmUfUfUfUmCm UmUmCmUmAmAmUmAm | 833/799 | (VPUm)sAfsUmUmAmGfAmAmGfAmA mAmAmGfGmUfGmGmCmsAmsGm | 858/812 |
| AL0185239 | GmsAmsCmCmUmUmUfUfCfUmUm CmUmAmAmUmGmAmAm | 834/800 | (VPUm)sUfsCmAmUmUfAmGmAfAmG mAmAmAfAmGfGmUmCmsGmsGm | 859/813 |
| AL0185240 | CmsAmsAmGmUmUmGfAfGfAmAm CmAmAmAmAmAmUmAm | 835/801 | (VPUm)sAfsUmUmUmUfUmGmUfUmC mUmCmAfAmCfUmUmGmsAmsAm | 860/814 |
| AL0185241 | GmsGmsGmCmAmAmGfAfAfCmCm AmGmUmGmUmUmUmAm | 836/802 | (VPUm)sAfsAmAmCmAfCmUmGfGmU mUmCmUfUmGfCmCmCmsCmsCm | 861/815 |
| AL0185242 | GmsGmsAmCmCmAmGfCfUfUmGm UmUmUmGmUmGmAmAm | 837/803 | (VPUm)sUfsCmAmCmAfAmAmCfAmA mGmCmUfGmGfUmCmCmsGmsUm | 862/816 |
| AL0185243 | CmsAmsCmCmAmGmCfUfUfGmUm UmUmGmUmGmAmAmAm | 838/804 | (VPUm)sUfsUmCmAmCfAmAmAfCmA mAmGmCfUmGfGmUmGmsGmsGm | 863/817 |
| AL0185244 | GmsCmsGmUmUmUmCmUmCfCfUf UmGmGmUmCmUmAmAmGmAm | 592/113 | (VPUm)sCfsUmUmAmGfAmCmCfAmIm GmGmAfGmAfAmAmCmGmCmsCmsU m | 864/818 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185245 | GmsCmsGmUmUmUmCmUmCfCfUfUmGmGmUmCmUmAmAmGmAm | 592/113 | (VPUm)sCfsUmUmAmGfAmCmCfImAmGmGmAfGmAfAmAmCmGmCmsCmsUm | 865/819 |
| AL0185246 | GmsCmsGmUmUmUmCmUmCfCfUfUmGmGmUmCmUmAmAmGmAm | 592/113 | (VPUm)sCfsUmUmAmGfImCmCfAmAmGmGmAfGmAfAmAmCmGmCmsCmsUm | 866/820 |
| AL0185247 | GmsCmsGmUmUmUmCmUmCfCfUfUmGmGmUmCmUmAmAmGmAm | 592/113 | (VPUm)sCfsUmUmImGfAmCmCfAmAmGmGmAfGmAfAmAmCmGmCmsCmsUm | 867/821 |
| AL0185248 | CmsCmsAmCmCmUmCmGmUfCfAfUmCmCmAmCmAmAmUmGmAm | 876/3 | UmsCfsAmUmUmGfUmGmGfAmUmGmAmCfGmAfGmGmUmGmGmsAmsAm | 600/222 |
| AL0185249 | GmsCmsAmCmCmUmCmGmUfCfAfUmCmCmAmCmAmAmUmGmAm | 877/868 | UmsCfsAmUmUmGfUmGmGfAmUmGmAmCfGmAfGmGmUmGmCmsAmsAm | 887/872 |
| AL0185250 | GmsCmsAmCmCmUmCmGmUfCfAfUmCmCmAmCmAmAmUmGmAm | 877/868 | (VPUm)sCfsAmUmUmGfUmGmGfAmUmGmAmCfGmAfGmGmUmGmCmsAmsAm | 888/872 |
| AL0185251 | GmsGmsCmAmUmGmCmAmCfAfGfUmGmAmGmCmUmAmUmGmAm | 878/14 | UmsCfsAmUmAmGfCmUmCfAmCmUmGmUfGmCfAmUmGmCmCmsAmsUm | 603/233 |
| AL0185252 | GmsGmsCmAmUmGmCmAmCfAfGfUmGmAmGmCmUmAmUmGmAm | 878/14 | (VPUm)sCfsAmUmAmGfCmUmCfAmCmUmGmUfGmCfAmUmGmCmCmsAmsUm | 889/233 |
| AL0185253 | UmsUmsGmCmUmGmCmUmGfAfGfAmAmGmAmUmUmGmAmCmAm | 879/19 | UmsGfsUmCmAmAfUmCmUfUmCmUmCmAfGmCfAmGmCmAmAmsCmsAm | 890/238 |
| AL0185254 | GmsUmsGmCmUmGmCmUmGfAfGfAmAmGmAmUmUmGmAmCmAm | 880/869 | UmsGfsUmCmAmAfUmCmUfUmCmUmCmAfGmCfAmGmCmAmCmsCmsAm | 891/873 |
| AL0185255 | GmsUmsGmCmUmGmCmUmGfAfGfAmAmGmAmUmUmGmAmCmAm | 880/869 | (VPUm)sGfsUmCmAmAfUmCmUfUmCmUmCmAfGmCfAmGmCmAmCmsCmsAm | 892/873 |
| AL0185256 | GmsAmsGmAmAmGmAmUmUfGfAfCmAmGmGmUmUmCmAmUmAm | 881/72 | UmsAfsUmGmAmAfCmCmUfGmUmCmAmAfUmCfUmUmCmUmCmsAmsGm | 615/290 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185257 | GmsAmsGmAmAmGmAmUmUfGfAfCmAmGmGmUmUmCmAmUmAm | 881/72 | (VPUm)sAfsUmGmAmAfCmCmUfGmUmCmAmAfUmCfUmUmCmUmCmsAmsGm | 893/290 |
| AL0185258 | GmsCmsUmGmAmAmCmAmGfCfGfUmUmUmUmUmUmUmUmGmAm | 882/74 | UmsCfsAmAmAmAfAmAmAfAmUmGmCmUfGmUfUmCmAmGmCmsAmsCm | 606/259 |
| AL0185259 | GmsCmsUmGmAmAmCmAmGfCfGfUmUmUmUmUmUmUmUmGmAm | 882/74 | (VPUm)sCfsAmAmAmAfAmAmAfAmUmGmCmUfGmUfUmCmAmGmCmsAmsCm | 894/259 |
| AL0185260 | GmsAmsGmAmGmAmGmAmGfCfCfCmAmCmAmGmAmGmUmCmUm | 883/46 | AmsGfsAmCmUmCfUmGmUfGmGmGmCmUfCmUfCmUmCmUmCmsAmsUm | 608/265 |
| AL0185261 | GmsAmsGmAmGmAmGmAmGfCfCfCmAmCmAmGmAmGmUmCmAm | 884/870 | UmsGfsAmCmUmCfUmGmUfGmGmGmCmUfCmUfCmUmCmUmCmsAmsUm | 895/874 |
| AL0185262 | GmsAmsGmAmGmAmGmAmGfCfCfCmAmCmAmGmAmGmUmCmAm | 884/870 | (VPUm)sGfsAmCmUmCfUmGmUfGmGmGmCmUfCmUfCmUmCmUmCmsAmsUm | 896/874 |
| AL0185263 | CmsCmsUmUmUmUmCmUmUfCfUfAmAmUmGmAmGmUmCmGmAm | 885/65 | UmsCfsGmAmCmUfCmAmUfUmAmGmAmAfGmAfAmAmAmGmGmsUmsGm | 611/284 |
| AL0185264 | GmsCmsUmUmUmUmCmUmUfCfUfAmAmUmGmAmGmUmCmGmAm | 886/871 | UmsCfsGmAmCmUfCmAmUfUmAmGmAmAfGmAfAmAmAmGmCmsUmsGm | 897/875 |
| AL0185265 | GmsCmsUmUmUmUmCmUmUfCfUfAmAmUmGmAmGmUmCmGmAm | 886/871 | (VPUm)sdCsGmAmdCUmdCAmUmUmAmdGAmAfGmAmAmAmAmGmCmsUmsGm | 898/875 |
| AL0185266 | GmsUmsGmCmUmGmCmUmGfAfGfAmAmGmAmUmUmGmAmCmAm | 880/869 | (VPUm)sdGsUmCmdAAmdTCmUmUmCmdTCmAfGmCmAmGmCmAmCmsCmsAm | 899/873 |
| AL0185267 | GmsAmsGmAmAmGmAmUmUfGfAfCmAmGmGmUmUmCmAmUmAm | 881/72 | (VPUm)sdAsUmGmdAAmdCCmUmGmUmdCAmAfUmCmUmUmCmUmCmsAmsGm | 900/290 |
| AL0185268 | GmsCmsUmGmAmAmCmAmGfCfGfUmUmUmUmUmUmUmUmGmAm | 882/74 | UmsdCsAmAmdAAmdAAmAmAmUmdGCmUfGmUmUmCmAmGmCmsAmsCm | 901/259 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185269 | GmsCmsUmGmAmAmCmAmGfCfGfUmUmUmUmUmUmUmUmGmAm | 882/74 | (VPUm)sdCsAmAmdAAmdAAmAmAmUmdGCmUfGmUmUmCmAmGmCmsAmsCm | 902/259 |
| AL0185270 | GmsAmsGmAmGmAmGmAmGfCfCfCmAmCmAmGmAmGmUmCmAm | 884/870 | (VPUm)sdGsAmCmdTCmdTGmUmGmGmdGCmUfCmUmCmUmCmUmCmsAmsUm | 903/874 |
| AL0185237 | GmsCmsGmUmUmUmCmUmCfCfUfUmGmGmUmCmUmAmAmGmAm | 592/113 | (VPUm)sCfsUmUmAmGfAmCmCfAmAmGmGmAfGmAfAmAmCmImCmsCmsUm | 857/811 |
| AL0185271 | GmsCmsGmUmUmUmCmUmCfCfUfUmGmGmUmCmUmAmAmGmAm | 592/113 | (VPUm)sCfsUmUmAm(G-2'5')AmCmCfAmAmGmGmAfGmAfAmAmCmImCmsCmsUm | 904/811 |
| AL0185272 | GmsCmsGmUmUmUmCmUmCfCfUfUmGmGmUmCmUmAmAmGmAm | 592/113 | (VPUm)sCfsUmUmAmGf(A-2'5')CmCfAmAmGmGmAfGmAfAmAmCmImCmsCmsUm | 905/811 |
| AL0185273 | GmsCmsGmUmUmUmCmUmCfCfUfUmGmGmUmCmUmAmAmGmAm | 592/113 | (VPUm)sCfsUmUmAmGm(A-2'5')CmCfAmAmGmGmAfGmAfAmAmCmImCmsCmsUm | 906/811 |
| AL0185274 | GmsCmsGmUmUmUmCmUmCfCfUfUmGmGmUmCmUmAmAmGmAm | 592/113 | (VPUm)sCfsUmUmdAGm(A-2'5')CmCfAmAmGmGmAfGmAfAmAmCmImCmsCmsUm | 907/811 |
| AL0185259 | GmsCmsUmGmAmAmCmAmGfCfGfUmUmUmUmUmUmUmUmGmAm | 882/74 | (VPUm)sCfsAmAmAmAfAmAmAfAmUmGmCmUfGmUfUmCmAmGmCmsAmsCm | 895/259 |
| AL0185275 | GmsCmsUmGmAmAmCmAmGfCfGfUmUmUmUmUmUmUmUmGmAm | 882/74 | (VPUm)sCfsAmAmAm(A-2'5')AmAmAfAmUmGmCmUfGmUfUmCmAmGmCmsAmsCm | 908/259 |
| AL0185276 | GmsCmsUmGmAmAmCmAmGfCfGfUmUmUmUmUmUmUmUmGmAm | 882/74 | (VPUm)sCfsAmAmAmAf(A-2'5')AmAfAmUmGmCmUfGmUfUmCmAmGmCmsAmsCm | 909/259 |

(continued)

| Modified siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0185277 | GmsCmsUmGmAmAmCmAmGfCfGfUmUmUmUmUmUmUmUmGmAm | 882/74 | (VPUm)sCfsAmAmAmAm(A-2'5')AmAfAmUmGmCmUfGmUfUmCmAmGmCmsAmsCm | 910/259 |
| AL0185278 | GmsCmsUmGmAmAmCmAmGfCfGfUmUmUmUmUmUmUmUmGmAm | 882/74 | (VPUm)sCfsAmAmdAAm(A-2'5')AmAfAmUmGmCmUfGmUfUmCmAmGmCmsAmsCm | 911/259 |

Table 4. siRNA sequences containing targeting ligands

| Targeting ligand-containing siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0187001 | GmsUmsCmAmUmCmCfAmCfAfAfUm GmAmGmAmGmUmAmCmAm L96 | 425/1 | UmsGfsUmAmCmUfCmUmCmAmUmUm GmUfGmGfAmUmGmAmCmsGmsAm | 599/220 |
| AL0187002 | GmsUmsCmAmUmCmCfAmCfAfAfUm GmAmGmAmGmUmAmCmAm L96 | 425/1 | UmsGfsUmAmCm(Tgn)CmUmCmAmUmU mGmUfGmGfAmUmGmAmCmsGmsAm | 677/220 |
| AL0187003 | CmsAmsGmUmCmUmCfCmCfAfGfCm UmUmUmUmCmUmUmCmUm L96 | 477/107 | AmsGfsAmAmGmAfAmAmAmGmGmUm GmGfGmAfGmAmCmUmGmsGmsGm | 643/281 |
| AL0187004 | GmsGmsCmCmGmUmUfUmCfUfCfCm UmUmGmGmUmCmUmAmAm L96 | 471/101 | UmsUfsAmGmAmCfCmAmAmGmGmAm GmAfAmAfCmGmGmCmCmsGmsCm | 644/311 |
| AL0187005 | GmsCmsCmGmUmUmUfCmUfCfGfUm UmGmGmUmCmUmAmAmAm L96 | 478/108 | UmsUfsUmAmGmAfCmCmAmAmGmGm AmGfAmAfAmCmGmGmCmsUmsGm | 645/286 |
| AL0187006 | GmsCmsGmUmUmUmCfUmCfCfUfUm GmGmUmCmUmAmAmGmUm L96 | 482/112 | AmsCfsUmUmAmGfAmCmCmAmAmGm GmAfGmAfAmAmCmGmCmsCmsUm | 647/318 |
| AL0187007 | GmsCmsGmUmUmUmCfUmCfCfUfUm GmGmUmCmUmAmAmGmAm L96 | 483/113 | UmsCfsUmUmAmGfAmCmCmAmAmGm GmAfGmAfAmAmCmGmCmsCmsUm | 648/319 |
| AL0187008 | CmsCmsGmUmUmUmCfUmCfCfUfUm GmGmUmCmUmAmAmGmAm L96 | 481/111 | (VPUm)sCfsUmUmAmGfAmCmCmAmAm GmGmAfGmAfAmAmCmGmGmsCmsUm | 681/317 |
| AL0187009 | GmsGmsCmCmGmUmUfUmCfUfCfCm UmUmGmGmUmCmUmAmAm L96 | 471/101 | (VPUm)sUfsAmGmAmCfCmAmAmGmGm AmGmAfAmAfCmGmGmCmCmsGmsCm | 678/311 |
| AL0187010 | GmsCmsCmGmUmUmUfCmUfCfGfUm UmGmGmUmCmUmAmAmAm L96 | 478/108 | (VPUm)sUfsUmAmGmAfCmCmAmAmGm GmAmGfAmAfAmCmGmGmCmsUmsGm | 679/286 |
| AL0187011 | GmsCmsGmUmUmUmCfUmCfCfUfUm GmGmUmCmUmAmAmGmAm L96 | 483/113 | (VPUm)sCfsUmUmAmGfAmCmCmAmAm GmGmAfGmAfAmAmCmGmCmsCmsUm | 680/319 |
| AL0187012 | CmsCmsGmUmUmUmCmUmCfCfUfU mGmGmUmCmUmAmAmGmAm A1 | 590/111 | (APU)sCfsUmUmAmGfAmCmCfAmAmG mGmAfGmAfAmAmCmGmGmsCmsUm | 787/317 |
| AL0187013 | GmsCmsCmGmUmUmUmCmUfCfGfU mUmGmGmUmCmUmAmAmAm A1 | 591/108 | (APU)sUfsUmAmGmAfCmCmAfAmGmG mAmGfAmAfAmCmGmGmCmsUmsGm | 788/286 |

(continued)

| Targeting ligand-containing siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0187014 | GmsCmsGmUmUmUmCmUmCfCfUfU mGmGmUmCmUmAmAmGmAm A1 | 592/113 | (APU)sCfsUmUmAmGfAmCmCfAmAmG mGmAfGmAfAmAmCmGmCmsCmsUm | 774/319 |
| AL0187015 | CmsAmsGmUmCmUmCmCmCfAfGfC mUmUmUmUmCmUmUmCmUm A1 | 593/107 | AmsGfsAmAmGmAfAmAmAfGmGmUmG mGfGmAfGmAmCmUmGmsGmsGm | 610/281 |
| AL0187016 | GmsCmsGmUmUmUmCfUmCfCfUfUm GmGmUmCmUmAmAmGmUm A1 | 482/112 | AmsCfsUmUmAmGfAmCmCmAmAmGm GmAfGmAfAmAmCmGmCmsCmsUm | 647/318 |
| AL0187017 | GmsCmsGmUmUmUmCmUmCfCfUfU mGmGmUmCmUmAmAmGmUm A1 | 594/112 | AmsCfsUmUmAmGfAmCmCmAmAmGm GmAfGmAfAmAmCmGmCmsCmsUm | 647/318 |
| AL0187018 | GmsCmsGmUmUmUmCmUmCfCfUfU mGmGmUmCmUmAmAmGmUm A1 | 594/112 | AmsCfsUmUmAmGfAmCfCmAmAmGmG mAfGmAfAmAmCmGmCmsCmsUm | 763/318 |
| AL0187019 | GmsCmsGmUmUmUmCmUmCfCfUfU mGmGmUmCmUmAmAmGmUm A1 | 594/112 | AmsCfsUmUmAmGfAmCmCfAmAmGmG mAfGmAfAmAmCmGmCmsCmsUm | 764/318 |
| AL0187020 | GmsCmsGmUmUmUmCmUmCfCfUfU mGmGmUmCmUmAmAmGmUm L96 | 594/112 | AmsCfsUmUmAmGfAmCmCfAmAmGmG mAfGmAfAmAmCmGmCmsCmsUm | 764/318 |
| AL0187021 | CmsAmsGmUmCmUmCmCmCfAfGfC mUmUmUmUmCmUmUmCmUm L96 | 593/107 | AmsGfsAmAmGmAfAmAmAfGmGmUmG mGfGmAfGmAmCmUmGmsGmsGm | 610/281 |
| AL0187022 | GmsCmsGmUmUmUmCmUmCfCfUfU mGmGmUmCmUmAmAmGmAm L96 | 592/113 | UmsCfsUmUmAmGfAmCmCfAmAmGmG mAfGmAfAmAmCmGmCmsCmsUm | 773/319 |
| AL0187023 | GmsCmsGmUmUmUmCmUmCfCfUfU mGmGmUmCmUmAmAmGmAm L96 | 592/113 | (APU)sCfsUmUmAmGfAmCmCfAmAmG mGmAfGmAfAmAmCmGmCmsCmsUm | 774/319 |
| AL0187024 | GmsCmsGmUmUmUmCmUmCfCfUfU mGmGmUmCmUmAmAmGmAm L96 | 592/113 | (VPUm)sCfsUmUmAmGfAmCmCfAmAm GmGmAfGmAfAmAmCmGmCmsCmsUm | 775/319 |
| AL0187025 | CmsUmsCmCmCmAmCfCfUfUmUmU mCmUmUmCmUmAmAm L96 | 595/129 | (APU)sUfsAmGmAmAfGmAmAfAmAmG mGmUfGmGfGmAmGmsAmsCm | 776/334 |
| AL0187026 | CmsCmsCmAmCmCmUfUfUfUmCmU mUmCmUmAmAmUmAm L96 | 596/133 | (APU)sAfsUmUmAmGfAmAmGfAmAmA mAmGfGmUfGmGmGmsAmsGm | 777/338 |

(continued)

| Targeting ligand-containing siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0187027 | CmsAmsCmCmUmUmUfUfCfUmUmCmUmAmAmUmGmAmAm L96 | 597/135 | (APU)sUfsCmAmUmUfAmGmAfAmGmAmAmAfAmGfGmUmGmsGmsGm | 778/340 |
| AL0187028 | CmsCmsUmUmUmUmCfUfUfCmUmAmAmUmGmAmGmUmAm L96 | 598/137 | (APU)sAfsCmUmCmAfUmUmAfGmAmAmGmAfAmAfAmGmGmsUmsGm | 779/342 |
| AL0187029 | CmsAmsAmGmUmUmGfAfGfAmAmCmAmAmAmAmAmUmUm L96 | 518/148 | AmsAfsUmUmUmUfUmGmUfUmCmUmCmAfAmCfUmUmGmsAmsAm | 780/353 |
| AL0187030 | AmsGmsGmCmAmAmGfAfAfCmCmAmGmUmGmUmUmUmAm L96 | 532/162 | (APU)sAfsAmAmCmAfCmUmGfGmUmUmCmUfUmGfCmCmUmsCmsCm | 781/367 |
| AL0187031 | GmsUmsUmUmUmAmAfAfAfUmUmAmAmAmGmUmAmUmAm L96 | 536/166 | (APU)sAfsUmAmCmUfUmUmAfAmUmUmUmUfAmAfAmAmCmsCmsCm | 782/371 |
| AL0187032 | GmsAmsCmCmAmGmCfUfUfGmUmUmUmGmUmGmAmAmAm L96 | 548/178 | (APU)sUfsUmCmAmCfAmAmAfCmAmAmGmCfUmGfGmUmCmsGmsGm | 783/383 |
| AL0187033 | GmsGmsUmCmUmCmAfCfUfUmUmCmCmAmGmCmAmAmAm L96 | 550/180 | (APU)sUfsUmGmCmUfGmGmAfAmAmGmUmGfAmGfAmCmCmsCmsUm | 784/385 |
| AL0187034 | CmsGmsAmCmCmAmGfCfUfUmGmUmUmUmGmUmGmAmAm L96 | 554/184 | (APU)sUfsCmAmCmAfAmAmCfAmAmGmCmUfGmGfUmCmGmsGmsUm | 785/389 |
| AL0187037 | GmsCmsGmUmUmUmCmUmCfCfUfUmGmGmUmCmUmAmAmGmAm L96 | 592/113 | (VPUm)sCfsUmUmAmGfAmCmCfAmAmGmGmAfImAfAmAmCmGmCmsCmsUm | 854/808 |
| AL0187040 | GmsCmsGmUmUmUmCmUmCfCfUfUmGmGmUmCmUmAmAmGmAm L96 | 592/113 | (VPUm)sCfsUmUmAmGfAmCmCfAmAmGmGmAfGmAfAmAmCmImCmsCmsUm | 857/811 |
| AL0187041 | GmsCmsCmAmCmCmUfUfUfUmCmUmUmCmUmAmAmUmAm L96 | 833/799 | (VPUm)sAfsUmUmAmGfAmAmGfAmAmAmAmGfGmUfGmGmCmsAmsGm | 858/812 |
| AL0187042 | GmsAmsCmCmUmUmUfUfCfUmUmCmUmAmAmUmGmAmAm L96 | 834/800 | (VPUm)sUfsCmAmUmUfAmGmAfAmGmAmAmAfAmGfGmUmCmsGmsGm | 859/813 |
| AL0187043 | CmsAmsAmGmUmUmGfAfGfAmAmCmAmAmAmAmAmUmAm L96 | 835/801 | (VPUm)sAfsUmUmUmUfUmGmUfUmCmUmCmAfAmCfUmUmGmsAmsAm | 860/814 |

(continued)

| Targeting ligand-containing siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0187044 | GmsGmsGmCmAmAmGfAfAfCmCmAmGmUmGmUmUmUmAm L96 | 836/802 | (VPUm)sAfsAmAmCmAfCmUmGfGmUmUmCmUfUmGfCmCmCmsCmsCm | 861/815 |
| AL0187045 | GmsGmsAmCmCmAmGfCfUfUmGmUmUmUmGmUmGmAmAm L96 | 837/803 | (VPUm)sUfsCmAmCmAfAmAmCfAmAmGmCmUfGmGfUmCmCmsGmsUm | 862/816 |
| AL0187046 | CmsAmsCmCmAmGmCfUfUfGmUmUmUmGmUmGmAmAmAm L96 | 838/804 | (VPUm)sUfsUmCmAmCfAmAmAfCmAmAmGmCfUmGfGmUmGmsGmsGm | 863/817 |
| AL0187047 | GmsUmsCmCmCmAmCfCfUfUmUmUmCmUmUmCmUmAmAm L96 | 822/789 | (VPUm)sUfsAmGmAmAfGmAmAfAmAmGmGmUfGmGfGmAmCmsAmsCm | 839/805 |
| AL0187048 | GmsUmsCmCmCmAmCfCfUfCmUmUmCmUmUmCmUmAmAm L96 | 827/794 | (VPUm)sUfsAmGmAmAfGmAmAfAmAmGmGmUfGmGfGmAmCmsAmsCm | 839/805 |
| AL0187049 | GmsUmsCmCmCmAmCfCfUfUmGmUmCmUmUmCmUmAmAm L96 | 831/798 | (VPUm)sUfsAmGmAmAfGmAmAfAmAmGmGmUfGmGfGmAmCmsAmsCm | 839/805 |
| AL0187050 | GmsCmsGmUmUmUmCmUmCfCfUfUmGmGmUmCmUmAmAmGmAm L96 | 592/113 | (VPUm)sCfsUmUmAmGfAmCmCfAmImGmGmAfGmAfAmAmCmGmCmsCmsUm | 864/818 |
| AL0187051 | GmsCmsUmGmAmAmCmAmGfCfGfUmUmUmUmUmUmUmUmGmAm L96 | 882/74 | (VPUm)sCfsAmAmAmAfAmAmAfAmUmGmCmUfGmUfUmCmAmGmCmsAmsCm | 894/259 |
| AL0187052 | GmsCmsUmGmAmAmCmAmGfCfGfUmUmUmUmUmUmUmUmGmAm_L96 | 882/74 | (VPUm)sdCsAmAmdAAmdAAmAmAmUmdGCmUfGmUmUmCmAmGmCmsAmsCm | 902/259 |
| AL0187053 | GmsAmsGmAmGmAmGmAmGfCfCfCmAmCmAmGmAmGmUmCmAm L96 | 884/870 | (VPUm)sGfsAmCmUmCfUmGmUfGmGmGmCmUfCmUfCmUmCmUmCmsAmsUm | 896/874 |
| AL0187054 | GmsAmsGmAmGmAmGmAmGfCfCfCmAmCmAmGmAmGmUmCmAm L96 | 884/870 | (VPUm)sdGsAmCmdTCmdTGmUmGmGmdGCmUfCmUmCmUmCmUmCmsAmsUm | 903/874 |
| AL0187055 | GmsCmsGmUmUmUmCmUmCfCfUfUmGmGmUmCmUmAmAmGmAm_L96 | 592/113 | (VPUm)sCfsUmUmAmGf(A-2'5')CmCfAmAmGmGmAfGmAfAmAmCmImCmsCmsUm | 905/811 |

(continued)

| Targeting ligand-containing siRNA No. | Modified sequence of sense strand | Modified sequence SEQ ID NO/corresponding naked sequence SEQ ID NO | Modified sequence of antisense strand | Modified sequence SEQ ID NO/corresponding naked sequence SEQ ID NO |
|---|---|---|---|---|
| AL0187056 | GmsCmsGmUmUmUmCmUmCfCfUfUmGmGmUmCmUmAmAmGmAm_L96 | 592/113 | (VPUm)sCfsUmUmdAGm(A-2'5')CmCfAmAmGmGmAfGmAfAmAmCmImCmsCmsUm | 907/811 |
| AL0187057 | GmsCmsUmGmAmAmCmAmGfCfGfUmUmUmUmUmUmUmUmGmAm_L96 | 882/74 | (VPUm)sCfsAmAmAmAm(A-2'5')AmAfAmUmGmCmUfGmUfUmCmAmGmCmsAmsCm | 910/259 |
| AL0187058 | GmsCmsUmGmAmAmCmAmGfCfGfUmUmUmUmUmUmUmUmGmAm_L96 | 882/74 | (VPUm)sCfsAmAmdAAm(A-2'5')AmAfAmUmGmCmUfGmUfUmCmAmGmCmsAmsCm | 911/259 |

(1) Hep3B cell culture and transfection:

The human liver cancer cell line Hep3B (Shanghai Fushen Biotechnology Co., Ltd.) was used. Cells of the cell line were placed in an incubator at 37 °C with 5% $CO_2$ and cultured using a DMEM culture medium (Hyclone, SH30022.01, 2 g/L glucose) supplemented with 10% FBS (aqlabteech, AQ-MV-06600) and 1% penicillin-streptomycin (Keygen Biotech, KGY0023). When the cell confluency reached 90%, the cells were digested with trypsin (Amresco, 0458-250G) and counted using a counter (Nexcelom, cellometer Mini). The cell suspension was inoculated into a 96-well plate at 150 μL/well (cell count: $2 \times 10^4$ cells/well). The next day, adherent cells were transfected.

Transfection was performed using Lipofectamine™ RNAiMAX (thermofisher, 13778150). 5 μL (50 nM) of a compound dilution was dispersed in 20 μL of Opti-MEM (thermofisher, 1105821), and 0.2 μL of RNAiMAX was dispersed in 25 μL of Opti-MEM. The final concentration of siRNA was 5 nM. After 5 min of incubation, the RNAiMAX dispersion was mixed with the compound dispersion, and the mixture was incubated for 10 min. Cells were added to the transfection complex (n = 2) and cultured in an incubator at 37 °C with 5% $CO_2$ for 24 h.

(2) Cell lysis

After one day of transfection, the culture medium was removed, and PBS was added to wash the cells. 50 μL of lysis buffer was added to each well, and the plate was stored at -80 °C.

(3) RT-qPCR

Mixtures were prepared in RNase-free centrifuge tubes: 2.5 μL of buffer, 0.2 μL of Enzyme Mix (Foregene, DRT-02011), 0.4 μL of lysate, and 0.4 μL of 5 μM primer were mixed, and RNase-free dd$H_2O$ was added until the volume was 5 μL. Each sample was tested in triplicate. The 96-well plate was placed into a qPCR instrument (ROCGENE, Archimed), and the following program was executed: stage 1, 42 °C, 5 min; 95 °C, 10 sec; amplification, 95 °C, 5 sec; 59 °C, 20 sec; 72 °C, 10 sec; 40 cycles; dissolution curve, 95 °C, 15 sec, 59 °C, 60 sec, 95 °C, 15 sec.

(4) Statistical analysis of data:

Data were exported in EXCEL format, and $CT_{AGT}$-$CT_{hTBP}$ was used to normalize the control group. To calculate fold changes in relative silencing efficiency, data were analyzed using the ΔΔCT method. The results are shown in Tables 5 and 6. The data in each table were obtained from an individual test. Due to variations in cell batches, the target gene silencing efficiency may vary.

The inhibitory effects of the naked sequence groups AL0181001-AL0061069 and AL0181070-AL0181223 at a dose of 0.05 nM on AGT gene expression levels in the Hep3B cell strain are shown in Tables 5 and 6. Their inhibition rates were between 10% and 90%. The AGT mRNA knockdown rates of the AL0181001, AL0181002, AL0181003, AL0181004, AL0181005, AL0181006, AL0181012, AL0181014, AL0181019, AL0181020, AL0181021, AL0181033, AL0181038, AL0181040, AL0181042, AL0181043, AL0181045, AL0181046, AL0181050, AL0181051, AL0181053, AL0181056, AL0181059, AL0181060, AL0181061, AL0181062, AL0181063, AL0181065, AL0181066, AL0181067, AL0181069, AL0181153, AL0181074, AL0181154, AL0181156, AL0181077, AL0181078, AL0181160, AL0181084, AL0181167, AL0181089, AL0181169, AL0181170, AL0181171, AL0181090, AL0181091, AL0181092, AL0181097, AL0181098, AL0181099, AL0181178, AL0181102, AL0181103, AL0181104, AL0181105, AL0181181, AL0181183, AL0181185, AL0181108, AL0181110, AL0181111, AL0181112, AL0181187, AL0181189, AL0181113, AL0181115, AL0181195, AL0181128, AL0181135, AL0181136, AL0181215, AL0181217, AL0181218, AL0181147, and AL0181222 groups were greater than 75%, and were even greater than 90%.

Table 2. AGT siRNA knockdown levels of naked sequences

| Sequence No. | Dose of 0.05 nM AGT mRNA residual activity (%) | | Sequence No. | Dose of 0.05 nM AGT mRNA residual activity (%) | |
|---|---|---|---|---|---|
| | Mean | SD | | Mean | SD |
| AL0181001 | 4 | 0 | AL0181036 | 84 | 1 |
| AL0181002 | 21 | 2 | AL0181037 | 83 | 2 |
| AL0181003 | 12 | 1 | AL0181038 | 13 | 1 |
| AL0181004 | 6 | 1 | AL0181039 | 26 | 2 |
| AL0181005 | 21 | 2 | AL0181040 | 9 | 0 |

(continued)

| Sequence No. | Dose of 0.05 nM AGT mRNA residual activity (%) | | Sequence No. | Dose of 0.05 nM AGT mRNA residual activity (%) | |
|---|---|---|---|---|---|
| | Mean | SD | | Mean | SD |
| AL0181006 | 8 | 0 | AL0181041 | 27 | 0 |
| AL0181007 | 27 | 4 | AL0181042 | 15 | 0 |
| AL0181008 | 52 | 2 | AL0181043 | 10 | 0 |
| AL0181009 | 53 | 0 | AL0181044 | 49 | 2 |
| AL0181010 | 30 | 1 | AL0181045 | 19 | 1 |
| AL0181011 | 30 | 1 | AL0181046 | 8 | 0 |
| AL0181012 | 18 | 0 | AL0181047 | 26 | 1 |
| AL0181013 | 34 | 4 | AL0181048 | 35 | 2 |
| AL0181014 | 8 | 3 | AL0181049 | 48 | 6 |
| AL0181015 | 31 | 2 | AL0181050 | 21 | 7 |
| AL0181016 | 30 | 3 | AL0181051 | 12 | 6 |
| AL0181017 | 91 | 8 | AL0181052 | 39 | 10 |
| AL0181018 | 74 | 3 | AL0181053 | 23 | 6 |
| AL0181019 | 11 | 1 | AL0181054 | 40 | 3 |
| AL0181020 | 12 | 0 | AL0181055 | 46 | 1 |
| AL0181021 | 10 | 0 | AL0181056 | 14 | 1 |
| AL0181022 | 32 | 1 | AL0181057 | 26 | 3 |
| AL0181023 | 42 | 1 | AL0181058 | 51 | 3 |
| AL0181024 | 65 | 4 | AL0181059 | 14 | 1 |
| AL0181025 | 59 | 2 | AL0181060 | 17 | 2 |
| AL0181026 | 56 | 2 | AL0181061 | 10 | 1 |
| AL0181027 | 26 | 3 | AL0181062 | 10 | 1 |
| AL0181028 | 44 | 4 | AL0181063 | 14 | 0 |
| AL0181029 | 78 | 9 | AL0181064 | 42 | 1 |
| AL0181030 | 28 | 3 | AL0181065 | 5 | 0 |
| AL0181031 | 35 | 2 | AL0181066 | 6 | 0 |
| AL0181032 | 50 | 3 | AL0181067 | 6 | 0 |
| AL0181033 | 12 | 1 | AL0181068 | 36 | 2 |
| AL0181034 | 41 | 4 | AL0181069 | 17 | 1 |
| AL0181035 | 58 | 6 | | | |

Table 6. AGT siRNA knockdown levels of naked sequences

| Sequence No. | Dose of 0.05 nM AGT mRNA residual activity (%) | | Sequence name | Dose of 0.05 nM mRNA residual activity (%) | |
|---|---|---|---|---|---|
| | Mean | SD | | Mean | SD |
| AL0181070 | 94 | 11 | AL0181150 | 73 | 11 |
| AL0181071 | 87 | 7 | AL0181151 | 69 | 13 |
| AL0181072 | 94 | 12 | AL0181152 | 80 | 13 |

(continued)

| Sequence No. | Dose of 0.05 nM AGT mRNA residual activity (%) | | Sequence name | Dose of 0.05 nM mRNA residual activity (%) | |
|---|---|---|---|---|---|
| | Mean | SD | | Mean | SD |
| AL0181073 | 41 | 9 | AL0181153 | 18 | 5 |
| AL0181074 | 23 | 4 | AL0181154 | 16 | 3 |
| AL0181075 | 26 | 2 | AL0181155 | 41 | 12 |
| AL0181076 | 32 | 12 | AL0181156 | 24 | 6 |
| AL0181077 | 21 | 2 | AL0181157 | 58 | 6 |
| AL0181078 | 21 | 1 | AL0181158 | 59 | 4 |
| AL0181079 | 40 | 2 | AL0181159 | 33 | 2 |
| AL0181080 | 40 | 7 | AL0181160 | 15 | 2 |
| AL0181081 | 37 | 6 | AL0181161 | 45 | 7 |
| AL0181082 | 72 | 14 | AL0181162 | 68 | 8 |
| AL0181083 | 37 | 4 | AL0181163 | 94 | 12 |
| AL0181084 | 19 | 10 | AL0181164 | 87 | 26 |
| AL0181085 | 51 | 2 | AL0181165 | 34 | 2 |
| AL0181086 | 61 | 5 | AL0181166 | 95 | 12 |
| AL0181087 | 66 | 5 | AL0181167 | 14 | 1 |
| AL0181088 | 63 | 16 | AL0181168 | 30 | 3 |
| AL0181089 | 19 | 4 | AL0181169 | 23 | 3 |
| AL0181090 | 19 | 4 | AL0181170 | 22 | 3 |
| AL0181091 | 8 | 2 | AL0181171 | 20 | 0 |
| AL0181092 | 13 | 3 | AL0181172 | 69 | 13 |
| AL0181094 | 40 | 11 | AL0181173 | 93 | 14 |
| AL0181095 | 50 | 34 | AL0181174 | 93 | 7 |
| AL0181096 | 55 | 10 | AL0181175 | 67 | 4 |
| AL0181097 | 18 | 3 | AL0181176 | 40 | 8 |
| AL0181098 | 21 | 5 | AL0181177 | 42 | 9 |
| AL0181099 | 14 | 3 | AL0181178 | 16 | 1 |
| AL0181100 | 26 | 9 | AL0181179 | 48 | 4 |
| AL0181101 | 37 | 20 | AL0181180 | 56 | 6 |
| AL0181102 | 15 | 4 | AL0181181 | 24 | 1 |
| AL0181103 | 13 | 5 | AL0181182 | 39 | 4 |
| AL0181104 | 17 | 2 | AL0181183 | 14 | 3 |
| AL0181105 | 17 | 4 | AL0181184 | 28 | 10 |
| AL0181106 | 46 | 10 | AL0181185 | 15 | 5 |
| AL0181107 | 67 | 36 | AL0181186 | 65 | 10 |
| AL0181108 | 21 | 5 | AL0181187 | 8 | 2 |
| AL0181110 | 24 | 6 | AL0181188 | 72 | 13 |
| AL0181111 | 23 | 9 | AL0181189 | 19 | 2 |
| AL0181112 | 22 | 4 | AL0181190 | 35 | 4 |

(continued)

| Sequence No. | Dose of 0.05 nM AGT mRNA residual activity (%) | | Sequence name | Dose of 0.05 nM mRNA residual activity (%) | |
|---|---|---|---|---|---|
| | Mean | SD | | Mean | SD |
| AL0181113 | 23 | 8 | AL0181191 | 27 | 3 |
| AL0181114 | 48 | 2 | AL0181192 | 60 | 6 |
| AL0181115 | 22 | 4 | AL0181193 | 67 | 6 |
| AL0181116 | 40 | 11 | AL0181194 | 104 | 17 |
| AL0181118 | 26 | 4 | AL0181195 | 22 | 5 |
| AL0181119 | 75 | 14 | AL0181196 | 29 | 6 |
| AL0181120 | 124 | 25 | AL0181197 | 119 | 28 |
| AL0181121 | 40 | 3 | AL0181198 | 89 | 17 |
| AL0181122 | 61 | 9 | AL0181199 | 29 | 4 |
| AL0181123 | 62 | 7 | AL0181200 | 32 | 8 |
| AL0181124 | 113 | 12 | AL0181201 | 112 | 12 |
| AL0181125 | 33 | 12 | AL0181202 | 48 | 6 |
| AL0181126 | 45 | 6 | AL0181203 | 40 | 3 |
| AL0181127 | 77 | 9 | AL0181204 | 53 | 5 |
| AL0181128 | 23 | 6 | AL0181205 | 56 | 15 |
| AL0181129 | 36 | 5 | AL0181206 | 51 | 9 |
| AL0181130 | 47 | 4 | AL0181207 | 68 | 17 |
| AL0181133 | 43 | 2 | AL0181208 | 65 | 7 |
| AL0181134 | 33 | 12 | AL0181209 | 71 | 4 |
| AL0181135 | 22 | 3 | AL0181210 | 64 | 9 |
| AL0181136 | 23 | 2 | AL0181211 | 61 | 8 |
| AL0181137 | 35 | 8 | AL0181212 | 43 | 9 |
| AL0181138 | 40 | 6 | AL0181213 | 66 | 20 |
| AL0181139 | 31 | 4 | AL0181214 | 46 | 8 |
| AL0181140 | 36 | 5 | AL0181215 | 24 | 5 |
| AL0181141 | 50 | 7 | AL0181216 | 54 | 5 |
| AL0181142 | 26 | 4 | AL0181217 | 18 | 5 |
| AL0181143 | 34 | 9 | AL0181218 | 20 | 3 |
| AL0181144 | 132 | 14 | AL0181219 | 46 | 3 |
| AL0181145 | 104 | 23 | AL0181220 | 26 | 7 |
| AL0181146 | 63 | 6 | AL0181221 | 67 | 12 |
| AL0181147 | 20 | 2 | AL0181222 | 21 | 2 |
| AL0181148 | 120 | 17 | AL0181223 | 35 | 2 |
| AL0181149 | 68 | 12 | AL0181001 | 28 | 2 |

After the sequences were ranked in descending order of their mRNA inhibition rates, the top 15 sequences were selected, and walking siRNA designs were generated based on sequence positions. All sequences were chemically modified. The knockdown of AGT mRNA by the modified sequence groups AL0185001-AL0185180 at doses of 0.5 nM and 0.05 nM is shown in Table 7.

Table 7. AGT siRNA knockdown levels of modified sequences

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185001 | 15 | 4 | 55 | 6 |
| AL0185002 | 49 | 13 | 74 | 4 |
| AL0185003 | 51 | 3 | 84 | 15 |
| AL0185004 | 50 | 7 | 95 | 12 |
| AL0185005 | 54 | 25 | 69 | 29 |
| AL0185006 | 66 | 9 | 88 | 16 |
| AL0185007 | 57 | 1 | 76 | 1 |
| AL0185008 | 57 | 12 | 78 | 8 |
| AL0185009 | 102 | 4 | 97 | 16 |
| AL0185010 | 52 | 14 | 78 | 17 |
| AL0185011 | 49 | 11 | 99 | 7 |
| AL0185012 | 29 | 8 | 65 | 1 |
| AL0185013 | 53 | 18 | 72 | 8 |
| AL0185014 | 29 | 1 | 62 | 2 |
| AL0185015 | 26 | 5 | 48 | 0 |
| AL0185016 | 68 | 13 | 121 | 24 |
| AL0185017 | 41 | 1 | 80 | 10 |
| AL0185018 | 38 | 3 | 57 | 1 |
| AL0185019 | 52 | 1 | 97 | 13 |
| AL0185020 | 38 | 3 | 53 | 32 |
| AL0185021 | 65 | 13 | 85 | 11 |
| AL0185022 | 18 | 1 | 54 | 10 |
| AL0185023 | 29 | 4 | 70 | 7 |
| AL0185024 | 33 | 3 | 24 | 3 |
| AL0185025 | 64 | 4 | 41 | 3 |
| AL0185026 | 41 | 4 | 80 | 7 |
| AL0185027 | 15 | 1 | 44 | 4 |
| AL0185028 | 38 | 2 | 42 | 3 |
| AL0185029 | 46 | 4 | 58 | 5 |
| AL0185030 | 40 | 2 | 51 | 4 |
| AL0185031 | 59 | 5 | 70 | 6 |
| AL0185032 | 108 | 8 | 70 | 5 |
| AL0185033 | 52 | 4 | 72 | 5 |
| AL0185034 | 26 | 2 | 45 | 4 |
| AL0185035 | 20 | 1 | 73 | 5 |
| AL0185036 | 91 | 8 | 108 | 10 |
| AL0185037 | 33 | 2 | 61 | 4 |
| AL0185038 | 2 | 0 | 19 | 1 |

(continued)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185039 | 9 | 4 | 47 | 8 |
| AL0185040 | 15 | 13 | 49 | 11 |
| AL0185041 | 3 | 1 | 35 | 1 |
| AL0185042 | 49 | 12 | 72 | 6 |
| AL0185043 | 19 | 6 | 51 | 4 |
| AL0185044 | 13 | 8 | 33 | 8 |
| AL0185045 | 62 | 12 | 63 | 4 |
| AL0185046 | 9 | 5 | 40 | 16 |
| AL0185047 | 13 | 1 | 49 | 11 |
| AL0185048 | 5 | 1 | 28 | 19 |
| AL0185049 | 13 | 11 | 42 | 23 |
| AL0185050 | 72 | 3 | 70 | 4 |
| AL0185051 | 32 | 14 | 72 | 19 |
| AL0185052 | 48 | 0 | 75 | 10 |
| AL0185053 | 35 | 5 | 44 | 3 |
| AL0185054 | 4 | 2 | 13 | 8 |
| AL0185055 | 15 | 5 | 70 | 11 |
| AL0185056 | 10 | 2 | 45 | 16 |
| AL0185057 | 11 | 4 | 69 | 12 |
| AL0185058 | 21 | 7 | 74 | 12 |
| AL0185059 | 19 | 4 | 63 | 8 |
| AL0185060 | 28 | 2 | 76 | 15 |
| AL0185061 | 17 | 6 | 64 | 9 |
| AL0185062 | 13 | 4 | 56 | 2 |
| AL0185063 | 11 | 3 | 48 | 6 |
| AL0185064 | 11 | 3 | 19 | 5 |
| AL0185065 | 7 | 4 | 22 | 4 |
| AL0185066 | 30 | 11 | 72 | 11 |
| AL0185067 | 48 | 9 | 81 | 3 |
| AL0185068 | 43 | 17 | 68 | 15 |
| AL0185069 | 49 | 13 | 71 | 15 |
| AL0185070 | 98 | 19 | 90 | 5 |
| AL0185071 | 133 | 5 | 103 | 24 |
| AL0185072 | 84 | 10 | 99 | 29 |
| AL0185073 | 65 | 22 | 74 | 21 |
| AL0185074 | 66 | 25 | 70 | 13 |
| AL0185075 | 22 | 2 | 44 | 15 |
| AL0185076 | 65 | 6 | 75 | 9 |

(continued)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185077 | 63 | 12 | 75 | 22 |
| AL0185078 | 30 | 21 | 67 | 4 |
| AL0185081 | 10 | 7 | 48 | 10 |
| AL0185082 | 33 | 15 | 79 | 20 |
| AL0185083 | 23 | 12 | 68 | 10 |
| AL0185084 | 53 | 25 | 83 | 19 |
| AL0185085 | 6 | 1 | 30 | 7 |
| AL0185086 | 6 | 4 | 15 | 4 |
| AL0185087 | 9 | 3 | 30 | 8 |
| AL0185088 | 60 | 7 | 119 | 15 |
| AL0185089 | 6 | 4 | 24 | 2 |
| AL0185090 | 27 | 6 | 77 | 8 |
| AL0185100 | 70 | 9 | 97 | 3 |
| AL0185101 | 65 | 6 | 82 | 9 |
| AL0185102 | 48 | 20 | 103 | 10 |
| AL0185103 | 33 | 5 | 88 | 2 |
| AL0185104 | 5 | 1 | 29 | 5 |
| AL0185105 | 19 | 5 | 86 | 1 |
| AL0185106 | 79 | 6 | 109 | 3 |
| AL0185107 | 75 | 5 | 83 | 7 |
| AL0185108 | 62 | 5 | 77 | 2 |
| AL0185109 | 4 | 2 | 55 | 24 |
| AL0185110 | 6 | 4 | 55 | 20 |
| AL0185111 | 13 | 3 | 60 | 11 |
| AL0185112 | 15 | 6 | 66 | 21 |
| AL0185113 | 21 | 10 | 57 | 7 |
| AL0185114 | 66 | 8 | 70 | 10 |
| AL0185115 | 81 | 19 | 106 | 19 |
| AL0185116 | 47 | 17 | 89 | 11 |
| AL0185117 | 70 | 11 | 73 | 13 |
| AL0185118 | 96 | 6 | 104 | 4 |
| AL0185119 | 63 | 17 | 89 | 7 |
| AL0185120 | 58 | 8 | 80 | 12 |
| AL0185121 | 50 | 16 | 100 | 36 |
| AL0185122 | 66 | 25 | 98 | 4 |
| AL0185123 | 6 | 1 | 39 | 3 |
| AL0185124 | 28 | 8 | 111 | 25 |
| AL0185125 | 12 | 5 | 55 | 20 |

(continued)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185126 | 11 | 5 | 59 | 28 |
| AL0185127 | 5 | 2 | 69 | 16 |
| AL0185128 | 69 | 10 | 88 | 25 |
| AL0185129 | 54 | 3 | 56 | 6 |
| AL0185130 | 77 | 20 | 76 | 6 |
| AL0185131 | 38 | 10 | 79 | 8 |
| AL0185132 | 62 | 21 | 96 | 13 |
| AL0185133 | 37 | 16 | 73 | 2 |
| AL0185134 | 35 | 15 | 91 | 7 |
| AL0185135 | 82 | 16 | 117 | 19 |
| AL0185136 | 88 | 13 | 99 | 16 |
| AL0185137 | 27 | 14 | 68 | 20 |
| AL0185138 | 37 | 10 | 101 | 4 |
| AL0185139 | 11 | 3 | 67 | 10 |
| AL0185140 | 63 | 15 | 100 | 20 |
| AL0185141 | 9 | 2 | 93 | 11 |
| AL0185142 | 80 | 12 | 117 | 28 |
| AL0185143 | 112 | 22 | 103 | 14 |
| AL0185144 | 110 | 21 | 130 | 9 |
| AL0185145 | 4 | 2 | 40 | 13 |
| AL0185146 | 124 | 24 | 142 | 11 |
| AL0185147 | 19 | 6 | 71 | 26 |
| AL0185148 | 80 | 19 | 100 | 21 |
| AL0185149 | 52 | 13 | 72 | 25 |
| AL0185150 | 107 | 21 | 82 | 9 |
| AL0185151 | 75 | 22 | 98 | 20 |
| AL0185152 | 25 | 10 | 90 | 15 |
| AL0185153 | 65 | 11 | 92 | 28 |
| AL0185154 | 94 | 14 | 122 | 10 |
| AL0185155 | 72 | 17 | 111 | 51 |
| AL0185156 | 27 | 9 | 89 | 19 |
| AL0185157 | 95 | 43 | 103 | 39 |
| AL0185158 | 68 | 28 | 99 | 29 |
| AL0185159 | 72 | 13 | 88 | 5 |
| AL0185160 | 88 | 15 | 94 | 14 |
| AL0185161 | 69 | 11 | 78 | 7 |
| AL0185162 | 64 | 6 | 82 | 5 |
| AL0185163 | 94 | 22 | 107 | 34 |

(continued)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185164 | 82 | 17 | 95 | 20 |
| AL0185165 | 71 | 14 | 99 | 16 |
| AL0185166 | 90 | 32 | 104 | 23 |
| AL0185167 | 70 | 9 | 90 | 15 |
| AL0185168 | 37 | 0 | 79 | 9 |
| AL0185169 | 59 | 9 | 88 | 5 |
| AL0185170 | 25 | 15 | 63 | 21 |
| AL0185171 | 40 | 7 | 92 | 12 |
| AL0185172 | 84 | 3 | 108 | 35 |
| AL0185173 | 45 | 21 | 87 | 16 |
| AL0185174 | 66 | 19 | 74 | 9 |
| AL0185175 | 100 | 13 | 86 | 21 |
| AL0185176 | 53 | 5 | 84 | 18 |
| AL0185177 | 91 | 20 | 100 | 16 |
| AL0185178 | 20 | 4 | 80 | 27 |
| AL0185179 | 97 | 22 | 114 | 24 |
| AL0185180 | 15 | 10 | 60 | 13 |

**Example 2. *In Vitro* Activity Screening of Chemically Modified AGT-siRNAs in Primary Monkey Hepatocytes**

**(1) Cell culture and transfection of cynomolgus monkey hepatocytes:**

Cynomolgus monkey hepatocytes (Beijing ReaderBio Biotechnology Co., Ltd., cmTCSC) were used. The culture medium was preheated first. A thawing culture medium (Beijing ReaderBio Biotechnology Co., Ltd., HEPO24) was transferred to a biosafety cabinet, and 4 mL of FBS was added to 36 mL of the thawing culture medium (TPCS, HEPO24) to prepare a complete thawing culture medium. Then, the culture medium was heated in a water bath at 37 °C for 10 min. Treatment was performed with a coating medium (Beijing ReaderBio Biotechnology Co., Ltd., HEPO44) in a $CO_2$ incubator at 37 °C for 0.5 h. Cells were removed from liquid nitrogen and thawed in a water bath at 37 °C. After about 2 min, the cell suspension was transferred to 40 mL of the preheated thawing culture medium. A cell cryovial was washed with 2 mL of the complete thawing culture medium. The cell suspension was centrifuged at 180× g for 1 min, and the supernatant was discarded. 2 mL of a preheated CM seeding culture medium (Beijing ReaderBio Biotechnology Co., Ltd., CMHEP054) was added, and the cell suspension was mixed well by gentle pipetting. Then, 20 μL of the cell suspension was taken for counting. On the basis of the counting results, cells were seeded into a 12-well plate at 3 × 10^5/well and cultured in an incubator at 37 °C with 5% $CO_2$. After 4-5 h of adherent culture, the CM seeding culture medium was removed using a pipette and replaced with a preheated culture medium (Beijing ReaderBio Biotechnology Co., Ltd., CMHEP064). After 6 h of adherent culture, the cells were transfected. Transfection was performed using Lipofectamine™ 3000 Transfection Reagent (thermofisher, L3000150). System (1): 50 nM modified siRNA (Suzhou Biosyntech Co., Ltd.) was diluted with 50 μL of Opti-MEM (thermofisher, 1105821). System (2): 3 μL of Lip3000 was diluted with 50 μL of Opti-MEM. After standing for 5 min, systems (1) and (2) were mixed. After standing for another 15 min, the mixture was added dropwise to the 12-well plate. After 4 h of transfection, the culture medium was replaced with a DMEM/F12 complete culture medium, and the 12-well plate was incubated in an incubator for 48 h.

**(2) Total RNA extraction using RNA-Quick Purification Kit (Yishan Biotechnology, RN001):**

The 12-well plate was removed from the incubator. The culture medium was removed using a pipette, and the plate was washed once with an appropriate amount of PBS. 500 μL of lysis buffer was added to each well, and the

supernatants were transferred to new 1.5 mL centrifuge tubes. 500 μL of absolute ethanol was added to the lysed cells and mixed well (in case of precipitation, which is normal, continue the operation). The centrifuge tubes were inverted several times, or the mixtures were vigorously pipetted 10 times to disperse the generated precipitate. Then, the liquids were added to a centrifuge column, the centrifuge tubes were symmetrically placed into a centrifuge (eppendorf, 5430), and centrifugation was performed at 4000× g for 1 min. The centrifuge tubes were removed, 500 μL of wash buffer was added to the column, and centrifugation was performed at 12,000× g for 1 min. When removing the column after centrifugation, be careful not to let the waste liquid in the collection tube come into contact with the RNA column to avoid contamination. The waste liquid was poured, the RNA column was placed back into the collection tube, and centrifugation was performed once with the tube being empty to completely remove possible wash buffer residues. The column was placed above a clean RNase-free 1.5 mL centrifuge tube, uncapped, and dried for 2 min. 30 μL of elution buffer was added to the central part of the membrane of the RNA column, and the column was left to stand at room temperature for 2 min and centrifuged at 2000× g for 1 min. After elution, the RNA eluate was placed on ice. The eluted RNA concentration was determined to facilitate use in subsequent experiments. The extracted RNA can be used immediately for subsequent experiments or stored at -80 °C for later use.

**(3) cDNA synthesis using HiScript® II Q RT SuperMix for qPCR (+gDNA wiper) reverse transcription kit (Vazyme, R223-01):**

A mixture was prepared in an RNase-free centrifuge tube: 4 μL of 4× gDNA wiper Mix and 1 μg of template RNA were added, and RNase-free $ddH_2O$ was added until the volume was 16 μL, to remove genomic DNA, and the contents in the tube were mixed well by gentle pipetting at 42 °C for 2 min. Then, 4 μL of 5× HiScript II qRT SuperMix II was directly added to the reaction tube, and the contents in the tube were mixed well by gentle pipetting and incubated in a PCR instrument (Applied Biosystems, 9700) at 50 °C for 15 min, at 85 °C for 5 sec, and at 4 °C. The product can be immediately used for qPCR reaction or stored at -20 °C, and should be used within half a year. For long-term storage, it should be aliquoted and then stored at - 80 °C. Repeated freeze-thaw cycles should be avoided for cDNA.

**(4) qPCR quantitation using ChamQ SYBR qPCR Master Mix (Vazyme, Q311-02):**

A mixture was prepared: 10 μL of 2× ChamQ SYBR qPCR Master Mix, 0.5 μL of Forword primer (Ruibiotech), 0.5 μL of Reverse primer (Ruibiotech), 1 μL of Template cDNA, and 8 μL of $ddH_2O$ were mixed to form a 20 μL system. Each sample was tested in triplicate. The 96-well plate was placed into a qPCR instrument (ROCGENE, Archimed), and the following program was executed: pre-denaturation, 95 °C, 30 sec; amplification, 95 °C, 10 sec, 60 °C, 30 sec, 40 cycles; dissolution curve, 95 °C, 15 sec, 60 °C, 60 sec, 95 °C, 15 sec.

**(5) Statistical analysis of data:**

Data were exported in EXCEL format, and $CT_{AGT}$-$CT_{GAPDH}$ was used to normalize the control group. To calculate fold changes in relative silencing efficiency, data were analyzed using the ΔΔCT method. The mean and standard deviation of the obtained three parallel replicate data were calculated. The results of 7 primary monkey cell screening tests are shown in Tables 8-14. The data in each table were obtained from an individual test. Due to variations in cell batches, the target gene silencing efficiency may vary. In certain tests, low concentrations may occur due to the cell state. For example, in Table 14, at a dose of 0.01 nM, the deviations between the replicate wells of biological replicates were relatively large, in which case the data with relatively small errors at other concentrations and the same concentration should be referred to.

It can be seen from Table 8 that with the AL0185001 group as the quality control standard, at a dose of 0.5 nM, the inhibitory effects of the siRNAs of the AL0185055-AL0185057, AL0185061-64, and AL0185091-AL0185094 groups were all higher than 90%; at a low-concentration dose of 0.05 nM, the inhibition rates of siRNAs other than the AL0185062 and AL01850663 groups were all greater than 80%.

It can be seen from Table 9 that with the AL0185001 group as the quality control standard, at a dose of 0.5 nM, the inhibitory effects of siRNAs other than the AL0185081 and AL0185188 groups were all better than that of the AL0185001 group; at a low-concentration dose of 0.05 nM, the inhibition rates of siRNAs other than the AL0185089 group were all better than that of the AL0185001 group. The AL0185188-AL0185195 groups were obtained by introducing thermolabile base modifications (VPU and APU) into the AL0185081, AL0185085, AL0185087, and AL0185089 groups. The results show that the siRNAs exhibited further improved efficacy after undergoing a VPU or APU modification.

It can be seen from Table 10 that with the AL0185001 group as the quality control standard, at a dose of 0.5 nM, the inhibitory effects of siRNAs other than the AL0185104 group were all around 70%; at a low-concentration dose of 0.05 nM, the inhibition rates of these siRNAs decreased.

It can be seen from Table 11 that with the AL0185001 group as the quality control standard, at a dose of 0.5 nM, the inhibitory effects of the siRNAs of the AL0185210, AL0185215, AL0185219, AL0185220, AL0185221, AL0185222,

AL0185228, AL0185229, and AL0185230 groups were all higher than 98%; at a low-concentration dose of 0.05 nM, their inhibitory effects were all higher than 94%. The siRNAs described above all underwent thermolabile base modifications (VPU and APU). Among them, the AL0185222 group also had a (Tgn) modification, the AL0185228 group also had a (C-2'5') modification, the AL0185229 group also had an (A-2'5') modification, and the AL0185230 group also had a (U-2'5') modification. The results show that the siRNA exhibited further improved efficacy after undergoing a VPU modification. The GNA modifications and 2'5' modifications can reduce the miRNA-like off-target activity of the seed region (positions 2-8 of the siRNA antisense strand), and the inhibitory effect on the AGT mRNA could still reach an inhibitory efficacy of greater than 98%. The results show that the efficacy could be further improved after a VPU modification, an APU modification, a GNA modification, or a 2'5' modification.

It can be seen from Table 12 that with the AL0185001 group as the quality control standard, at a dose of 0.01 nM or 0.05 nM, introducing Im modifications to certain positions could further improve the efficacy. Examples include the series AL0185234, AL0185237, and AL0185244 groups.

It can be seen from Table 13 that the efficacy could be further improved after a VPUm was added or a base was replaced with a DNA modification. For example, compared to the AL0185258 group, the efficacy could be increased by a factor of 2-3 after addition of a VPUm or replacement with a DNA modification, especially at a relatively low-concentration dose. For example, the target protein mRNA silencing efficiency of the AL0185255, AL0185259, AL0185257, AL0185268, AL0185269, and AL0185270 groups could reach more than 80%.

It can be seen from Table 14 that after an A-2'5' or U-2'5' or C-2'5' or G-2'5' modification was added to positions 6-7 of the antisense strand, the efficacy could be stable, because this modification can reduce the thermal stability of the seed region, thereby reducing the off-target risk of the sequence. At a dose of 0.05 nM, the efficacy of the AL0185259 group was the best. At a concentration of 0.01 nM, the silencing activity of siRNAs was improved compared to those without the A-2'5' or U-2'5' or C-2'5' or G-2'5' modification; examples include the AL0185272, AL0185274, AL0185277, and AL0185278 groups. This indicates, to a certain extent, that at a relatively low dose, the A-2'5' or U-2'5' or C-2'5' or G-2'5' modification could sometimes achieve a relatively good target gene silencing effect.

Table 8. Inhibition results of AGT siRNAs in primary cynomolgus monkeys hepatocytes (I)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185055 | 8.05 | 0.43 | 16.47 | 2.53 |
| AL0185056 | 6.79 | *0.05* | 16.34 | 1.46 |
| AL0185057 | 5.89 | 0.48 | 14.75 | 0.68 |
| AL0185061 | 5.67 | 0.08 | 17.28 | 3.49 |
| AL0185062 | 6.81 | 0.32 | 25.78 | 7.97 |
| AL0185063 | 7.23 | *0.05* | 26.90 | 6.26 |
| AL0185064 | 6.04 | 0.14 | 10.25 | 3.08 |
| AL0185001 | 7.51 | 3.60 | 17.40 | 1.48 |
| AL0185091 | 6.66 | 0.18 | 16.33 | 3.37 |
| AL0185092 | 4.63 | 0.17 | 13.63 | 3.68 |
| AL0185093 | 4.71 | 0.62 | 13.79 | 4.38 |
| AL0185094 | 4.48 | 0.19 | 8.92 | 0.23 |

Table 9. Inhibition results of AGT siRNAs in primary cynomolgus monkey hepatocytes (II)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185081 | 47.80 | 17.05 | 31.12 | 0.93 |
| AL0185085 | 34.94 | 1.65 | 34.52 | 0.90 |
| AL0185087 | 27.14 | 0.04 | 31.37 | 0.97 |

(continued)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185089 | 35.73 | 7.75 | 43.75 | 5.96 |
| AL0185188 | 54.71 | 2.16 | 26.55 | 2.06 |
| AL0185189 | 30.76 | 1.08 | 28.96 | 1.42 |
| AL0185190 | 31.99 | 3.43 | 31.22 | 0.85 |
| AL0185191 | 30.79 | 0.91 | 30.37 | 2.19 |
| AL0185192 | 34.66 | 9.21 | 23.27 | 2.95 |
| AL0185193 | 33.75 | 0.16 | 31.94 | 6.37 |
| AL0185194 | 27.48 | 2.81 | 29.42 | 3.87 |
| AL0185195 | 22.63 | 0.87 | 26.23 | 0.59 |
| AL0185001 | 41.89 | 6.43 | 38.96 | 2.80 |

Table 10. Inhibition results of AGT siRNAs in primary cynomolgus monkey hepatocytes (III)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185104 | 51.29 | 4.92 | 102.33 | 7.82 |
| AL0185109 | 26.06 | 0.55 | 37.81 | 4.46 |
| AL0185123 | 24.58 | 3.67 | 54.22 | 4.71 |
| AL0185127 | 29.40 | 1.20 | 50.13 | 0.27 |
| AL0185139 | 27.96 | 0.59 | 46.29 | 3.26 |
| AL0185141 | 26.89 | 3.48 | 55.53 | 5.07 |
| AL0185145 | 27.85 | 1.92 | 44.43 | 5.13 |
| AL0185189 | 31.21 | 8.19 | 36.94 | 0.24 |
| AL0185195 | 25.86 | 2.16 | 30.12 | 2.19 |
| AL0185061 | 34.14 | 3.52 | 50.50 | 8.13 |
| AL0185094 | 28.09 | 1.68 | 29.37 | 1.59 |
| AL0185001 | 32.27 | 5.03 | 40.58 | 0.81 |

Table 11. Inhibition results of AGT siRNAs in primary cynomolgus monkey hepatocytes (IV)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185210 | 1.04 | 0.46 | 2.12 | 0.40 |
| AL0185215 | 0.89 | 0.13 | 2.88 | 0.82 |
| AL0185219 | 0.88 | 0.25 | 3.17 | 0.58 |
| AL0185220 | 0.74 | 0.24 | 3.24 | 0.46 |
| AL0185221 | 0.75 | 0.13 | 2.10 | 0.14 |
| AL0185222 | 1.15 | 0.14 | 5.23 | 0.39 |

(continued)

| Modified siRNA No. | Dose of 0.5 nM AGT mRNA residual activity (%) | | Dose of 0.05 nM AGT mRNA residual activity (%) | |
|---|---|---|---|---|
| | Mean | SD | Mean | SD |
| AL0185228 | 0.66 | 0.15 | 2.96 | 1.14 |
| AL0185229 | 0.72 | 0.07 | 2.94 | 0.43 |
| AL0185230 | 0.79 | 0.06 | 3.06 | 0.13 |
| AL0115001 | 1.25 | 0.16 | 4.39 | 0.57 |

Table 12. Inhibition results of AGT siRNAs in primary cynomolgus monkey hepatocytes (V)

| Modified siRNA No. | Dose of 0.01 nM AGT mRNA residual activity (%) | | Dose of 0.05 nM AGT mRNA residual activity (%) | |
|---|---|---|---|---|
| | Mean | SD | Mean | SD |
| AL0185232 | 21.18 | 4.16 | 18.11 | 3.05 |
| AL0185234 | 9.85 | 1.26 | 1.72 | 0.83 |
| AL0185235 | 24.24 | 7.17 | 20.42 | 4.79 |
| AL0185236 | 24.21 | 2.06 | 14.44 | 3.29 |
| AL0185237 | 16.32 | 1.64 | 2.09 | 1.48 |
| AL0185244 | 10.95 | 3.02 | 4.83 | 1.11 |
| AL0185245 | 18.18 | 3.35 | 16.67 | 2.76 |
| AL0185246 | 17.42 | 7.36 | 5.48 | 1.05 |
| AL0185247 | 19.13 | 4.54 | 19.74 | 5.45 |
| AL0185199 | 13.00 | 2.29 | 1.42 | 0.25 |
| AL0115001 | 14.05 | 3.77 | 14.32 | 2.02 |

Table 13. Inhibition results of AGT siRNAs in primary cynomolgus monkey hepatocytes (VI)

| Modified siRNA No. | Dose of 0.05 nM AGT mRNA residual activity (%) | | Dose of 0.01 nM AGT mRNA residual activity (%) | |
|---|---|---|---|---|
| | Mean | SD | Mean | SD |
| AL0185255 | 4.09 | 1.48 | 15.86 | 3.00 |
| AL0185266 | 4.17 | 1.59 | 21.38 | 4.20 |
| AL0185257 | 4.87 | 1.32 | 17.08 | 3.19 |
| AL0185267 | 12.78 | 3.13 | 28.98 | 4.97 |
| AL0185258 | 5.86 | 0.67 | 25.18 | 3.55 |
| AL0185268 | 3.86 | 0.81 | 14.10 | 1.05 |
| AL0185259 | 4.89 | 1.96 | 8.63 | 0.52 |
| AL0185269 | 9.77 | 1.78 | 13.48 | 2.00 |
| AL0185262 | 6.40 | 1.11 | 7.09 | 0.72 |
| AL0185270 | 5.75 | 0.29 | 12.30 | 1.40 |
| AL0185001 | 5.31 | 0.56 | 5.89 | 0.75 |

Table 14. Inhibition results of AGT siRNAs in primary cynomolgus monkey hepatocytes (VII)

| Modified siRNA No. | Dose of 0.05 nM AGT mRNA residual activity (%) | | Dose of 0.01 nM AGT mRNA residual activity (%) | |
|---|---|---|---|---|
| | Mean | SD | Mean | SD |
| AL0185271 | 8.83 | 3.00 | 82.31 | 20.34 |
| AL0185272 | 10.16 | 2.10 | 65.91 | 8.79 |
| AL0185273 | 11.14 | 3.06 | 81.68 | 11.46 |
| AL0185274 | 9.68 | 1.19 | 65.90 | 8.65 |
| AL0185259 | 6.82 | 0.86 | 103.32 | 49.22 |
| AL0185275 | 10.78 | 2.37 | 87.90 | 31.36 |
| AL0185276 | 27.12 | 9.06 | 92.20 | 49.64 |
| AL0185277 | 23.46 | 5.42 | 74.09 | 10.75 |
| AL0185278 | 15.58 | 1.56 | 97.83 | 49.93 |
| AL0185001 | 12.60 | 3.05 | 76.39 | 18.01 |

**Example 3. Activity Screening of AGT siRNAs in Liver Cell Lines**

(1) Cell culture and transfection:

The human liver cancer cell lines Hep3B, Huh7, and HepG2 (Shanghai Fushen Biotechnology Co., Ltd.) were used. Cells of the cell lines were placed in an incubator at 37 °C with 5% $CO_2$ and cultured using a DMEM culture medium (Hyclone, SH30022.01, 2 g/L glucose) supplemented with 10% FBS (aqlabteech, AQ-MV-06600) and 1% penicillin-streptomycin (Keygen Biotech, KGY0023). When the cell confluency reached 90%, the cells were digested with trypsin (Amresco, 0458-250G) and counted using a counter (Nexcelom, cellometer Mini). The cell suspensions were inoculated into 96-well plates at 150 μL/well (cell count: $2 \times 10^4$ cells/well). The next day, adherent cells were transfected.

Transfection was performed using Lipofectamine™ RNAiMAX (thermofisher, 13778150). 5 μL (5 nM, 0.5 nM, and 0.1 nM) of a compound dilution was dispersed in 20 μL of Opti-MEM (thermofisher, 1105821), and 0.2 μL of RNAiMAX was dispersed in 25 μL of Opti-MEM. The final concentrations of siRNA were 0.5 nM, 0.05 nM, and 0.01 nM, respectively. After 5 min of incubation, the RNAiMAX dispersion was mixed with the compound dispersion, and the mixture was incubated for 10 min. Cells were added to the transfection complex (n = 2) and cultured in an incubator at 37 °C with 5% $CO_2$ for 24 h.

(2) Cell lysis

After one day of transfection, the culture medium was removed, and PBS was added to wash the cells. 50 μL of lysis buffer was added to each well, and the plates were stored at -80 °C.

(3) RT-qPCR

Mixtures were prepared in RNase-free centrifuge tubes: 2.5 μL of buffer, 0.2 μL of Enzyme Mix (Foregene, DRT-02011), 0.4 μL of lysate, and 0.4 μL of 5 μM primer were mixed, and RNase-free $ddH_2O$ was added until the volume was 5 μL. Each sample was tested in triplicate. The 96-well plates were placed into a qPCR instrument (ROCGENE, Archimed), and the following program was executed: stage 1, 42 °C, 5 min; 95 °C, 10 sec; amplification, 95 °C, 5 sec; 59 °C, 20 sec; 72 °C, 10 sec; 40 cycles; dissolution curve, 95 °C, 15 sec, 59 °C, 60 sec, 95 °C, 15 sec.

(4) Statistical analysis of data:

Data were exported in EXCEL format, and $CT_{AGT}$-$CT_{hTBP}$ was used to normalize the control group. To calculate fold changes in relative silencing efficiency, data were analyzed using the ΔΔCT method. The results are shown in Tables 15 and 16. The data in each table were obtained from an individual test. Due to variations in cell batches, the target gene silencing efficiency may vary.

It can be seen from Table 15 that at doses of 0.5 nM and 0.01 nM, the mean AGT gene expression level inhibitory effects of the naked sequence AL0181240 group in the Hep3B, HepG2, and Huh7 cell strains were better than those of the

other sequences in most cases; in particular, it performed better at a low dose of 0.01 nM.

It can be seen from Table 16 that at 3 doses of 0.5 nM, 0.05 nM, and 0.01 nM, the AGT mRNA silencing efficiency of the modified sequence groups AL0185199, AL0185234, and AL0185237 was significantly better than that of the AL0185300 group (P < 0.05).

Table 15. Inhibitory effects of AGT siRNAs in liver cell lines

| Sequence No. | Dose of 0.5 nM AGT mRNA residual activity (%) | | Dose of 0.01 nM AGT mRNA residual activity (%) | |
|---|---|---|---|---|
| | Mean | SD | Mean | SD |
| **Hep3B cells** | | | | |
| AL0181115 | 18.67 | 1.90 | 47.21 | 1.36 |
| AL0181237 | 20.60 | 1.45 | 57.89 | 4.57 |
| AL0181240 | 18.95 | 1.03 | 47.13 | 4.28 |
| AL0181300 | 26.61 | 5.40 | 47.36 | 6.54 |
| **HepG2 cells** | | | | |
| AL0181115 | 41.12 | 5.07 | 64.48 | 4.59 |
| AL0181237 | 45.61 | 1.45 | 73.91 | 8.52 |
| AL0181240 | 35.34 | 5.78 | 61.02 | 6.27 |
| AL0181300 | 56.96 | 5.36 | 69.74 | 3.64 |
| **Huh7 cell** | | | | |
| AL0181115 | 11.71 | 1.02 | 46.68 | 7.60 |
| AL0181237 | 8.22 | 0.97 | 69.42 | 5.85 |
| AL0181240 | 12.32 | 1.23 | 34.99 | 3.23 |
| AL0181300 | 16.98 | 2.56 | 62.85 | 6.83 |

Table 16. Inhibitory effects of AGT siRNAs in liver cell lines

| Sequence No. | Dose of 0.5 nM AGT mRNA residual activity (%) | | Dose of 0.05 nM AGT mRNA residual activity (%) | | Dose of 0.01 nM AGT mRNA residual activity (%) | | t test p value |
|---|---|---|---|---|---|---|---|
| | Mean | SD | Mean | SD | Mean | SD | |
| AL0185199 | 16.86 | 3.05 | 27.43 | 2.16 | 49.41 | 4.70 | 0.012 |
| AL0185234 | 15.87 | 4.58 | 24.87 | 3.53 | 51.84 | 6.88 | 0.038 |
| AL0185237 | 12.15 | 1.08 | 24.12 | 6.80 | 44.56 | 12.98 | 0.004 |
| AL0185300 | 24.04 | 3.75 | 45.31 | 6.20 | 83.47 | 14.00 | - |
| Note: Paired t tests were performed with AL018300. | | | | | | | |

**Example 4.** ***In Vivo*** **Test I of AGT RNAi Agents in Mice**

SPF male B6.Cg-Tg (hAGT) 2041Sig/J mice aged 8-9 weeks (source: Jackson Laboratory) were used in the test. Pre-dose serum samples were obtained on day 0 of dosing, and the animals were randomized into groups according to their hAGT levels. Each B6.Cg-Tg (hAGT) 2041 Sig/J mouse was subcutaneously given a single dose of an AGT RNAi agent at 3 mg/kg. Blood samples were collected from mice (from their eyeballs, sent for testing within 1 h of collecting blood) at weeks 1, 2, 3, 4, 5, 6, 7, and 8 post-dose, and the hAGT expression levels were determined. With the pre-dose levels as controls, the hAGT knockdown levels were determined. During the experiment, no death or moribundity was observed in any of the animals. Clinical observation showed no significant abnormalities in any of the animals.

The experimental groups are shown in Table 17, and hAGT change levels are shown in FIG. 3.

Table 17. Treatment groups

| No. | Group | Dose | Mode of administration |
|---|---|---|---|
| 1 | AL0187002 | 3mg/kg | Single subcutaneous injection |
| 2 | AL0187003 | 3mg/kg | Single subcutaneous injection |
| 3 | AL0187004 | 3mg/kg | Single subcutaneous injection |
| 4 | AL0187005 | 3mg/kg | Single subcutaneous injection |
| 5 | AL0187006 | 3mg/kg | Single subcutaneous injection |
| 6 | AL0187007 | 3mg/kg | Single subcutaneous injection |
| 7 | AL0187008 | 3mg/kg | Single subcutaneous injection |
| 8 | AL0187009 | 3mg/kg | Single subcutaneous injection |
| 9 | AL0187010 | 3mg/kg | Single subcutaneous injection |
| 10 | AL0187011 | 3mg/kg | Single subcutaneous injection |

It can be seen from FIG. 3 that compared to the pre-dose levels, at week 3 after drug intervention, the knockdown effects of all AGT siRNAs other than AL0187004 reached minima (more than 90%), and then gradually and slowly increased; after week 6, the blood hAGT levels in the AL0187002, AL0187004, and AL0187009 groups had returned to the pre-dose levels. All drug interventions could significantly reduce the blood hAGT levels in mice, and the AL0187003, AL0187006, AL0187007, AL0187008, AL0187010, and AL0187011 groups exhibited the most significant and longest-lasting reducing effects. Moreover, 8 weeks after dosing, the inhibitory effects of the drugs against hAGT remained at about 50%, and the effects of the AL0187005, AL0187006, AL0187007, AL0187008, AL0187010, and AL0187011 groups were significantly different from that of the AL0187002 group ($P < 0.05$).

**Example 4. *In Vivo* Test II of AGT RNAi Agents in Mice**

siRNA sequences exhibiting relatively good efficacy *in vitro* and *in vivo* were differently modified to investigate the effects of different modifications on efficacy. SPF male B6.Cg-Tg (hAGT) 2041Sig/J mice aged 8-9 weeks (source: Jackson Laboratory) were used in the test. Pre-dose serum samples were obtained on day 0 of dosing, and the animals were randomized into groups according to their hAGT levels. Each B6.Cg-Tg (hAGT) 2041Sig/J mouse was subcutaneously given a single dose of an AGT RNAi agent at 3 mg/kg. Blood samples were collected from mice (from their eyeballs, sent for testing within 1 h of collecting blood) at weeks 1, 2, 3, 4, and 5 post-dose, and the hAGT expression levels were determined. With the pre-dose levels as controls, the hAGT knockdown levels were determined. During the experiment, no death or moribundity was observed in any of the animals. Clinical observation showed no significant abnormalities in any of the animals.

The experimental groups are shown in Table 18, and hAGT change levels are shown in FIG. 4.

Table 18. Treatment groups

| No. | Group | Dose | Mode of administration |
|---|---|---|---|
| 1 | AL0187002 | 3mg/kg | Single subcutaneous injection |
| 2 | AL0187006 | 3mg/kg | Single subcutaneous injection |
| 3 | AL0187012 | 3mg/kg | Single subcutaneous injection |
| 4 | AL0187013 | 3mg/kg | Single subcutaneous injection |
| 5 | AL0187014 | 3mg/kg | Single subcutaneous injection |
| 6 | AL0187015 | 3mg/kg | Single subcutaneous injection |
| 7 | AL0187016 | 3mg/kg | Single subcutaneous injection |
| 8 | AL0187017 | 3mg/kg | Single subcutaneous injection |
| 9 | AL0187018 | 3mg/kg | Single subcutaneous injection |
| 10 | AL0187019 | 3mg/kg | Single subcutaneous injection |

It can be seen from FIG. 4 that compared to the pre-dose levels, at week 2 after drug intervention, the knockdown effects of all AGT siRNAs other than AL0187002, AL0187013, and AL0187015 reached minima (more than 90%), and then gradually and slowly increased; after week 5, the blood hAGT levels in the AL0187002 and AL0187013 groups recovered rapidly. All drug interventions could significantly reduce the blood hAGT levels in mice, and the AL0187006, AL0187012, AL0187014, AL0187015, AL0187016, AL0187017, AL0187018, and AL0187019 groups exhibited the most significant effects. Moreover, 5 weeks after dosing, the inhibitory effects of the drugs against hAGT remained at about 75%, and the effects were significantly different from that of the AL0187002 group ($P < 0.05$).

**Example 5. *In Vivo* Test III of AGT RNAi Agents in Mice**

siRNA sequences exhibiting relatively good efficacy *in vitro* and *in vivo* were differently modified to investigate the effects of different modifications on efficacy. SPF male B6.Cg-Tg (hAGT) 2041Sig/J mice aged 8-9 weeks (source: Jackson Laboratory) were used in the test. Pre-dose serum samples were obtained on day 0 of dosing, and the animals were randomized into groups according to their hAGT levels. Each B6.Cg-Tg (hAGT) 2041Sig/J mouse was subcutaneously given a single dose of an AGT RNAi agent at 3 mg/kg. Blood samples were collected from mice (from their eyeballs, sent for testing within 1 h of collecting blood) at weeks 1, 2, 3, 4, and 5 post-dose, and the hAGT expression levels were determined. With the pre-dose levels as controls, the hAGT knockdown levels were determined. During the experiment, no death or moribundity was observed in any of the animals. Clinical observation showed no significant abnormalities in any of the animals.

The experimental groups are shown in Table 19, and hAGT change levels are shown in FIGs. 5 and 6.

Table 19. Treatment groups

| No. | Group | Dose | Mode of administration |
|---|---|---|---|
| 1 | AL0187002 | 3mg/kg | Single subcutaneous injection |
| 2 | AL0187020 | 3mg/kg | Single subcutaneous injection |
| 3 | AL0187021 | 3mg/kg | Single subcutaneous injection |
| 4 | AL0187022 | 3mg/kg | Single subcutaneous injection |
| 5 | AL0187023 | 3mg/kg | Single subcutaneous injection |
| 6 | AL0187024 | 3mg/kg | Single subcutaneous injection |
| 7 | AL0187025 | 3mg/kg | Single subcutaneous injection |
| 8 | AL0187026 | 3mg/kg | Single subcutaneous injection |
| 9 | AL0187028 | 3mg/kg | Single subcutaneous injection |
| 10 | AL0187029 | 3mg/kg | Single subcutaneous injection |
| 11 | AL0187030 | 3mg/kg | Single subcutaneous injection |
| 12 | AL0187031 | 3mg/kg | Single subcutaneous injection |
| 13 | AL0187032 | 3mg/kg | Single subcutaneous injection |
| 14 | AL0187033 | 3mg/kg | Single subcutaneous injection |
| 15 | AL0187034 | 3mg/kg | Single subcutaneous injection |

It can be seen from FIGs. 5 and 6 that compared to the pre-dose levels, at week 2 after drug intervention, the knockdown effects of AGT siRNAs other than the AL0187002, AL0187021, AL0187030, and AL0187033 groups reached minima (more than 90%), and then gradually and slowly increased; after week 5, the blood hAGT levels in the AL0187030, AL0187033, and AL0187002 groups recovered rapidly. All drug interventions could significantly reduce the blood hAGT levels in mice, and the AL0187020, AL0187023, AL0187024, AL0187025, and AL0187028 groups exhibited the most significant effects. Moreover, 5 weeks after dosing, the inhibitory effects of the drugs against hAGT could still reach 80%, and the effects were significantly different from that of the AL0187002 group ($P < 0.05$).

**Example 6. *In Vivo* Test IV of AGT RNAi Agents in Mice**

The experiments were the same as that in Example 5. The groups are shown in Tables 20 and 21. Two experiments were conducted separately. During the experiments, no death or moribundity was observed in any of the

animals. Clinical observation showed no significant abnormalities in any of the animals. hAGT change levels are shown in FIGs. 7 and 8.

Table 20. Treatment groups

| No. | Group | Dose | Mode of administration |
|---|---|---|---|
| 1 | AL0187002 | 3mg/kg | Single subcutaneous injection |
| 2 | AL0187037 | 3mg/kg | Single subcutaneous injection |
| 3 | AL0187040 | 3mg/kg | Single subcutaneous injection |
| 4 | AL0187041 | 3mg/kg | Single subcutaneous injection |
| 5 | AL0187042 | 3mg/kg | Single subcutaneous injection |
| 6 | AL0187043 | 3mg/kg | Single subcutaneous injection |
| 7 | AL0187044 | 3mg/kg | Single subcutaneous injection |
| 8 | AL0187045 | 3mg/kg | Single subcutaneous injection |
| 9 | AL0187046 | 3mg/kg | Single subcutaneous injection |
| 10 | AL0187047 | 3mg/kg | Single subcutaneous injection |
| 11 | AL0187048 | 3mg/kg | Single subcutaneous injection |
| 12 | AL0187049 | 3mg/kg | Single subcutaneous injection |
| 13 | AL0187050 | 3mg/kg | Single subcutaneous injection |
| 14 | AL0187024 | 3mg/kg | Single subcutaneous injection |

Table 21. Treatment groups

| No. | Group | Dose | Mode of administration |
|---|---|---|---|
| 1 | AL0187002 | 1mg/kg | Single subcutaneous injection |
| 2 | AL0187024 | 1mg/kg | Single subcutaneous injection |
| 3 | AL0187040 | 1mg/kg | Single subcutaneous injection |
| 4 | AL0187047 | 1mg/kg | Single subcutaneous injection |
| 5 | AL0187051 | 1mg/kg | Single subcutaneous injection |
| 6 | AL0187052 | 1mg/kg | Single subcutaneous injection |
| 7 | AL0187053 | 1mg/kg | Single subcutaneous injection |
| 8 | AL0187054 | 1mg/kg | Single subcutaneous injection |

It can be seen from FIG. 7 that compared to the pre-dose levels, at week 2 after drug intervention, the knockdown effects of AGT siRNAs other than the AL0187041 group reached minima (more than 80%), and then gradually and slowly increased. All drug interventions could significantly reduce the blood hAGT levels in mice, and the AL0187024, AL0187037, AL0187040, AL0187043, AL0187045, AL0187047, AL0187048, and AL0187049 groups exhibited the most significant effects. Moreover, 5 weeks after dosing, the inhibitory effects of the drugs against hAGT could still reach 80%.

It can be seen from FIG. 8 that at a dose of 1 mg/kg, compared to the pre-dose levels, at week 2 after drug intervention, the knockdown effects of AGT siRNAs reached minima, and then gradually and slowly increased. All drug interventions could significantly reduce the blood hAGT levels in mice, and the AL0187024, AL0187040, AL0187047, and AL0187051 groups exhibited the most significant effects. Moreover, 4 weeks after dosing, the inhibitory effects of the drugs against hAGT could still reach 80%.

**Example 7. Efficacy Test of AGT RNAi Agents in Non-Human Primates (NHPs)**

The efficacy of siRNA sequences exhibiting relatively good efficacy in hAGT transgenic mice was evaluated in cynomolgus monkeys. Male cynomolgus monkeys aged 10-22 years (source: Kunming Biomed International Ltd. (KBI)) were used in the test. Pre-dose serum samples were obtained on day 0 of dosing, and the animals were randomized into

groups according to their AGT levels. Each experimental group was given an AGT RNAi agent at 1 mg/kg. The experiment included the following three groups:

Group 1: Blood samples were collected from cynomolgus monkeys at weeks 1, 2, 3, 4, and 5 post-dose, and the AGT expression levels were determined. With the pre-dose levels as controls, the AGT knockdown levels were determined. During the experiment, clinical observation showed no significant abnormalities in any of the animals. The experimental groups are shown in Table 22, and AGT change levels are shown in FIG. 9.
Group 2: Blood samples were collected from cynomolgus monkeys at weeks 1, 2, 3, 4, 5, and 6 post-dose, and the AGT expression levels were determined. With the pre-dose levels as controls, the AGT knockdown levels were determined. During the experiment, clinical observation showed no significant abnormalities in any of the animals. The experimental groups are shown in Table 23, and AGT change levels are shown in FIG. 10.
Group 3: Blood samples were collected from cynomolgus monkeys at weeks 1, 2, 3, 4, 5, 6, 7, 8, and 10 post-dose, and the AGT expression levels were determined. With the pre-dose levels as controls, the AGT knockdown levels were determined. During the experiment, clinical observation showed no significant abnormalities in any of the animals. The experimental groups are shown in Table 24, and AGT change levels are shown in FIG. 11.

Table 22. Treatment groups

| No. | Group | Dose | Mode of administration |
|---|---|---|---|
| 1 | AL0187002 | 1mg/kg | Single subcutaneous injection |
| 2 | AL0187020 | 1mg/kg | Single subcutaneous injection |
| 3 | AL0187021 | 1mg/kg | Single subcutaneous injection |
| 4 | AL0187022 | 1mg/kg | Single subcutaneous injection |
| 5 | AL0187024 | 1mg/kg | Single subcutaneous injection |

Table 23. Treatment groups

| No. | Group | Dose | Mode of administration |
|---|---|---|---|
| 1 | AL0187043 | 1mg/kg | Single subcutaneous injection |
| 2 | AL0187045 | 1mg/kg | Single subcutaneous injection |
| 3 | AL0187047 | 1mg/kg | Single subcutaneous injection |

Table 24. Treatment groups

| No. | Group | Dose | Mode of administration |
|---|---|---|---|
| 1 | AL0187040 | 1 mg/kg | Single subcutaneous injection |
| 2 | AL0187051 | 1mg/kg | Single subcutaneous injection |

It can be seen from FIGs. 9, 10, and 11 that compared to the pre-dose levels, at week 3 after drug intervention, the knockdown effects of all AGT siRNAs reached minima, and then gradually and slowly increased. All drug interventions could significantly reduce the blood AGT levels in cynomolgus monkeys. In the experiment for group 1, the AL0187020, AL0187022, and AL0187024 groups exhibited the most significant efficacy. Moreover, 5 weeks after dosing, the inhibitory effects of the drugs against AGT could still reach 75%, and the effects were significantly different from that of the AL0187002 group ($P < 0.05$). In the experiments for groups 2 and 3, the AL0187047, AL0187040, and AL0187051 groups exhibited significant efficacy, and at week 7 post-dose, their inhibitory effects against AGT could still reach 70%, or even more than 80%.

## Claims

1. An oligonucleotide or a pharmaceutically acceptable salt thereof for reducing AGT expression, wherein the oligonucleotide comprises a sense strand and an antisense strand; the sense strand has a sequence having at least 80% or

more sequence identity, preferably 85%, 90%, 95%, 96%, 97%, 98%, 99% or more sequence identity, to any one of the sequences set forth in SEQ ID NOs: 2-219, 789-804, and 868-871 or a fragment thereof, or a modified sequence of the sequence or the fragment thereof, and the antisense strand has a sequence having at least 80% or more sequence identity, preferably 85%, 90%, 95%, 96%, 97%, 98%, 99% or more sequence identity, to any one of the sequences set forth in SEQ ID NOs: 221-424, 805-821, and 872-875 or a fragment thereof, or a modified sequence of the sequence or the fragment thereof;

and/or the modified sequence of the sense strand comprises any one selected from the sequences set forth in SEQ ID NOs: 426-598, 822-838, and 876-886;
and/or the modified sequence of the antisense strand comprises any one selected from the sequences set forth in SEQ ID NOs: 600-676, 678-788, 839-867, and 887-911.

**2.** The oligonucleotide or the pharmaceutically acceptable salt thereof according to claim 1, wherein the oligonucleotide or the pharmaceutically acceptable salt thereof is selected from a carboxylate, a sodium salt, a triethylamine salt, and other pharmaceutically acceptable salts.

**3.** The oligonucleotide or the pharmaceutically acceptable salt thereof according to claim 1 or 2, wherein the sense strand comprises any one selected from the unmodified oligonucleotides set forth in SEQ ID NOs: 74, 101, 107, 108, 111-113, 129, 133, 135, 137, 148, 166, 178, 184, 789, 794, 798, 799, 800, 801, 802, 803, 804, and 870, or any one selected from the modified oligonucleotides set forth in SEQ ID NOs: 471, 477, 478, 481-483, 518, 536, 548, 554, 592-598, 822, 827, 831, 832, 833, 834, 835, 836, 837, and 838; the antisense strand comprises any one selected from the unmodified oligonucleotides set forth in SEQ ID NOs: 259, 281, 286, 311, 317, 318, 319, 334, 338, 340, 342, 353, 371, 383, 389, 805, 808, 811, 812, 813, 814, 815, 816, 817, 818, and 874, or any one selected from the modified oligonucleotides set forth in SEQ ID NOs: 643, 644, 645, 647, 648, 681, 678, 679, 680, 764, 610, 773, 774, 775, 776, 777, 778, 779, 780, 782, 783, 785, 839, 854, 857, 858, 859, 860, 861, 862, 863, and 864.

**4.** The oligonucleotide or the pharmaceutically acceptable salt thereof according to any one of claims 1-3, wherein the oligonucleotide comprises any one selected from the following combinations of sense and antisense strands:

(1) the sense strand comprises the sequence set forth in SEQ ID NO: 101, and the antisense strand comprises the sequence set forth in SEQ ID NO: 311;
(2) the sense strand comprises the sequence set forth in SEQ ID NO: 107, and the antisense strand comprises the sequence set forth in SEQ ID NO: 281;
(3) the sense strand comprises the sequence set forth in SEQ ID NO: 108, and the antisense strand comprises the sequence set forth in SEQ ID NO: 286;
(4) the sense strand comprises the sequence set forth in SEQ ID NO: 111, and the antisense strand comprises the sequence set forth in SEQ ID NO: 317;
(5) the sense strand comprises the sequence set forth in SEQ ID NO: 112, and the antisense strand comprises the sequence set forth in SEQ ID NO: 318;
(6) the sense strand comprises the sequence set forth in SEQ ID NO: 113, and the antisense strand comprises the sequence set forth in SEQ ID NO: 319;
(7) the sense strand comprises the sequence set forth in SEQ ID NO: 129, and the antisense strand comprises the sequence set forth in SEQ ID NO: 334;
(8) the sense strand comprises the sequence set forth in SEQ ID NO: 133, and the antisense strand comprises the sequence set forth in SEQ ID NO: 338;
(9) the sense strand comprises the sequence set forth in SEQ ID NO: 135, and the antisense strand comprises the sequence set forth in SEQ ID NO: 340;
(10) the sense strand comprises the sequence set forth in SEQ ID NO: 137, and the antisense strand comprises the sequence set forth in SEQ ID NO: 342;
(11) the sense strand comprises the sequence set forth in SEQ ID NO: 148, and the antisense strand comprises the sequence set forth in SEQ ID NO: 353;
(12) the sense strand comprises the sequence set forth in SEQ ID NO: 166, and the antisense strand comprises the sequence set forth in SEQ ID NO: 371;
(13) the sense strand comprises the sequence set forth in SEQ ID NO: 178, and the antisense strand comprises the sequence set forth in SEQ ID NO: 383;
(14) the sense strand comprises the sequence set forth in SEQ ID NO: 184, and the antisense strand comprises the sequence set forth in SEQ ID NO: 389;
(15) the sense strand comprises the sequence set forth in SEQ ID NO: 113, and the antisense strand comprises

the sequence set forth in SEQ ID NO: 808;
(16) the sense strand comprises the sequence set forth in SEQ ID NO: 113, and the antisense strand comprises the sequence set forth in SEQ ID NO: 811;
(17) the sense strand comprises the sequence set forth in SEQ ID NO: 113, and the antisense strand comprises the sequence set forth in SEQ ID NO: 818;
(18) the sense strand comprises the sequence set forth in SEQ ID NO: 789, and the antisense strand comprises the sequence set forth in SEQ ID NO: 805;
(19) the sense strand comprises the sequence set forth in SEQ ID NO: 794, and the antisense strand comprises the sequence set forth in SEQ ID NO: 805;
(20) the sense strand comprises the sequence set forth in SEQ ID NO: 798, and the antisense strand comprises the sequence set forth in SEQ ID NO: 805;
(21) the sense strand comprises the sequence set forth in SEQ ID NO: 799, and the antisense strand comprises the sequence set forth in SEQ ID NO: 812;
(22) the sense strand comprises the sequence set forth in SEQ ID NO: 800, and the antisense strand comprises the sequence set forth in SEQ ID NO: 813;
(23) the sense strand comprises the sequence set forth in SEQ ID NO: 801, and the antisense strand comprises the sequence set forth in SEQ ID NO: 814;
(24) the sense strand comprises the sequence set forth in SEQ ID NO: 802, and the antisense strand comprises the sequence set forth in SEQ ID NO: 815;
(25) the sense strand comprises the sequence set forth in SEQ ID NO: 803, and the antisense strand comprises the sequence set forth in SEQ ID NO: 816;
(26) the sense strand comprises the sequence set forth in SEQ ID NO: 804, and the antisense strand comprises the sequence set forth in SEQ ID NO: 817;
(27) the sense strand comprises the sequence set forth in SEQ ID NO: 868, and the antisense strand comprises the sequence set forth in SEQ ID NO: 872;
(28) the sense strand comprises the sequence set forth in SEQ ID NO: 869, and the antisense strand comprises the sequence set forth in SEQ ID NO: 873;
(29) the sense strand comprises the sequence set forth in SEQ ID NO: 870, and the antisense strand comprises the sequence set forth in SEQ ID NO: 874;
(30) the sense strand comprises the sequence set forth in SEQ ID NO: 871, and the antisense strand comprises the sequence set forth in SEQ ID NO: 875; and
(31) the sense strand comprises the sequence set forth in SEQ ID NO: 74, and the antisense strand comprises the sequence set forth in SEQ ID NO: 259;

wherein each of the strands is independently 19 to 25 nucleotides in length.

**5.** The oligonucleotide or the pharmaceutically acceptable salt thereof according to any one of claims 1-4, wherein the oligonucleotide comprises at least one modified nucleotide;

and/or at least one of the modified nucleotide(s) is selected from the group consisting of a deoxynucleotide, a 3'-terminal deoxythymidine (dT) nucleotide, a 2'-O-methyl-modified nucleotide, a 2'-fluoro-modified nucleotide, a 2'-deoxy-modified nucleotide, a locked nucleotide, a 2'-5'-linked ribonucleotide (3'-RNA), an unlocked nucleotide, a conformationally restricted nucleotide, a constrained ethyl nucleotide, an abasic nucleotide, a 2'-amino-modified nucleotide, a 2'-O-allyl-modified nucleotide, a 2'-O-alkyl-modified nucleotide, a 2'-hydroxy-modified nucleotide, a 2'-methoxyethyl-modified nucleotide, a 2'-O-alkyl-modified nucleotide, a morpholino nucleotide, a phosphoramidate, a nucleotide comprising a non-natural base, a tetrahydropyran-modified nucleotide, a 1,5-anhydrohexitol-modified nucleotide, a cyclohexenyl-modified nucleotide, a nucleotide comprising a phosphorothioate group, a nucleotide comprising a methylphosphonate group, a nucleotide comprising a 5'-phosphate, a nucleotide comprising a 5'-phosphate mimic, a vinyl-phosphonate nucleotide, a thermally destabilizing nucleotide, a glycol-modified nucleotide, a nucleotide comprising a 2'-phosphate, and a 2-O-(N-methylacetamide)-modified nucleotide, and a combination thereof;
and/or at least one of the modified nucleotide(s) is selected from the group consisting of LNA, HNA, CeNA, 2'-methoxyethyl, 2'-O-alkyl, 2'-O-allyl, 2'-C-allyl, 2'-fluoro, 2'-deoxy, 2'-hydroxy, and ethylene glycol, and a combination thereof;
and/or at least one of the modified nucleotide(s) is selected from the group consisting of a deoxynucleotide, a 2'-O-methyl-modified nucleotide, a 2'-fluoro-modified nucleotide, a 2'-deoxy-modified nucleotide, an ethylene glycol-modified nucleotide (GNA), a nucleotide comprising a 2'-phosphate, and a nucleotide comprising a phosphorothioate group, and a combination thereof;

and/or the oligonucleotide comprises at least one 2'-modified nucleotide;

and/or the 2'-modified nucleotide is one or more selected from a 2'-alkoxy-modified nucleotide, a 2'-substituted alkoxy-modified nucleotide, a 2'-alkyl-modified nucleotide, a 2'-substituted alkyl-modified nucleotide, a 2'-fluoro-modified nucleotide, a 2'-acylamino-modified nucleotide, a 2'-deoxy-modified nucleotide, a 2'-O-allyl-modified nucleotide, a 2'-O-alkyl-modified nucleotide, a 2'-hydroxy-modified nucleotide, a 2'-methoxyethyl-modified nucleotide, a 2'-amino-modified nucleotide, a 2'-substituted amino-modified nucleotide, a 2'-deoxynucleotide, a nucleotide comprising a 2'-phosphate, and a 2'-O-(N-methylacetamide)-modified nucleotide;

and/or the 2'-modification is a modification selected from 2'-methoxy, 2'-acetylamino, 2'-aminoethyl, 2'-fluoro, 2'-O-methoxyethyl, and 2'-fluoro-β-d-arabinonucleic acid;

and/or the oligonucleotide may comprise a glycol nucleic acid (GNA) modification; preferably, the glycol nucleic acid (GNA) modification is selected from an adenosine-glycol nucleic acid, a cytidine-glycol nucleic acid, a thymidine-glycol nucleic acid, and a guanosine-glycol nucleic acid; preferably, the glycol nucleic acid (GNA) modification is selected from a thymidine-glycol nucleic acid S-isomer (Tgn) represented by formula (I), a cytidine-glycol nucleic acid S-isomer (Cgn) represented by formula (II), an adenosine-glycol nucleic acid S-isomer (Agn) represented by formula (III), and a guanosine-glycol nucleic acid S-isomer (Ggn) represented by formula (IV):

Tgn

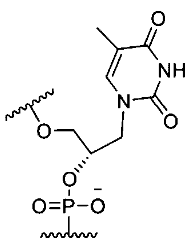

(I),

Cgn

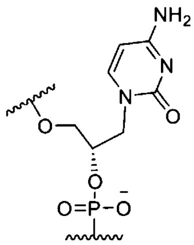

(II),

Agn

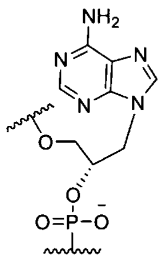

(III), and

Ggn

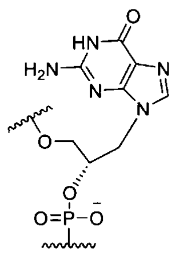

(IV);

and/or the oligonucleotide may comprise a 2'-5'-phosphodiester linkage; preferably, the oligonucleotide comprises a modification selected from uridine-2'-phosphate (U-2'5') represented by formula (V), guanosine-2'-phosphate (G-2'5') represented by formula (VI), cytidine-2'-phosphate (C-2'5') represented by formula (VII), adenosine-2'-phosphate (A-2'5') represented by formula (VIII), and thymidine-2'-phosphate (T-2'5') represented by formula (IX):

(U-2'5')

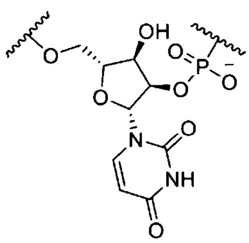

(V),

(G-2'5')

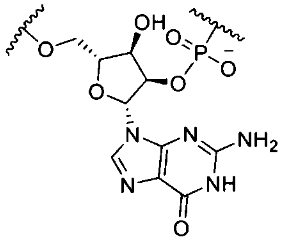

(VI),

(C-2'5')

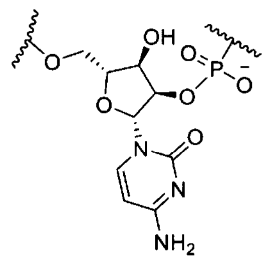

(VII),

(A-2'5')

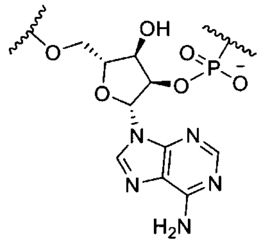

(VIII), and

(T-2'5')

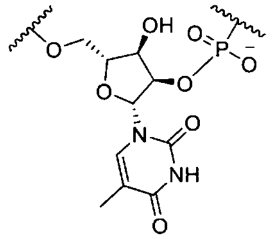

(IX);

and/or the oligonucleotide further comprises a 5'-phosphate analog-modified nucleotide;

and/or the 5'-phosphate analog-modified nucleotide is APU represented by formula (X); preferably, the 5'-phosphate analog-modified nucleotide is VPUm represented by formula (XI):

APU

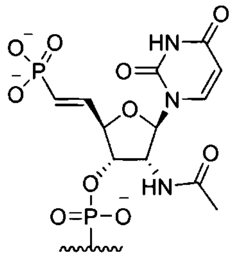

(X) and

VPUm

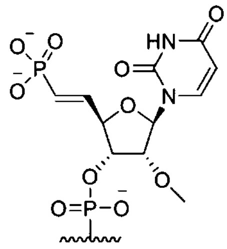

(XI) .

6. The oligonucleotide or the pharmaceutically acceptable salt thereof according to any one of claims 1-5, wherein the oligonucleotide comprises at least one modified internucleotide linkage;
and/or the at least one modified internucleotide linkage is a phosphorothioate linkage.

7. The oligonucleotide or the pharmaceutically acceptable salt thereof according to any one of claims 1-6, wherein the oligonucleotide comprises any one selected from the following combinations of sense and antisense strands:

(1) the sense strand comprises the sequence set forth in SEQ ID NO: 477, and the antisense strand comprises the sequence set forth in SEQ ID NO: 643;
(2) the sense strand comprises the sequence set forth in SEQ ID NO: 471, and the antisense strand comprises the sequence set forth in SEQ ID NO: 644;
(3) the sense strand comprises the sequence set forth in SEQ ID NO: 478, and the antisense strand comprises the sequence set forth in SEQ ID NO: 645;
(4) the sense strand comprises the sequence set forth in SEQ ID NO: 482, and the antisense strand comprises the sequence set forth in SEQ ID NO: 647;
(5) the sense strand comprises the sequence set forth in SEQ ID NO: 483, and the antisense strand comprises the sequence set forth in SEQ ID NO: 648;
(6) the sense strand comprises the sequence set forth in SEQ ID NO: 481, and the antisense strand comprises the sequence set forth in SEQ ID NO: 681;
(7) the sense strand comprises the sequence set forth in SEQ ID NO: 471, and the antisense strand comprises the sequence set forth in SEQ ID NO: 678;
(8) the sense strand comprises the sequence set forth in SEQ ID NO: 478, and the antisense strand comprises the sequence set forth in SEQ ID NO: 679;
(9) the sense strand comprises the sequence set forth in SEQ ID NO: 483, and the antisense strand comprises the sequence set forth in SEQ ID NO: 680;
(10) the sense strand comprises the sequence set forth in SEQ ID NO: 594, and the antisense strand comprises the sequence set forth in SEQ ID NO: 764;
(11) the sense strand comprises the sequence set forth in SEQ ID NO: 593, and the antisense strand comprises the sequence set forth in SEQ ID NO: 610;
(12) the sense strand comprises the sequence set forth in SEQ ID NO: 592, and the antisense strand comprises the sequence set forth in SEQ ID NO: 773;
(13) the sense strand comprises the sequence set forth in SEQ ID NO: 592, and the antisense strand comprises the sequence set forth in SEQ ID NO: 774;
(14) the sense strand comprises the sequence set forth in SEQ ID NO: 592, and the antisense strand comprises the sequence set forth in SEQ ID NO: 775;
(15) the sense strand comprises the sequence set forth in SEQ ID NO: 595, and the antisense strand comprises the sequence set forth in SEQ ID NO: 776;
(16) the sense strand comprises the sequence set forth in SEQ ID NO: 596, and the antisense strand comprises the sequence set forth in SEQ ID NO: 777;
(17) the sense strand comprises the sequence set forth in SEQ ID NO: 597, and the antisense strand comprises the sequence set forth in SEQ ID NO: 778;
(18) the sense strand comprises the sequence set forth in SEQ ID NO: 598, and the antisense strand comprises the sequence set forth in SEQ ID NO: 779;
(19) the sense strand comprises the sequence set forth in SEQ ID NO: 518, and the antisense strand comprises the sequence set forth in SEQ ID NO: 780;

(20) the sense strand comprises the sequence set forth in SEQ ID NO: 536, and the antisense strand comprises the sequence set forth in SEQ ID NO: 782;
(21) the sense strand comprises the sequence set forth in SEQ ID NO: 548, and the antisense strand comprises the sequence set forth in SEQ ID NO: 783;
(22) the sense strand comprises the sequence set forth in SEQ ID NO: 554, and the antisense strand comprises the sequence set forth in SEQ ID NO: 785;
(23) the sense strand comprises the sequence set forth in SEQ ID NO: 592, and the antisense strand comprises the sequence set forth in SEQ ID NO: 854;
(24) the sense strand comprises the sequence set forth in SEQ ID NO: 592, and the antisense strand comprises the sequence set forth in SEQ ID NO: 857;
(25) the sense strand comprises the sequence set forth in SEQ ID NO: 592, and the antisense strand comprises the sequence set forth in SEQ ID NO: 864;
(26) the sense strand comprises the sequence set forth in SEQ ID NO: 822, and the antisense strand comprises the sequence set forth in SEQ ID NO: 839;
(27) the sense strand comprises the sequence set forth in SEQ ID NO: 827, and the antisense strand comprises the sequence set forth in SEQ ID NO: 839;
(28) the sense strand comprises the sequence set forth in SEQ ID NO: 831, and the antisense strand comprises the sequence set forth in SEQ ID NO: 839;
(29) the sense strand comprises the sequence set forth in SEQ ID NO: 598, and the antisense strand comprises the sequence set forth in SEQ ID NO: 840;
(30) the sense strand comprises the sequence set forth in SEQ ID NO: 832, and the antisense strand comprises the sequence set forth in SEQ ID NO: 841;
(31) the sense strand comprises the sequence set forth in SEQ ID NO: 832, and the antisense strand comprises the sequence set forth in SEQ ID NO: 842;
(32) the sense strand comprises the sequence set forth in SEQ ID NO: 832, and the antisense strand comprises the sequence set forth in SEQ ID NO: 848;
(33) the sense strand comprises the sequence set forth in SEQ ID NO: 832, and the antisense strand comprises the sequence set forth in SEQ ID NO: 849;
(34) the sense strand comprises the sequence set forth in SEQ ID NO: 832, and the antisense strand comprises the sequence set forth in SEQ ID NO: 850;
(35) the sense strand comprises the sequence set forth in SEQ ID NO: 833, and the antisense strand comprises the sequence set forth in SEQ ID NO: 858;
(36) the sense strand comprises the sequence set forth in SEQ ID NO: 834, and the antisense strand comprises the sequence set forth in SEQ ID NO: 859;
(37) the sense strand comprises the sequence set forth in SEQ ID NO: 835, and the antisense strand comprises the sequence set forth in SEQ ID NO: 860;
(38) the sense strand comprises the sequence set forth in SEQ ID NO: 836, and the antisense strand comprises the sequence set forth in SEQ ID NO: 861;
(39) the sense strand comprises the sequence set forth in SEQ ID NO: 837, and the antisense strand comprises the sequence set forth in SEQ ID NO: 862;
(40) the sense strand comprises the sequence set forth in SEQ ID NO: 838, and the antisense strand comprises the sequence set forth in SEQ ID NO: 863;
(41) the sense strand comprises the sequence set forth in SEQ ID NO: 882, and the antisense strand comprises the sequence set forth in SEQ ID NO: 894;
(42) the sense strand comprises the sequence set forth in SEQ ID NO: 882, and the antisense strand comprises the sequence set forth in SEQ ID NO: 902;
(43) the sense strand comprises the sequence set forth in SEQ ID NO: 884, and the antisense strand comprises the sequence set forth in SEQ ID NO: 896;
(44) the sense strand comprises the sequence set forth in SEQ ID NO: 884, and the antisense strand comprises the sequence set forth in SEQ ID NO: 903;
(45) the sense strand comprises the sequence set forth in SEQ ID NO: 592, and the antisense strand comprises the sequence set forth in SEQ ID NO: 905;
(46) the sense strand comprises the sequence set forth in SEQ ID NO: 592, and the antisense strand comprises the sequence set forth in SEQ ID NO: 907;
(47) the sense strand comprises the sequence set forth in SEQ ID NO: 882, and the antisense strand comprises the sequence set forth in SEQ ID NO: 910; and
(48) the sense strand comprises the sequence set forth in SEQ ID NO: 882, and the antisense strand comprises the sequence set forth in SEQ ID NO: 911;

wherein each of the strands is independently 19 to 25 nucleotides in length.

**8.** The oligonucleotide or the pharmaceutically acceptable salt thereof according to any one of claims 1-7, wherein at least one nucleotide of the oligonucleotide is conjugated to one or more targeting ligands;

and/or the targeting ligands comprise a carbohydrate, an amino sugar, cholesterol, a polypeptide, or a lipid;
and/or the targeting ligands comprise a N-acetylgalactosamine (GalNAc) moiety;
and/or the GalNac moiety is a monovalent GalNAc moiety, a bivalent GalNAc moiety, a trivalent GalNAc moiety, or a tetravalent GalNAc moiety;
and/or the targeting ligands are A1 represented by formula (XII):

A1

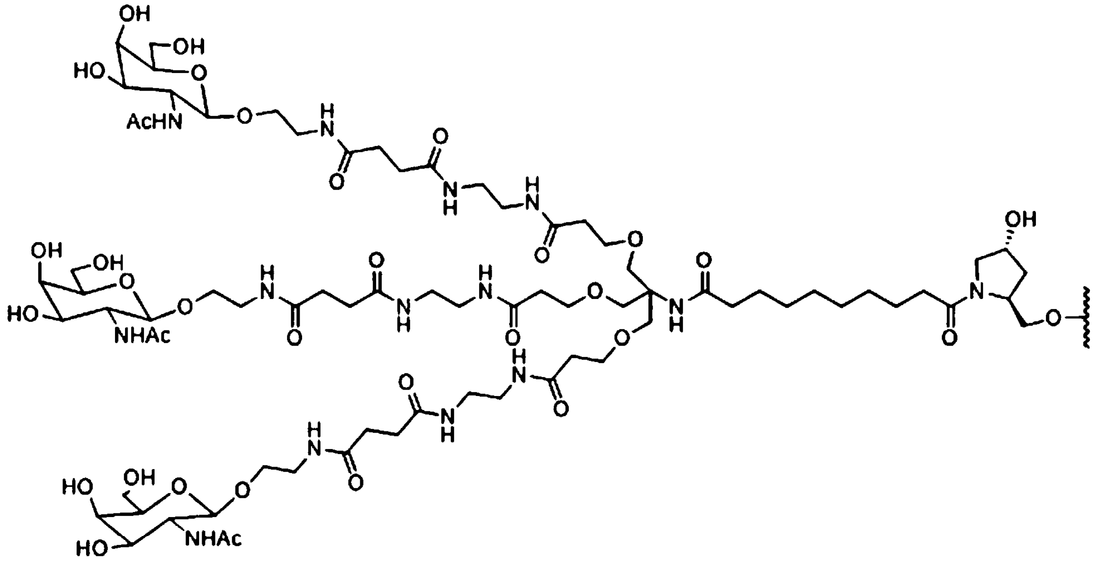

(XII)

and/or the targeting ligands are L96 represented by formula (XIII):

L96

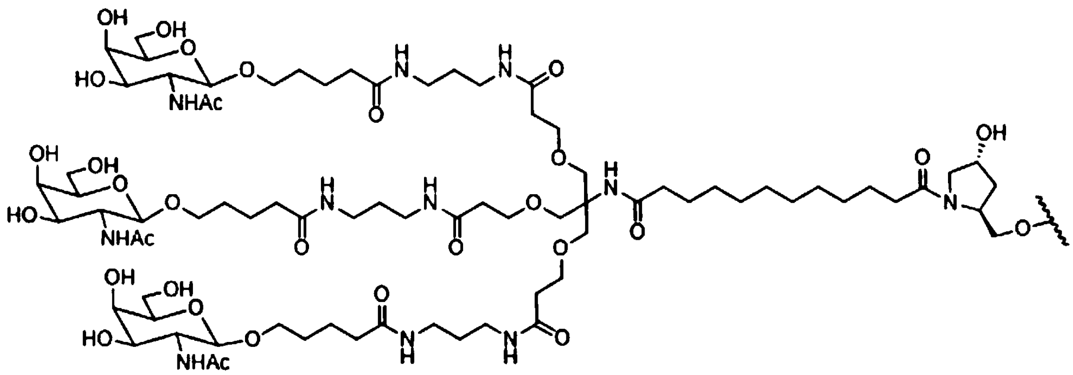

(XIII).

**9.** A composition, comprising the oligonucleotide or the pharmaceutically acceptable salt thereof according to any one of claims 1 to 8, and optionally a pharmaceutically acceptable carrier,

and/or wherein a dosage form of the composition is an oral agent or an injection; the injection is selected from an intravenous injection, a subcutaneous injection, or an intramuscular injection;
and/or the combination further comprises another drug for treating and/or preventing an AGT-related disease.

**10.** Use of the oligonucleotide or the pharmaceutically acceptable salt thereof according to any one of claims 1 to 8 or the composition according to claim 9 in the preparation of a medicament for treating or/and preventing an AGT-related disease, wherein the AGT-related disease is selected from hypertension, e.g., borderline hypertension (also known

as prehypertension), primary hypertension (also known as essential hypertension or idiopathic hypertension), secondary hypertension (also known as inessential hypertension), hypertensive emergency (also known as malignant hypertension), hypertensive urgency, isolated systolic or diastolic hypertension, pregnancy-related hypertension (e.g., preeclampsia, eclampsia, and postpartum preeclampsia), diabetic hypertension, resistant hypertension, refractory hypertension, paroxysmal hypertension, renovascular hypertension (also known as renal hypertension), Goldblatt hypertension, ocular hypertension, glaucoma, pulmonary hypertension, portal hypertension, systemic venous hypertension, systolic hypertension, labile hypertension, hypertensive heart disease, hypertensive nephropathy, atherosclerosis, arteriosclerosis, vasculopathy (including peripheral vascular disease), diabetic nephropathy, diabetic retinopathy, chronic heart failure, cardiomyopathy, diabetic cardiomyopathy, glomerulosclerosis, coarctation of the aorta, aortic aneurysm, ventricular fibrosis, Cushing's syndrome and other glucocorticoid excess states (including chronic steroid therapy), pheochromocytoma, reninoma, secondary hyperaldosteronism and other mineralocorticoid excess states, sleep apnea, thyroid/parathyroid disease, heart failure (e.g., left ventricular systolic dysfunction), myocardial infarction, angina pectoris, stroke, diabetes (e.g., diabetic nephropathy), nephropathy (e.g., chronic kidney disease or diabetic nephropathy, optionally in a pregnant environment), renal failure (e.g., chronic renal failure), cognitive disorder (e.g., Alzheimer's disease), and systemic sclerosis (e.g., scleroderma renal crisis); or the AGT-related disease is selected from intrauterine growth retardation (IUGR) and fetal growth restriction.

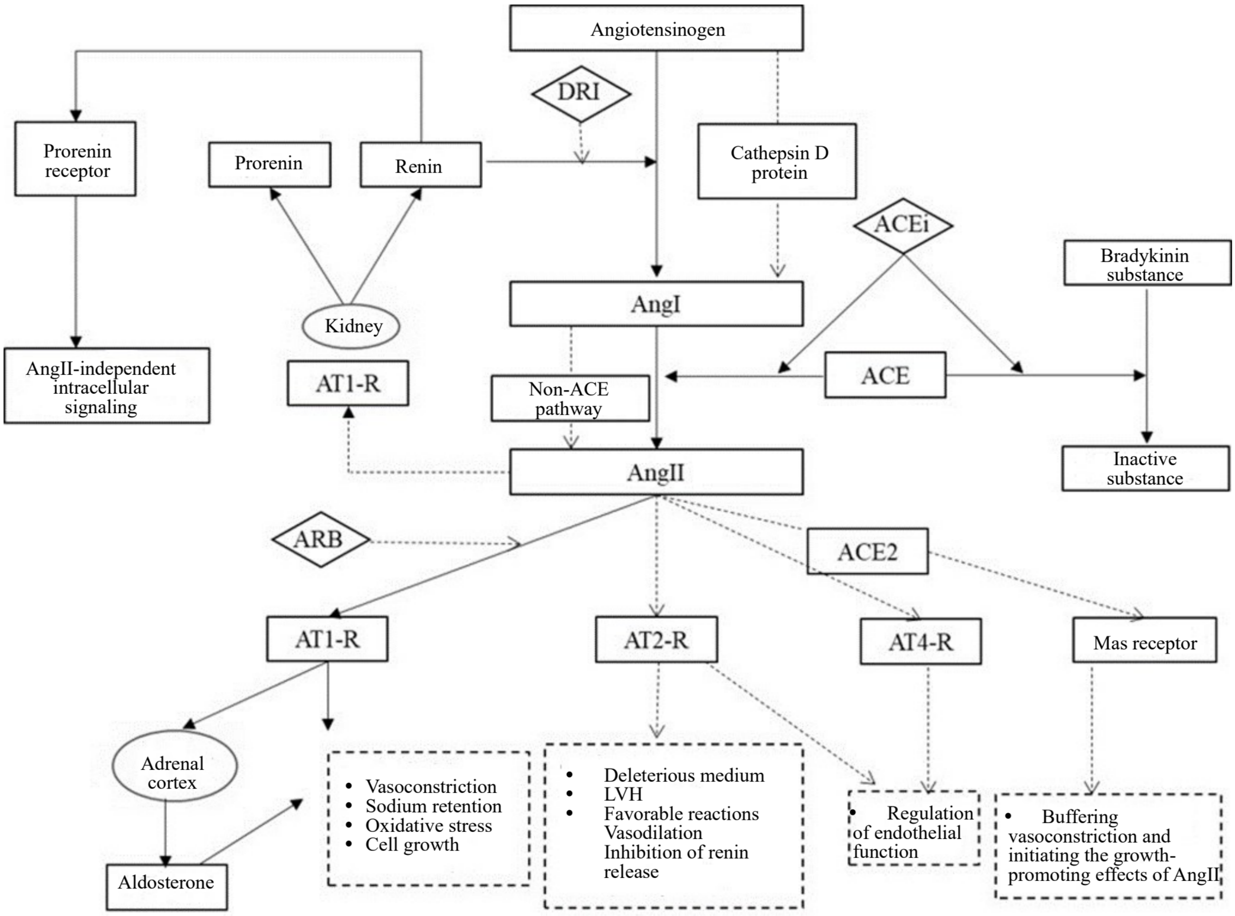
Angiotensinogen
DRI
Prorenin receptor
Prorenin
Renin
Cathepsin D protein
ACEi
Bradykinin substance
AngI
Kidney
AngII-independent intracellular signaling
AT1-R
Non-ACE pathway
ACE
Inactive substance
AngII
ARB
ACE2
AT1-R
AT2-R
AT4-R
Mas receptor
Adrenal cortex
• Vasoconstriction
• Sodium retention
• Oxidative stress
• Cell growth
• Deleterious medium
• LVH
• Favorable reactions
Vasodilation
Inhibition of renin release
• Regulation of endothelial function
• Buffering vasoconstriction and initiating the growth-promoting effects of AngII
Aldosterone

FIG. 1

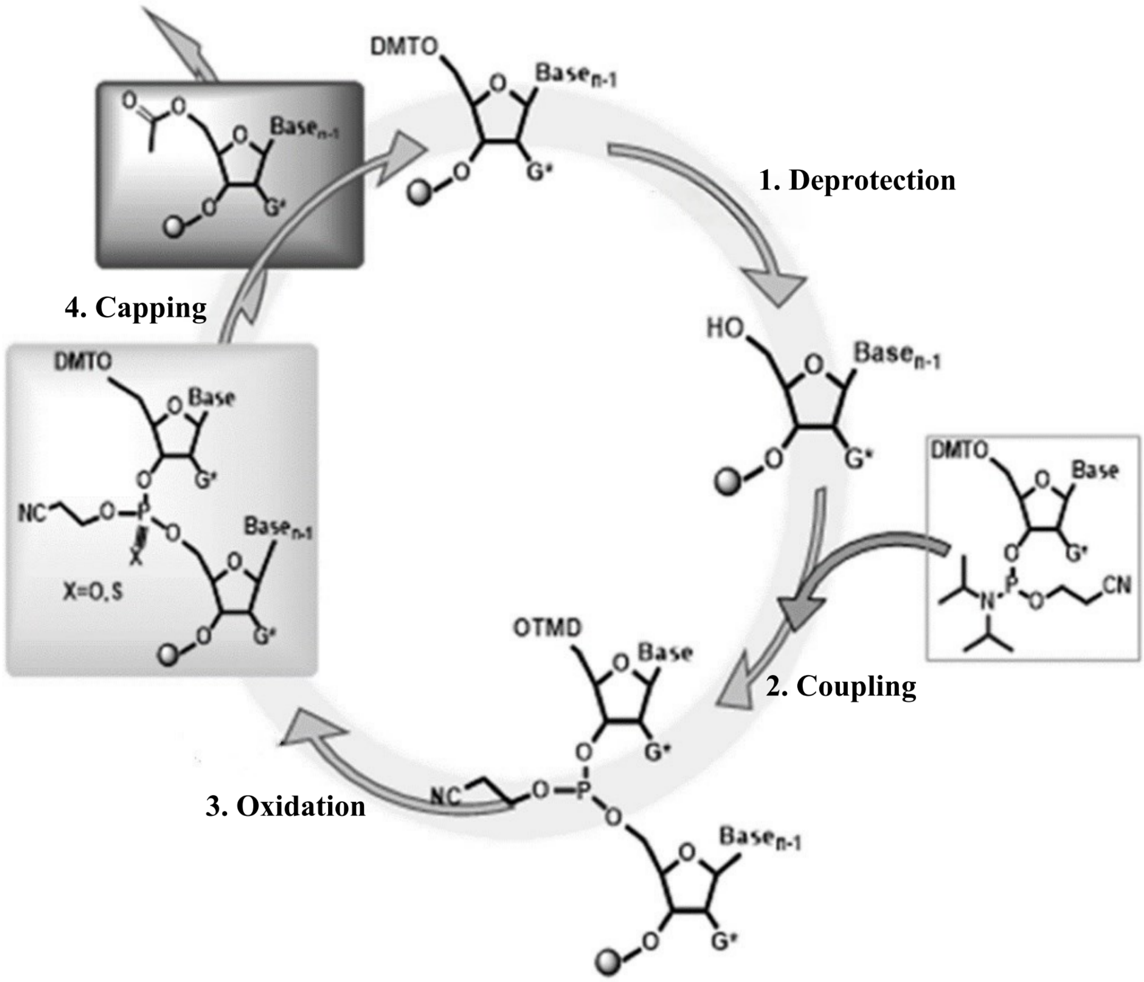
1. Deprotection
2. Coupling
3. Oxidation
4. Capping

FIG. 2

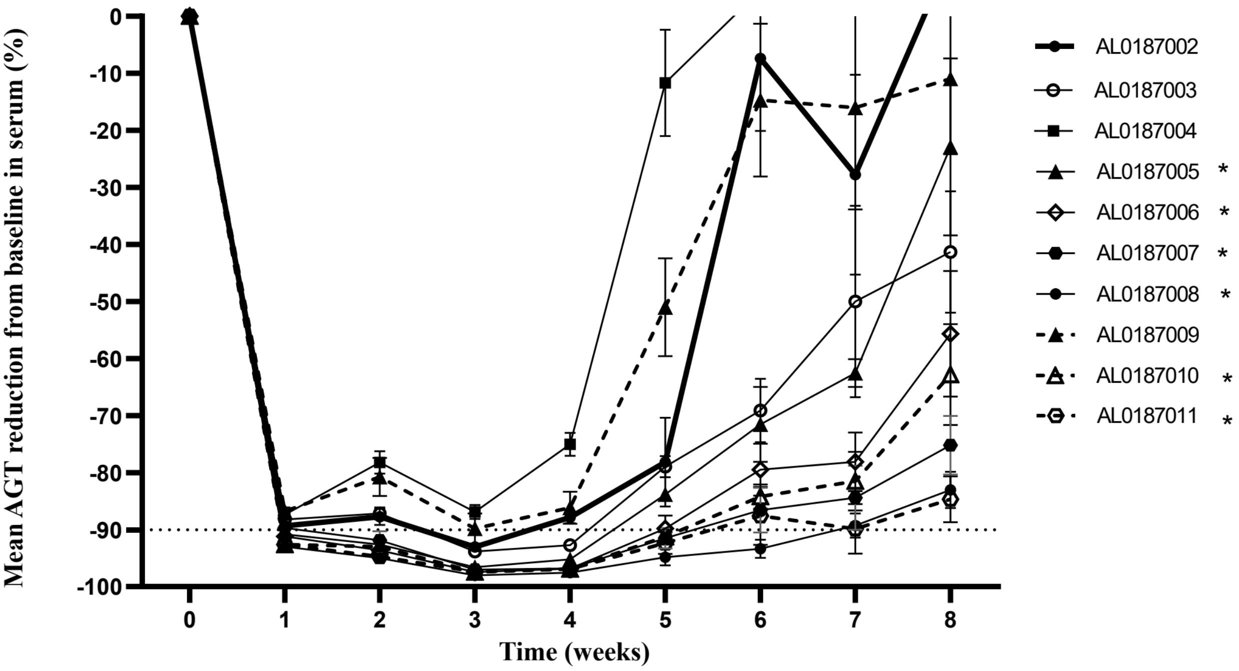
Mean AGT reduction from baseline in serum (%)
0
-10
-20
-30
-40
-50
-60
-70
-80
-90
-100
0
1
2
3
4
5
6
7
8
Time (weeks)
AL0187002
AL0187003
AL0187004
AL0187005 *
AL0187006 *
AL0187007 *
AL0187008 *
AL0187009
AL0187010 *
AL0187011 *

FIG. 3

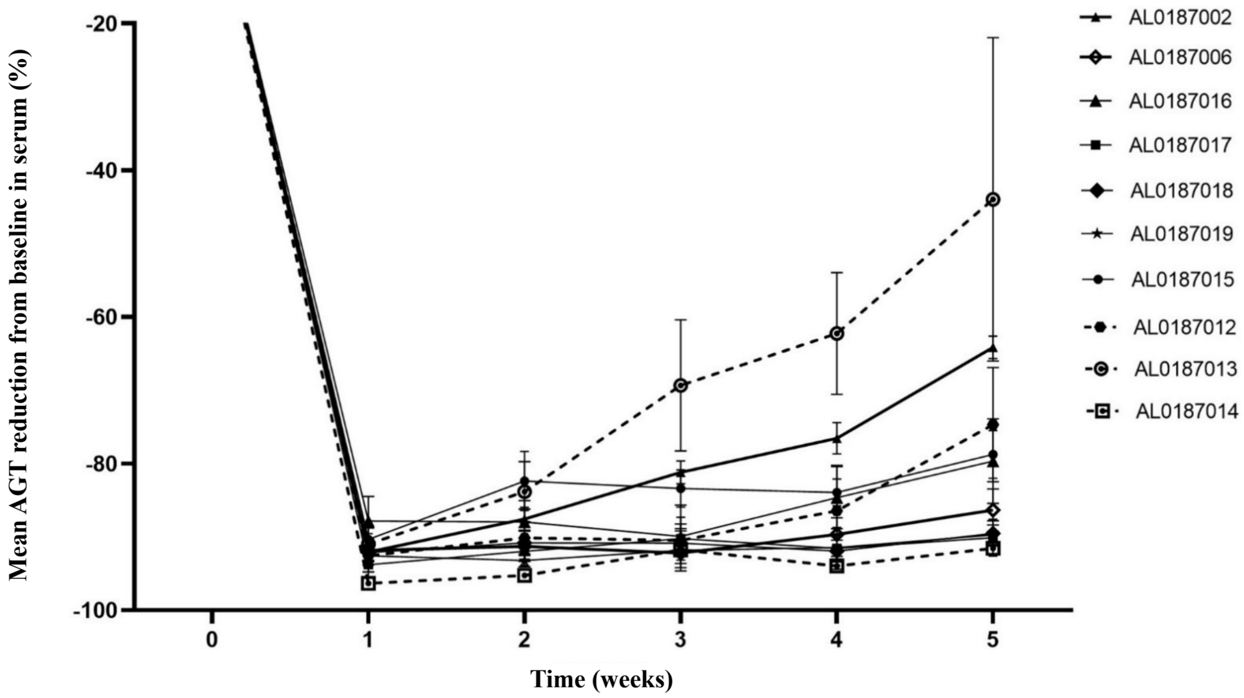

Mean AGT reduction from baseline in serum (%)
-20
-40
-60
-80
-100
0
1
2
3
4
5
Time (weeks)
AL0187002
AL0187006
AL0187016
AL0187017
AL0187018
AL0187019
AL0187015
AL0187012
AL0187013
AL0187014


FIG. 4

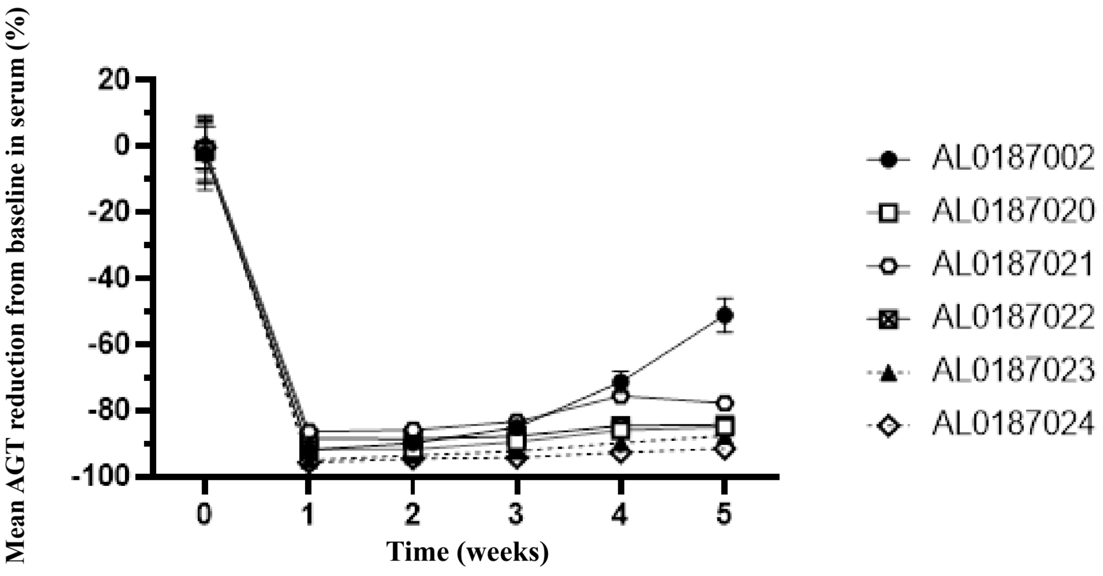

Mean AGT reduction from baseline in serum (%)
20
0
-20
-40
-60
-80
-100
0
1
2
3
4
5
Time (weeks)
AL0187002
AL0187020
AL0187021
AL0187022
AL0187023
AL0187024


FIG. 5

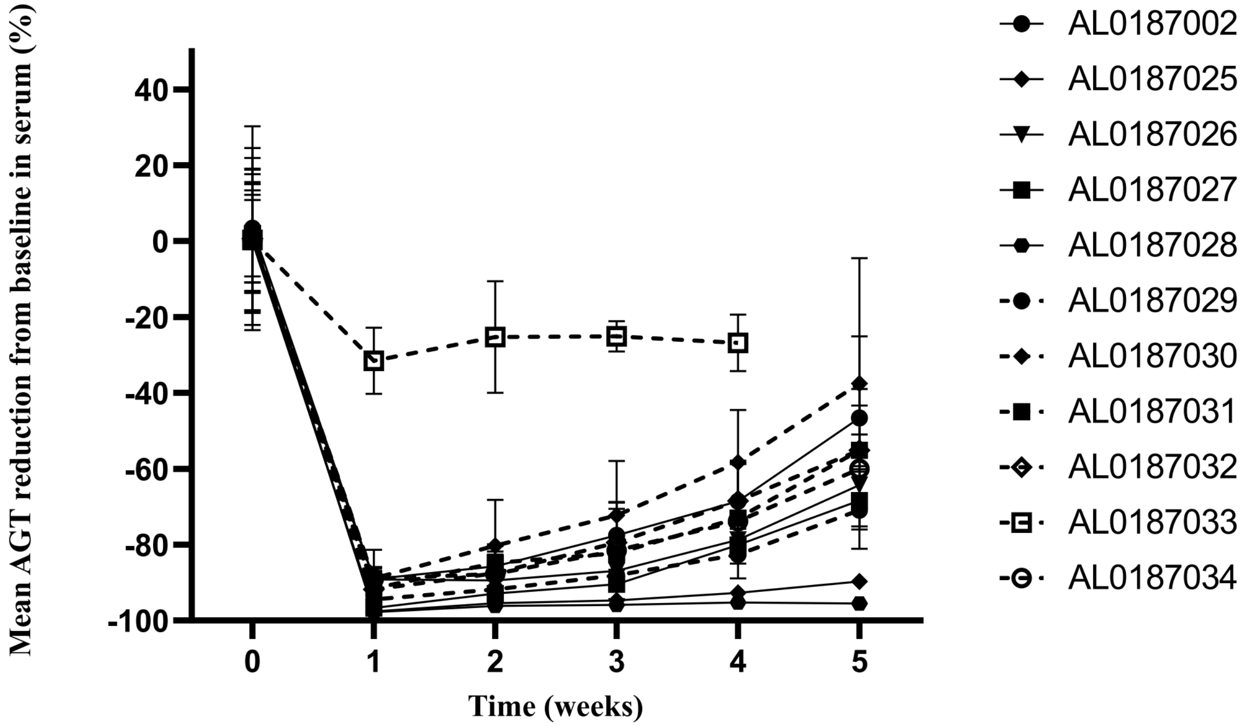
Mean AGT reduction from baseline in serum (%)
40
20
0
-20
-40
-60
-80
-100
0
1
2
3
4
5
Time (weeks)
AL0187002
AL0187025
AL0187026
AL0187027
AL0187028
AL0187029
AL0187030
AL0187031
AL0187032
AL0187033
AL0187034

FIG. 6

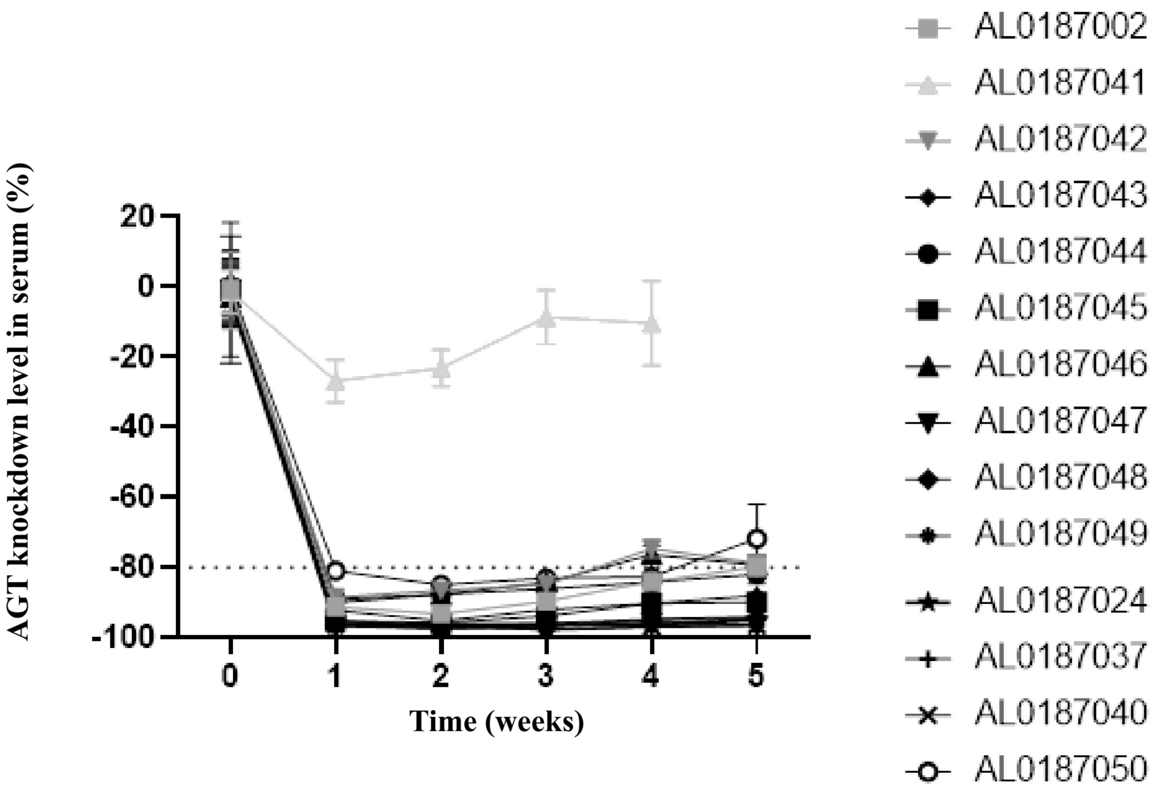
AGT knockdown level in serum (%)
20
0
-20
-40
-60
-80
-100
0
1
2
3
4
5
Time (weeks)
AL0187002
AL0187041
AL0187042
AL0187043
AL0187044
AL0187045
AL0187046
AL0187047
AL0187048
AL0187049
AL0187024
AL0187037
AL0187040
AL0187050

FIG. 7

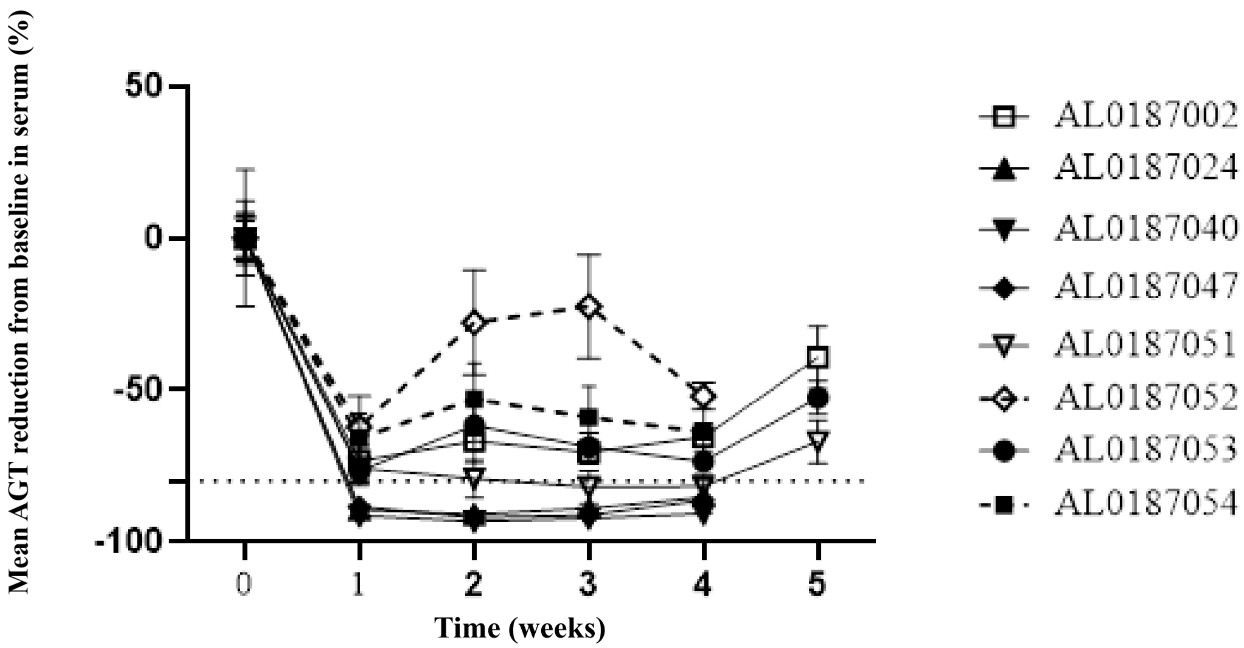
Mean AGT reduction from baseline in serum (%)
50
0
-50
-100
0
1
2
3
4
5
Time (weeks)
AL0187002
AL0187024
AL0187040
AL0187047
AL0187051
AL0187052
AL0187053
AL0187054

FIG. 8

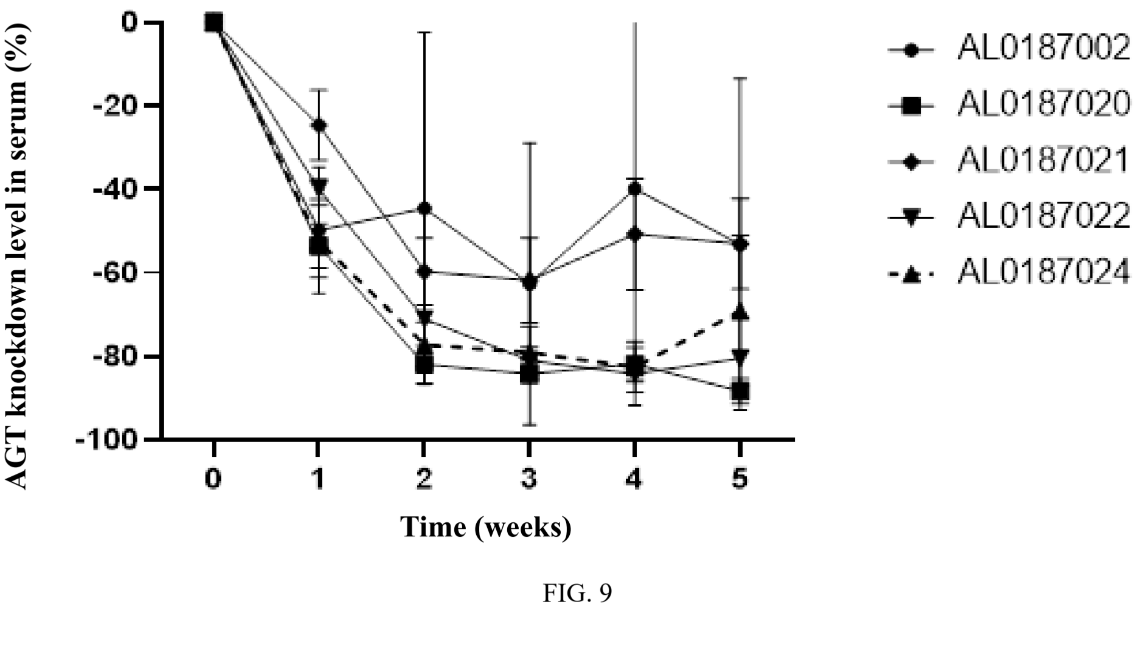
AGT knockdown level in serum (%)
0
-20
-40
-60
-80
-100
0
1
2
3
4
5
Time (weeks)
AL0187002
AL0187020
AL0187021
AL0187022
AL0187024

FIG. 9

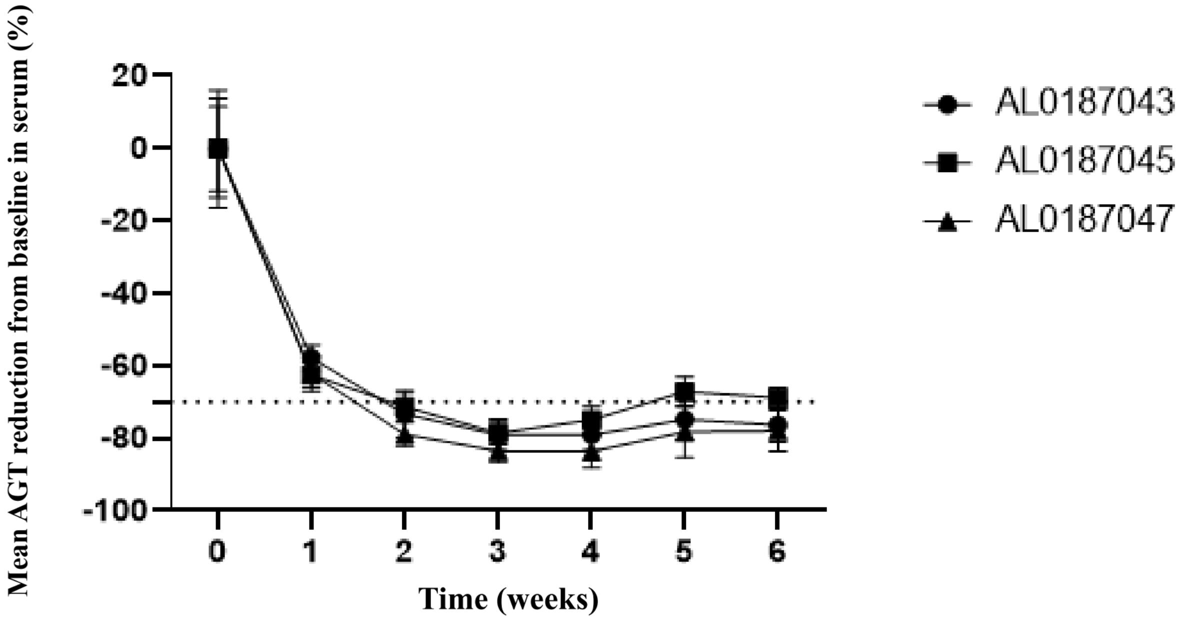
Mean AGT reduction from baseline in serum (%)
20
0
-20
-40
-60
-80
-100
0
1
2
3
4
5
6
Time (weeks)
AL0187043
AL0187045
AL0187047

FIG. 10

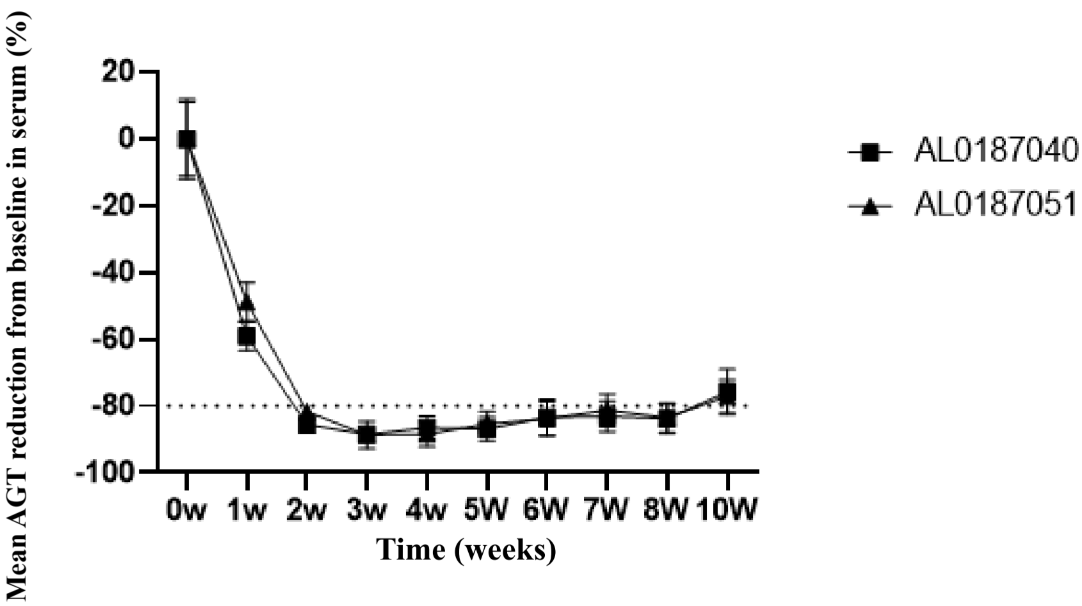
Mean AGT reduction from baseline in serum (%)
20
0
-20
-40
-60
-80
-100
0w
1w
2w
3w
4w
5W
6W
7W
8W
10W
Time (weeks)
AL0187040
AL0187051

FIG. 11

## INTERNATIONAL SEARCH REPORT

International application No.
**PCT/CN2024/105377**

### A. CLASSIFICATION OF SUBJECT MATTER

C12N15/113(2010.01)i； A61K31/713(2006.01)i； A61P9/12(2006.01)i； A61P9/10(2006.01)i； A61P9/00(2006.01)i； A61P13/12(2006.01)i； A61P9/04(2006.01)i； A61P3/10(2006.01)i

According to International Patent Classification (IPC) or to both national classification and IPC

### B. FIELDS SEARCHED

Minimum documentation searched (classification system followed by classification symbols)

IPC:C12N,A61K,A61P.

Documentation searched other than minimum documentation to the extent that such documents are included in the fields searched

Electronic data base consulted during the international search (name of data base and, where practicable, search terms used)

VEN, CNABS, PubMed, DWPI, CNTXT, WOTXT, USTXT, EPTXT, JPTXT, CNKI, ISI of Web Science, 百度学术, BAIDU SCHOLAR, Patentics, 中国生物序列检索系统, China Biological Sequence Search System, NCBI Genbank, EBI, STNext: 申请人, 发明人, 序列检索, sequence search, 血管紧张素原, AGT, angiotensinogen, ANHU, IRNA, RNAI, SERPINA8, SHRNA, SIRNA.

### C. DOCUMENTS CONSIDERED TO BE RELEVANT

| Category* | Citation of document, with indication, where appropriate, of the relevant passages | Relevant to claim No. |
|---|---|---|
| X | CN 106574268 A (ALNYLAM PHARMACEUTICALS, INC.) 19 April 2017 (2017-04-19)<br>claims 1-101, and description, tables 3, 4, 7, 8, 11, 13, and 15 | 1-10 |
| X | WO 2023088227 A1 (SHANGHAI ARGO BIOPHARMACEUTICAL CO., LTD.) 25 May 2023 (2023-05-25)<br>claims 1-74, and description, table 4 | 1-10 |
| X | CN 112313335 A (ALNYLAM PHARMACEUTICALS, INC.) 02 February 2021 (2021-02-02)<br>claims 1-78, and description, table 3 | 1-10 |
| A | CN 114981431 A (ALNYLAM PHARMACEUTICALS, INC.) 30 August 2022 (2022-08-30)<br>claims 1-97 | 1-10 |
| A | CN 114763547 A (SYNERK BIOTECH LIMITED) 19 July 2022 (2022-07-19)<br>claims 1-9 | 1-10 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **31 October 2024** | **05 November 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/105377**

**C. DOCUMENTS CONSIDERED TO BE RELEVANT**

| Category* | Citation of document, with indication, where appropriate, of the relevant passages | Relevant to claim No. |
|---|---|---|
| A | WO 2023284763 A1 (SUZHOU RIBO LIFE SCIENCE CO., LTD.) 19 January 2023 (2023-01-19) claims 1-49 | 1-10 |
| A | WO 2023088427 A1 (TUOJIE BIOTECH SHANGHAI CO., LTD.) 25 May 2023 (2023-05-25) claims 1-13 | 1-10 |

Form PCT/ISA/210 (second sheet) (July 2022)

**INTERNATIONAL SEARCH REPORT**

International application No.
**PCT/CN2024/105377**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/105377**

| Patent document cited in search report | | | Publication date (day/month/year) | Patent family member(s) | | | Publication date (day/month/year) |
|---|---|---|---|---|---|---|---|
| CN | 106574268 | A | 19 April 2017 | WO | 2015179724 | A1 | 26 November 2015 |
| | | | | WO | 2015179724 | A8 | 18 February 2016 |
| | | | | WO | 2015179724 | A9 | 10 March 2016 |
| | | | | SG | 11201609376 | SA | 29 December 2016 |
| | | | | BR | 122020023687 | B1 | 07 March 2023 |
| | | | | JP | 2017517511 | A | 29 June 2017 |
| | | | | JP | 6811094 | B2 | 13 January 2021 |
| | | | | IL | 281638 | A | 31 May 2021 |
| | | | | IL | 281638 | B1 | 01 March 2023 |
| | | | | IL | 281638 | B2 | 01 July 2023 |
| | | | | IL | 248816 | A0 | 31 January 2017 |
| | | | | IL | 248816 | B | 29 July 2021 |
| | | | | US | 2023123192 | A1 | 20 April 2023 |
| | | | | US | 2019298842 | A1 | 03 October 2019 |
| | | | | US | 10814007 | B2 | 27 October 2020 |
| | | | | AU | 2015264038 | A1 | 01 December 2016 |
| | | | | AU | 2015264038 | A2 | 22 December 2016 |
| | | | | AU | 2015264038 | B2 | 11 February 2021 |
| | | | | EP | 3739048 | A1 | 18 November 2020 |
| | | | | CA | 2948381 | A1 | 26 November 2015 |
| | | | | CA | 2948381 | C | 07 November 2023 |
| | | | | AU | 2021202552 | A1 | 27 May 2021 |
| | | | | US | 2017189541 | A1 | 06 July 2017 |
| | | | | US | 10238749 | B2 | 26 March 2019 |
| | | | | BR | 112016026950 | A2 | 31 October 2017 |
| | | | | BR | 112016026950 | B1 | 07 March 2023 |
| | | | | KR | 20170005130 | A | 11 January 2017 |
| | | | | KR | 102410687 | B1 | 22 June 2022 |
| | | | | MX | 2021006053 | A | 06 July 2021 |
| | | | | JP | 2022104985 | A | 12 July 2022 |
| | | | | JP | 7566814 | B2 | 15 October 2024 |
| | | | | KR | 20220087576 | A | 24 June 2022 |
| | | | | JP | 2021063085 | A | 22 April 2021 |
| | | | | JP | 7101748 | B2 | 15 July 2022 |
| | | | | NZ | 727615 | A | 05 July 2024 |
| | | | | SG | 10202104570 | TA | 29 June 2021 |
| | | | | US | 2021046187 | A1 | 18 February 2021 |
| | | | | US | 11419942 | B2 | 23 August 2022 |
| | | | | EP | 3146049 | A1 | 29 March 2017 |
| | | | | EP | 3146049 | B1 | 26 February 2020 |
| | | | | EA | 201692370 | A1 | 31 March 2017 |
| | | | | MX | 2016015126 | A | 23 February 2017 |
| | | | | CA | 3215908 | A1 | 26 November 2015 |
| | | | | ZA | 201607666 | B | 30 January 2019 |
| WO | 2023088227 | A1 | 25 May 2023 | KR | 20240103025 | A | 03 July 2024 |
| | | | | US | 2024084304 | A1 | 14 March 2024 |
| | | | | ECSP | 24036299 | A | 31 July 2024 |
| | | | | CO | 2024006020 | A2 | 08 August 2024 |
| | | | | AU | 2022394667 | A1 | 13 June 2024 |
| | | | | TW | 202334422 | A | 01 September 2023 |

Form PCT/ISA/210 (patent family annex) (July 2022)

**INTERNATIONAL SEARCH REPORT**
**Information on patent family members**

International application No.
**PCT/CN2024/105377**

| Patent document cited in search report | Publication date (day/month/year) | Patent family member(s) | Publication date (day/month/year) |
|---|---|---|---|
| | | IL 312811 A | 01 July 2024 |
| | | MX 2024005862 A | 10 July 2024 |
| | | EP 4435103 A1 | 25 September 2024 |
| | | CA 3238865 A1 | 25 May 2023 |
| CN 112313335 A | 02 February 2021 | ES 2967713 T3 | 03 May 2024 |
| | | PE 14 January 2021 A1 | 19 January 2021 |
| | | US 2021310006 A1 | 07 October 2021 |
| | | US 11834661 B2 | 05 December 2023 |
| | | EP 3794122 A1 | 24 March 2021 |
| | | EP 3794122 B1 | 09 August 2023 |
| | | CL 2023002870 A1 | 28 June 2024 |
| | | JP 2021522841 A | 02 September 2021 |
| | | JP 7346460 B2 | 19 September 2023 |
| | | IL 278539 B | 01 June 2022 |
| | | MX 2020012048 A | 29 January 2021 |
| | | ZA 202108348 B | 29 November 2023 |
| | | RS 64812 B1 | 29 December 2023 |
| | | EP 4324520 A2 | 21 February 2024 |
| | | EP 4324520 A3 | 28 August 2024 |
| | | IL 292877 A | 01 July 2022 |
| | | IL 292877 B1 | 01 April 2024 |
| | | IL 292877 B2 | 01 August 2024 |
| | | CO 2020015314 A2 | 19 March 2021 |
| | | JP 2023182578 A | 26 December 2023 |
| | | WO 2019222166 A1 | 21 November 2019 |
| | | KR 20210010529 A | 27 January 2021 |
| | | US 2021095290 A1 | 01 April 2021 |
| | | US 11015201 B2 | 25 May 2021 |
| | | SG 11202011144 QA | 30 December 2020 |
| | | CL 2021002326 A1 | 13 May 2022 |
| | | LT 3794122 T | 27 November 2023 |
| | | PT 3794122 T | 22 November 2023 |
| | | TW 202016304 A | 01 May 2020 |
| | | BR 112020022943 A2 | 17 February 2021 |
| | | CA 3100003 A1 | 21 November 2019 |
| | | FI 3794122 T3 | 07 November 2023 |
| | | HUE 064073 T2 | 28 February 2024 |
| | | SI 3794122 T1 | 29 February 2024 |
| | | US 2024191236 A1 | 13 June 2024 |
| | | AU 2019269418 A1 | 07 January 2021 |
| | | CL 2020002865 A1 | 30 April 2021 |
| | | DK 3794122 T3 | 13 November 2023 |
| | | DK 3794122 T5 | 05 August 2024 |
| | | PH 12020551904 A1 | 21 June 2021 |
| | | HRP 20231412 T1 | 16 February 2024 |
| | | PL 3794122 T3 | 11 March 2024 |
| CN 114981431 A | 30 August 2022 | JP 2023502038 A | 20 January 2023 |
| | | CL 2022001256 A1 | 10 February 2023 |
| | | KR 20220115946 A | 19 August 2022 |
| | | MX 2022005692 A | 08 June 2022 |

Form PCT/ISA/210 (patent family annex) (July 2022)

**INTERNATIONAL SEARCH REPORT**
**Information on patent family members**

International application No.
**PCT/CN2024/105377**

| Patent document cited in search report | Publication date (day/month/year) | Patent family member(s) | Publication date (day/month/year) |
|---|---|---|---|
| | | TW 202132568 A | 01 September 2021 |
| | | CO 2022006092 A2 | 20 May 2022 |
| | | BR 112022009216 A2 | 02 August 2022 |
| | | AU 2020382478 A1 | 02 June 2022 |
| | | US 2023002765 A1 | 05 January 2023 |
| | | PE 20230179 A1 | 01 February 2023 |
| | | IL 292865 A | 01 July 2022 |
| | | CA 3161703 A1 | 20 May 2021 |
| | | WO 2021096763 A1 | 20 May 2021 |
| | | WO 2021096763 A8 | 24 June 2021 |
| | | EP 4058577 A1 | 21 September 2022 |
| CN 114763547 A | 19 July 2022 | None | |
| WO 2023284763 A1 | 19 January 2023 | JP 2024528634 A | 30 July 2024 |
| | | TW 202308665 A | 01 March 2023 |
| | | US 2024309378 A1 | 19 September 2024 |
| | | EP 4372086 A1 | 22 May 2024 |
| WO 2023088427 A1 | 25 May 2023 | EP 4435104 A1 | 25 September 2024 |
| | | AU 2022394690 A1 | 23 May 2024 |
| | | TW 202344687 A | 16 November 2023 |
| | | KR 20240107327 A | 09 July 2024 |
| | | MX 2024005818 A | 28 May 2024 |
| | | CA 3238317 A1 | 25 May 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 110582283 A
- WO 2009082607 A2
- CN 104717982 B
- US 20130178612 A1
- US 2015100197 A1


### Non-patent literature cited in the description


- **ASHWELL G** ; **HARFORD J**. Carbohydrate specific Receptors of the Liver. *Ann Rev Biochem*, 1982, vol. 51, 531-554
- Clinical Practice Guidelines for the Management of Hypertension in China. 2022
- *2022 China Hypertension Meeting and the 24th International Symposium on Hypertension & Related Diseases*, 11 March 2023
- **LAM et al.** *Molecular Therapy Nucleic Acids*, 2015, vol. 4, e252
- *J. Med. Chem.*, 2018, vol. 61, 734-744